# EUROPEAN PATENT APPLICATION

(11) **EP 4 703 388 A1**
(43) Date of publication of application: **04.03.2026**
(21) Application number: 24796241.8
(22) Date of filing: 26.04.2024
(51) Int. Cl.: C07K 16/36, A61K 39/395, A61P 35/00

(54) **ANTI-TF ANTIBODY AND ANTI-TF ANTIBODY-DRUG CONJUGATE AND MEDICAL USE THEREOF**

(30) Priority: 28.04.2023 CN 202310476753; 14.11.2023 CN 202311512744
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: SHI, Jinping, Shanghai 200245 (CN); XU, Shaoyu, Shanghai 200245 (CN); JIN, Xinsheng, Shanghai 200245 (CN); LI, Xun, Shanghai 200245 (CN); YING, Hanxiao, Shanghai 200245 (CN)
(74) Representative: Potter Clarkson
(86) International application number: PCT/CN2024/090058
(87) International publication number: WO 2024/222868

(57) **Abstract**

The present invention relates to an anti-TF antibody and an anti-TF antibody-drug conjugate and a medical use thereof. Specifically, the present invention relates to an anti-TF antibody, and an anti-TF antibody-drug conjugate represented by general formula (Pc-L-Y-D), wherein the Pc is the anti-TF antibody, and L, Y, and n are as defined in the description.

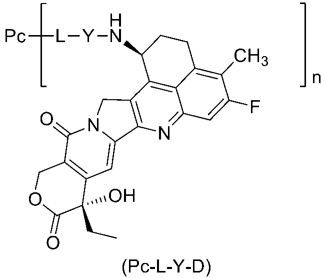

## Description

### TECHNICAL FIELD

The present disclosure relates to an anti-TF antibody and an anti-TF antibody-drug conjugate and medical use thereof.

### BACKGROUND

The statements herein merely provide background information related to the present disclosure and may not necessarily constitute the prior art.

Tissue factor, also known as TF/tissue thromboplastin/FIII/CD142, is a transmembrane glycoprotein that is 47 kDa in length, with 219 amino acids in an extracellular region, 23 amino acids in a transmembrane region, and 21 amino acids in an intracellular region. The gene of human tissue factor is named F3.

TF is mainly expressed by perivascular cells (such as adventitial fibroblasts and pericytes) and body surfaces (such as skin keratinocytes), and it is also expressed by monocytes, brain astrocytes, lung epithelial cells, cardiac fibroblasts, cardiomyocytes, etc. This broad expression favors the formation of a systemic hemostatic barrier from it.

TF is a promoter of extrinsic blood coagulation under normal physiological conditions and is a cellular receptor for FVII (coagulation factor VII) and FVIIa, the activated form of FVII, in the blood. When a blood vessel is damaged, TF forms a complex with FVII in the blood and activates FVII into FVIIa, forming a TF/FVIIa complex. TF/FVIIa can bind to FX (coagulation factor X) in the blood and activate it into FXa, forming a TF/FVIIa/FXa complex, which further triggers a coagulation cascade, activating prothrombin (FII) into thrombin (FIIa). Thrombin converts fibrinogen into insoluble fibrin, finally completing blood coagulation.

The TF/FVIIa complex can activate the PAR2 (proteinase-activated receptors 2) signaling pathway. The TF/FVIIa complex can also form a TF/FVIIa/integrin β1 complex with integrin β1. The downstream signaling pathways of PAR2 and integrin β1 can promote tumor growth. The downstream products of the TF/FVIIa coagulation cascade, thrombin and fibrin, are associated with tumor metastasis and thrombosis.

TF is highly expressed under pathological conditions in a variety of solid tumors, such as pancreatic cancer, lung cancer, breast cancer, gastric cancer, colorectal cancer, liver cancer, esophageal cancer, ovarian cancer, and bladder cancer. Various growth factors and cytokines induce TF expression, and various transcription factors and microRNAs regulate TF gene expression in cancer cells. For example, the hepatocyte growth factor (HGF)/c-Met and epidermal growth factor receptor (EGFR) signaling pathways can up-regulate TF expression by activating multiple kinase pathways, including JNK, Src, PI3k/Akt/mTOR, KRAS/Raf/MEK/ERK, etc. Transforming growth factor β (TGF-β) and vascular endothelial growth factor (VEGF) can also increase TF expression. During inflammation, TF can be up-regulated by a variety of cytokines, such as interferon-γ (IFN-γ) and tumor necrosis factor-α (TNF-α). TF expression can also be down-regulated by some micro RNAs such as miR-181b and miR19, or its activity is inhibited by the TFPI (tissue factor pathway inhibitor) on monocytes.

The high expression of TF under pathological conditions can promote the growth and metastasis of tumors and the formation of thrombi and is associated with poor prognoses. Therefore, a TF-targeted ADC can not only inhibit tumor growth through the signal blocking effect of the antibody but also achieve the specific killing of tumors by the toxin through the targeting capability of the TF antibody.

### SUMMARY

The present disclosure relates to an anti-TF antibody, an anti-TF antibody-drug conjugate (particularly an anti-TF antibody-exatecan analog conjugate), and pharmaceutical use thereof.

The present disclosure provides an anti-TF antibody, wherein the anti-TF antibody comprises a heavy chain variable region and a light chain variable region, wherein:
a. the heavy chain variable region comprises a HCDR1 comprising the amino acid sequence of SEQ ID NO: 40, a HCDR2 comprising the amino acid sequence of SEQ ID NO: 64 or 41, and a HCDR3 comprising the amino acid sequence of SEQ ID NO: 65 or 42, and the light chain variable region comprises a LCDR1 comprising the amino acid sequence of SEQ ID NO: 66 or 43, a LCDR2 comprising the amino acid sequence of SEQ ID NO: 67 or 44, and a LCDR3 comprising the amino acid sequence of SEQ ID NO: 68, 83, or 45;
b. the heavy chain variable region comprises a HCDR1 comprising the amino acid sequence of SEQ ID NO: 46, a HCDR2 comprising the amino acid sequence of SEQ ID NO: 47, and a HCDR3 comprising the amino acid sequence of SEQ ID NO: 48, and the light chain variable region comprises a LCDR1 comprising the amino acid sequence of SEQ ID NO: 49, 111, 112, or 113, a LCDR2 comprising the amino acid sequence of SEQ ID NO: 50, and a LCDR3 comprising the amino acid sequence of SEQ ID NO: 51;
c. the heavy chain variable region comprises a HCDR1 comprising the amino acid sequence of SEQ ID NO: 73 or 144, a HCDR2 comprising the amino acid sequence of SEQ ID NO: 74, 142, 145, or 146, and a HCDR3 comprising the amino acid sequence of SEQ ID NO: 75 or 143, and the light chain variable region comprises a LCDR1 comprising the amino acid sequence of SEQ ID NO: 76, 151, 147, or 150, a LCDR2 comprising the amino acid sequence of SEQ ID NO: 77 or 148, and a LCDR3 comprising the amino acid sequence of SEQ ID NO: 78 or 149;
preferably,
a. the heavy chain variable region comprises a HCDR1 comprising the amino acid sequence of SEQ ID NO: 40, a HCDR2 comprising the amino acid sequence of SEQ ID NO: 64, and a HCDR3 comprising the amino acid sequence of SEQ ID NO: 65, and the light chain variable region comprises a LCDR1 comprising the amino acid sequence of SEQ ID NO: 66, a LCDR2 comprising the amino acid sequence of SEQ ID NO: 67, and a LCDR3 comprising the amino acid sequence of SEQ ID NO: 68 or 83; or
   the heavy chain variable region comprises a HCDR1 comprising the amino acid sequence of SEQ ID NO: 40, a HCDR2 comprising the amino acid sequence of SEQ ID NO: 41, and a HCDR3 comprising the amino acid sequence of SEQ ID NO: 42, and the light chain variable region comprises a LCDR1 comprising the amino acid sequence of SEQ ID NO: 43, a LCDR2 comprising the amino acid sequence of SEQ ID NO: 44, and a LCDR3 comprising the amino acid sequence of SEQ ID NO: 45;
b. the heavy chain variable region comprises a HCDR1 comprising the amino acid sequence of SEQ ID NO: 46, a HCDR2 comprising the amino acid sequence of SEQ ID NO: 47, and a HCDR3 comprising the amino acid sequence of SEQ ID NO: 48, and the light chain variable region comprises a LCDR1 comprising the amino acid sequence of SEQ ID NO: 49, a LCDR2 comprising the amino acid sequence of SEQ ID NO: 50, and a LCDR3 comprising the amino acid sequence of SEQ ID NO: 51;
c. the heavy chain variable region comprises a HCDR1 comprising the amino acid sequence of SEQ ID NO: 73, a HCDR2 comprising the amino acid sequence of SEQ ID NO: 74, and a HCDR3 comprising the amino acid sequence of SEQ ID NO: 75, and the light chain variable region comprises a LCDR1 comprising the amino acid sequence of SEQ ID NO: 76, or 151, a LCDR2 comprising the amino acid sequence of SEQ ID NO: 77 or 148, and a LCDR3 comprising the amino acid sequence of SEQ ID NO: 78.

In some embodiments, provided is the anti-TF antibody described above, wherein the HCDR1-3 and LCDR1-3 described above are determined according to the Kabat numbering scheme. In some embodiments, provided is the anti-TF antibody described above, wherein the HCDR1-3 and LCDR1-3 described above are determined according to the IMGT numbering scheme. In some embodiments, provided is the anti-TF antibody described above, wherein the HCDR1-3 and LCDR1-3 described above are determined according to the Chothia numbering scheme. In some embodiments, provided is the anti-TF antibody described above, wherein the HCDR1-3 and LCDR1-3 described above are determined according to the AbM numbering scheme. In some embodiments, provided is the anti-TF antibody described above, wherein the HCDR1-3 and LCDR1-3 described above are determined according to the Contact numbering scheme.

The present disclosure provides an anti-TF antibody, wherein the anti-TF antibody comprises a heavy chain variable region and a light chain variable region, wherein:
a. the heavy chain variable region comprises a HCDR1, a HCDR2, and a HCDR3 set forth in SEQ ID NO: 40, SEQ ID NO: 64, and SEQ ID NO: 65, respectively; the light chain variable region comprises a LCDR1, a LCDR2, and a LCDR3 set forth in SEQ ID NO: 66, SEQ ID NO: 67, and SEQ ID NO: 68, respectively; or
   the heavy chain variable region comprises a HCDR1, a HCDR2, and a HCDR3 set forth in SEQ ID NO: 40, SEQ ID NO: 64, and SEQ ID NO: 65, respectively; the light chain variable region comprises a LCDR1, a LCDR2, and a LCDR3 set forth in SEQ ID NO: 66, SEQ ID NO: 67, and SEQ ID NO: 83, respectively; or
   the heavy chain variable region comprises a HCDR1, a HCDR2, and a HCDR3 set forth in SEQ ID NO: 40, SEQ ID NO: 41, and SEQ ID NO: 42, respectively; the light chain variable region comprises a LCDR1, a LCDR2, and a LCDR3 set forth in SEQ ID NO: 43, SEQ ID NO: 44, and SEQ ID NO: 45, respectively;
b. the heavy chain variable region comprises a HCDR1, a HCDR2, and a HCDR3 set forth in SEQ ID NO: 46, SEQ ID NO: 47, and SEQ ID NO: 48, respectively; the light chain variable region comprises a LCDR1, a LCDR2, and a LCDR3 set forth in SEQ ID NO: 49, SEQ ID NO: 50, and SEQ ID NO: 51, respectively;
c. the heavy chain variable region comprises a HCDR1, a HCDR2, and a HCDR3 set forth in SEQ ID NO: 73, SEQ ID NO: 74, and SEQ ID NO: 75, respectively; the light chain variable region comprises a LCDR1, a LCDR2, and a LCDR3 set forth in SEQ ID NO: 76, SEQ ID NO: 77, and SEQ ID NO: 78, respectively; or
   the heavy chain variable region comprises a HCDR1, a HCDR2, and a HCDR3 set forth in SEQ ID NO: 73, SEQ ID NO: 74, and SEQ ID NO: 75, respectively; the light chain variable region comprises a LCDR1, a LCDR2, and a LCDR3 set forth in SEQ ID NO: 151, SEQ ID NO: 148, and SEQ ID NO: 78, respectively;
   the HCDR1-3 and LCDR1-3 described above are determined according to the Kabat numbering scheme.

The present disclosure provides an anti-TF antibody, wherein the anti-TF antibody comprises a heavy chain variable region and a light chain variable region, wherein:
a. the heavy chain variable region comprises a HCDR1, a HCDR2, and a HCDR3 set forth in SEQ ID NO: 40, SEQ ID NO: 64, and SEQ ID NO: 65, respectively; the light chain variable region comprises a LCDR1, a LCDR2, and a LCDR3 set forth in SEQ ID NO: 66, SEQ ID NO: 67, and SEQ ID NO: 68, respectively;
b. the heavy chain variable region comprises a HCDR1, a HCDR2, and a HCDR3 set forth in SEQ ID NO: 46, SEQ ID NO: 47, and SEQ ID NO: 48, respectively; the light chain variable region comprises a LCDR1, a LCDR2, and a LCDR3 set forth in SEQ ID NO: 49, SEQ ID NO: 50, and SEQ ID NO: 51, respectively;
c. the heavy chain variable region comprises a HCDR1, a HCDR2, and a HCDR3 set forth in SEQ ID NO: 73, SEQ ID NO: 74, and SEQ ID NO: 75, respectively; the light chain variable region comprises a LCDR1, a LCDR2, and a LCDR3 set forth in SEQ ID NO: 76, SEQ ID NO: 77, and SEQ ID NO: 78, respectively.

In some embodiments, provided is the anti-TF antibody described above, which is a murine antibody, a chimeric antibody, or a humanized antibody.

In some embodiments, provided is the anti-TF antibody described above, which is a chimeric antibody or a humanized antibody.

In some embodiments, provided is the anti-TF antibody described above, which is a humanized antibody.

In some embodiments, provided is the anti-TF antibody according to any one of the above, which comprises a heavy chain variable region and a light chain variable region, wherein:
a. the heavy chain variable region comprises an amino acid sequence having at least 80% sequence identity to SEQ ID NO: 62, 58, 130, 131, or 132, and the light chain variable region comprises an amino acid sequence having at least 80% sequence identity to SEQ ID NO: 63, 81, 59, 133, 134, or 135; or the heavy chain variable region comprises an amino acid sequence having at least 80% sequence identity to SEQ ID NO: 36, and the light chain variable region comprises an amino acid sequence having at least 80% sequence identity to SEQ ID NO: 37;
b. the heavy chain variable region comprises an amino acid sequence having at least 80% sequence identity to SEQ ID NO: 52 or 114, and the light chain variable region comprises an amino acid sequence having at least 80% sequence identity to SEQ ID NO: 53, 115, 116, 117, 118, 119, 120, or 121; or the heavy chain variable region comprises an amino acid sequence having at least 80% sequence identity to SEQ ID NO: 38, and the light chain variable region comprises an amino acid sequence having at least 80% sequence identity to SEQ ID NO: 39;
c. the heavy chain variable region comprises an amino acid sequence having at least 80% sequence identity to SEQ ID NO: 71, 84, 85, 86, 87, 88, 89, 90, 91, 92, 93, 94, 95, or 96, and the light chain variable region comprises an amino acid sequence having at least 80% sequence identity to SEQ ID NO: 72, 97, 102, 98, 99, 100, or 101;
preferably,
a. the heavy chain variable region comprises the amino acid sequence of SEQ ID NO: 62, 58, 130, 131, or 132, and the light chain variable region comprises the amino acid sequence of SEQ ID NO: 63, 81, 59, 133, 134, or 135; or the heavy chain variable region comprises the amino acid sequence of SEQ ID NO: 36, and the light chain variable region comprises the amino acid sequence of SEQ ID NO: 37;
b. the heavy chain variable region comprises the amino acid sequence of SEQ ID NO: 52 or 114, and the light chain variable region comprises the amino acid sequence of SEQ ID NO: 53, 115, 116, 117, 118, 119, 120, or 121; or the heavy chain variable region comprises the amino acid sequence of SEQ ID NO: 38, and the light chain variable region comprises the amino acid sequence of SEQ ID NO: 39;
c. the heavy chain variable region comprises the amino acid sequence of SEQ ID NO: 71, 84, 85, 86, 87, 88, 89, 90, 91, 92, 93, 94, 95, or 96, and the light chain variable region comprises the amino acid sequence of SEQ ID NO: 72, 97, 102, 98, 99, 100, or 101;
more preferably,
a. the heavy chain variable region comprises the amino acid sequence of SEQ ID NO: 62, and the light chain variable region comprises the amino acid sequence of SEQ ID NO: 63 or 81; or
   the heavy chain variable region comprises the amino acid sequence of SEQ ID NO: 58, and the light chain variable region comprises the amino acid sequence of SEQ ID NO: 59; or
   the heavy chain variable region comprises the amino acid sequence of SEQ ID NO: 36, and the light chain variable region comprises the amino acid sequence of SEQ ID NO: 37; or
   the heavy chain variable region comprises the amino acid sequence of SEQ ID NO: 130, and the light chain variable region comprises the amino acid sequence of SEQ ID NO: 133; or
   the heavy chain variable region comprises the amino acid sequence of SEQ ID NO: 131, and the light chain variable region comprises the amino acid sequence of SEQ ID NO: 133; or
   the heavy chain variable region comprises the amino acid sequence of SEQ ID NO: 58, and the light chain variable region comprises the amino acid sequence of SEQ ID NO: 133; or
   the heavy chain variable region comprises the amino acid sequence of SEQ ID NO: 132, and the light chain variable region comprises the amino acid sequence of SEQ ID NO: 133; or
   the heavy chain variable region comprises the amino acid sequence of SEQ ID NO: 130, and the light chain variable region comprises the amino acid sequence of SEQ ID NO: 134; or
   the heavy chain variable region comprises the amino acid sequence of SEQ ID NO: 131, and the light chain variable region comprises the amino acid sequence of SEQ ID NO: 134; or
   the heavy chain variable region comprises the amino acid sequence of SEQ ID NO: 58, and the light chain variable region comprises the amino acid sequence of SEQ ID NO: 134; or
   the heavy chain variable region comprises the amino acid sequence of SEQ ID NO: 132, and the light chain variable region comprises the amino acid sequence of SEQ ID NO: 134; or
   the heavy chain variable region comprises the amino acid sequence of SEQ ID NO: 130, and the light chain variable region comprises the amino acid sequence of SEQ ID NO: 59; or
   the heavy chain variable region comprises the amino acid sequence of SEQ ID NO: 131, and the light chain variable region comprises the amino acid sequence of SEQ ID NO: 59; or
   the heavy chain variable region comprises the amino acid sequence of SEQ ID NO: 132, and the light chain variable region comprises the amino acid sequence of SEQ ID NO: 59; or
   the heavy chain variable region comprises the amino acid sequence of SEQ ID NO: 130, and the light chain variable region comprises the amino acid sequence of SEQ ID NO: 135; or
   the heavy chain variable region comprises the amino acid sequence of SEQ ID NO: 131, and the light chain variable region comprises the amino acid sequence of SEQ ID NO: 135; or
   the heavy chain variable region comprises the amino acid sequence of SEQ ID NO: 58, and the light chain variable region comprises the amino acid sequence of SEQ ID NO: 135; or
   the heavy chain variable region comprises the amino acid sequence of SEQ ID NO: 132, and the light chain variable region comprises the amino acid sequence of SEQ ID NO: 135;
b. the heavy chain variable region comprises the amino acid sequence of SEQ ID NO: 52, and the light chain variable region comprises the amino acid sequence of SEQ ID NO: 53; or
   the heavy chain variable region comprises the amino acid sequence of SEQ ID NO: 38, and the light chain variable region comprises the amino acid sequence of SEQ ID NO: 39;
c. the heavy chain variable region comprises the amino acid sequence of SEQ ID NO: 71, and the light chain variable region comprises the amino acid sequence of SEQ ID NO: 72, 97, or 102.

In some embodiments, provided is the anti-TF antibody according to any one of the above, which comprises a heavy chain variable region and a light chain variable region, wherein:
a. the heavy chain variable region is set forth in SEQ ID NO: 36, and the light chain variable region is set forth in SEQ ID NO: 37; or
b. the heavy chain variable region is set forth in SEQ ID NO: 38, and the light chain variable region is set forth in SEQ ID NO: 39.

In some embodiments, provided is the anti-TF antibody according to any one of the above, which comprises a heavy chain variable region and a light chain variable region, wherein:
a. the heavy chain variable region is set forth in SEQ ID NO: 62, and the light chain variable region is set forth in SEQ ID NO: 63; or
   the heavy chain variable region is set forth in SEQ ID NO: 62, and the light chain variable region is set forth in SEQ ID NO: 81; or
   the heavy chain variable region is set forth in SEQ ID NO: 58, and the light chain variable region is set forth in SEQ ID NO: 59; or
   the heavy chain variable region is set forth in SEQ ID NO: 36, and the light chain variable region is set forth in SEQ ID NO: 37; or
   the heavy chain variable region is set forth in SEQ ID NO: 130, and the light chain variable region is set forth in SEQ ID NO: 133; or
   the heavy chain variable region is set forth in SEQ ID NO: 131, and the light chain variable region is set forth in SEQ ID NO: 133; or
   the heavy chain variable region is set forth in SEQ ID NO: 58, and the light chain variable region is set forth in SEQ ID NO: 133; or
   the heavy chain variable region is set forth in SEQ ID NO: 132, and the light chain variable region is set forth in SEQ ID NO: 133; or
   the heavy chain variable region is set forth in SEQ ID NO: 130, and the light chain variable region is set forth in SEQ ID NO: 134; or
   the heavy chain variable region is set forth in SEQ ID NO: 131, and the light chain variable region is set forth in SEQ ID NO: 134; or
   the heavy chain variable region is set forth in SEQ ID NO: 58, and the light chain variable region is set forth in SEQ ID NO: 134; or
   the heavy chain variable region is set forth in SEQ ID NO: 132, and the light chain variable region is set forth in SEQ ID NO: 134; or
   the heavy chain variable region is set forth in SEQ ID NO: 130, and the light chain variable region is set forth in SEQ ID NO: 59; or
   the heavy chain variable region is set forth in SEQ ID NO: 131, and the light chain variable region is set forth in SEQ ID NO: 59; or
   the heavy chain variable region is set forth in SEQ ID NO: 132, and the light chain variable region is set forth in SEQ ID NO: 59; or
   the heavy chain variable region is set forth in SEQ ID NO: 130, and the light chain variable region is set forth in SEQ ID NO: 135; or
   the heavy chain variable region is set forth in SEQ ID NO: 131, and the light chain variable region is set forth in SEQ ID NO: 135; or
   the heavy chain variable region is set forth in SEQ ID NO: 58, and the light chain variable region is set forth in SEQ ID NO: 135; or
   the heavy chain variable region is set forth in SEQ ID NO: 132, and the light chain variable region is set forth in SEQ ID NO: 135;
b. the heavy chain variable region is set forth in SEQ ID NO: 52, and the light chain variable region is set forth in SEQ ID NO: 53; or
   the heavy chain variable region is set forth in SEQ ID NO: 38, and the light chain variable region is set forth in SEQ ID NO: 39;
c. the heavy chain variable region is set forth in SEQ ID NO: 71, and the light chain variable region is set forth in SEQ ID NO: 72; or
   the heavy chain variable region is set forth in SEQ ID NO: 71, and the light chain variable region is set forth in SEQ ID NO: 97; or
   the heavy chain variable region is set forth in SEQ ID NO: 71, and the light chain variable region is set forth in SEQ ID NO: 102.

In some embodiments, provided is the anti-TF antibody according to any one of the above, which comprises a heavy chain variable region and a light chain variable region, wherein:
a. the heavy chain variable region is set forth in SEQ ID NO: 62, and the light chain variable region is set forth in SEQ ID NO: 63; or
b. the heavy chain variable region is set forth in SEQ ID NO: 52, and the light chain variable region is set forth in SEQ ID NO: 53; or
c. the heavy chain variable region is set forth in SEQ ID NO: 71, and the light chain variable region is set forth in SEQ ID NO: 72.

In some embodiments, provided is the anti-TF antibody according to any one of the above, which comprises a heavy chain variable region and a light chain variable region, wherein:
the heavy chain variable region is selected from the group consisting of SEQ ID NOs: 130, 131, and 132, and the light chain variable region is selected from SEQ ID NOs: 133, 134, and 135.

In some embodiments, provided is the anti-TF antibody according to any one of the above, which comprises a heavy chain variable region and a light chain variable region, wherein:
the heavy chain variable region is selected from the group consisting of SEQ ID NO: 114, and the light chain variable region is selected from the group consisting of SEQ ID NOs: 115, 116, 117, 118, 119, 120, and 121.

In some embodiments, provided is the anti-TF antibody according to any one of the above, which comprises a heavy chain variable region and a light chain variable region, wherein:
the heavy chain variable region is selected from the group consisting of SEQ ID NOs: 84, 85, 86, 87, 88, 89, 90, 91, 92, 93, 71, 94, 95, and 96, and the light chain variable region is selected from the group consisting of SEQ ID NOs: 97, 98, 99, 100, 101, and 102.

In some embodiments, provided is the anti-TF antibody according to any one of the above, which is an antibody fragment; preferably, the antibody fragment is selected from the group consisting of Fab, Fab', F(ab')2, Fd, Fv, scFv, dsFv, and dAb.

In some embodiments, the anti-TF antibody according to any one of the above further comprises an antibody heavy chain constant region and an antibody light chain constant region; preferably, the heavy chain constant region is selected from the group consisting of human IgG1, IgG2, IgG3, and IgG4 constant regions, and the light chain constant region is a human κ or λ chain constant region; more preferably, the heavy chain constant region comprises the amino acid sequence of SEQ ID NO: 54, and the light chain constant region comprises the amino acid sequence of SEQ ID NO: 55.

In some embodiments, the amino acid sequence of the heavy chain constant region is set forth in SEQ ID NO: 54, and the amino acid sequence of the light chain constant region is set forth in SEQ ID NO: 55.

In some embodiments, provided is the anti-TF antibody according to any one of the above, which is selected from the group consisting of antibodies comprising a heavy chain and a light chain according to any one of the following:
a. a heavy chain having at least 80% sequence identity to SEQ ID NO: 69, 60, 136, 137, or 138, and/or a light chain having at least 80% sequence identity to SEQ ID NO: 70, 82, 61, 139, 140, or 141; or
   a heavy chain having at least 80% sequence identity to SEQ ID NO: 107, and/or a light chain having at least 80% sequence identity to SEQ ID NO: 108;
b. a heavy chain having at least 80% sequence identity to SEQ ID NO: 56 or 122, and/or a light chain having at least 80% sequence identity to SEQ ID NO: 57, 123, 124, 125, 126, 127, 128, or 129; or
   a heavy chain having at least 80% sequence identity to SEQ ID NO: 109, and/or a light chain having at least 80% sequence identity to SEQ ID NO: 110;
c. a heavy chain having at least 80% sequence identity to SEQ ID NO: 79, 152, 153, 154, 155, 156, 157, 158, 159, 160, 161, 162, 163, or 164, and/or a light chain having at least 80% sequence identity to SEQ ID NO: 80, 165, 170, 166, 167, 168, or 169;
preferably,
a. a heavy chain having at least 80% sequence identity to SEQ ID NO: 69, and/or a light chain having at least 80% sequence identity to SEQ ID NO: 70 or 82; or
   a heavy chain having at least 80% sequence identity to SEQ ID NO: 60, and/or a light chain having at least 80% sequence identity to SEQ ID NO: 61; or
   a heavy chain having at least 80% sequence identity to SEQ ID NO: 107, and/or a light chain having at least 80% sequence identity to SEQ ID NO: 108; or
   a heavy chain having at least 80% sequence identity to SEQ ID NO: 136, and/or a light chain having at least 80% sequence identity to SEQ ID NO: 139; or
   a heavy chain having at least 80% sequence identity to SEQ ID NO: 137, and/or a light chain having at least 80% sequence identity to SEQ ID NO: 139; or
   a heavy chain having at least 80% sequence identity to SEQ ID NO: 60, and/or a light chain having at least 80% sequence identity to SEQ ID NO: 139; or
   a heavy chain having at least 80% sequence identity to SEQ ID NO: 138, and/or a light chain having at least 80% sequence identity to SEQ ID NO: 139; or
   a heavy chain having at least 80% sequence identity to SEQ ID NO: 136, and/or a light chain having at least 80% sequence identity to SEQ ID NO: 140; or
   a heavy chain having at least 80% sequence identity to SEQ ID NO: 137, and/or a light chain having at least 80% sequence identity to SEQ ID NO: 140; or
   a heavy chain having at least 80% sequence identity to SEQ ID NO: 60, and/or a light chain having at least 80% sequence identity to SEQ ID NO: 140; or
   a heavy chain having at least 80% sequence identity to SEQ ID NO: 138, and/or a light chain having at least 80% sequence identity to SEQ ID NO: 140; or
   a heavy chain having at least 80% sequence identity to SEQ ID NO: 136, and/or a light chain having at least 80% sequence identity to SEQ ID NO: 61; or
   a heavy chain having at least 80% sequence identity to SEQ ID NO: 137, and/or a light chain having at least 80% sequence identity to SEQ ID NO: 61; or
   a heavy chain having at least 80% sequence identity to SEQ ID NO: 138, and/or a light chain having at least 80% sequence identity to SEQ ID NO: 61; or
   a heavy chain having at least 80% sequence identity to SEQ ID NO: 136, and/or a light chain having at least 80% sequence identity to SEQ ID NO: 141; or
   a heavy chain having at least 80% sequence identity to SEQ ID NO: 137, and/or a light chain having at least 80% sequence identity to SEQ ID NO: 141; or
   a heavy chain having at least 80% sequence identity to SEQ ID NO: 60, and/or a light chain having at least 80% sequence identity to SEQ ID NO: 141; or
   a heavy chain having at least 80% sequence identity to SEQ ID NO: 138, and/or a light chain having at least 80% sequence identity to SEQ ID NO: 141;
b. a heavy chain having at least 80% sequence identity to SEQ ID NO: 56, and/or a light chain having at least 80% sequence identity to SEQ ID NO: 57; or
   a heavy chain having at least 80% sequence identity to SEQ ID NO: 109, and/or a light chain having at least 80% sequence identity to SEQ ID NO: 110;
c. a heavy chain having at least 80% sequence identity to SEQ ID NO: 79, and/or a light chain having at least 80% sequence identity to SEQ ID NO: 80, 165, or 170;
more preferably,
a. the heavy chain comprises the amino acid sequence of SEQ ID NO: 69, and the light chain comprises the amino acid sequence of SEQ ID NO: 70; or
   the heavy chain comprises the amino acid sequence of SEQ ID NO: 69, and the light chain comprises the amino acid sequence of SEQ ID NO: 82; or
   the heavy chain comprises the amino acid sequence of SEQ ID NO: 60, and the light chain comprises the amino acid sequence of SEQ ID NO: 61; or
   the heavy chain comprises the amino acid sequence of SEQ ID NO: 107, and the light chain comprises the amino acid sequence of SEQ ID NO: 108; or
   the heavy chain comprises the amino acid sequence of SEQ ID NO: 136, and the light chain comprises the amino acid sequence of SEQ ID NO: 139; or
   the heavy chain comprises the amino acid sequence of SEQ ID NO: 137, and the light chain comprises the amino acid sequence of SEQ ID NO: 139; or
   the heavy chain comprises the amino acid sequence of SEQ ID NO: 60, and the light chain comprises the amino acid sequence of SEQ ID NO: 139; or
   the heavy chain comprises the amino acid sequence of SEQ ID NO: 138, and the light chain comprises the amino acid sequence of SEQ ID NO: 139; or
   the heavy chain comprises the amino acid sequence of SEQ ID NO: 136, and the light chain comprises the amino acid sequence of SEQ ID NO: 140; or
   the heavy chain comprises the amino acid sequence of SEQ ID NO: 137, and the light chain comprises the amino acid sequence of SEQ ID NO: 140; or
   the heavy chain comprises the amino acid sequence of SEQ ID NO: 60, and the light chain comprises the amino acid sequence of SEQ ID NO: 140; or
   the heavy chain comprises the amino acid sequence of SEQ ID NO: 138, and the light chain comprises the amino acid sequence of SEQ ID NO: 140; or
   the heavy chain comprises the amino acid sequence of SEQ ID NO: 136, and the light chain comprises the amino acid sequence of SEQ ID NO: 61; or
   the heavy chain comprises the amino acid sequence of SEQ ID NO: 137, and the light chain comprises the amino acid sequence of SEQ ID NO: 61; or
   the heavy chain comprises the amino acid sequence of SEQ ID NO: 138, and the light chain comprises the amino acid sequence of SEQ ID NO: 61; or
   the heavy chain comprises the amino acid sequence of SEQ ID NO: 136, and the light chain comprises the amino acid sequence of SEQ ID NO: 141; or
   the heavy chain comprises the amino acid sequence of SEQ ID NO: 137, and the light chain comprises the amino acid sequence of SEQ ID NO: 141; or
   the heavy chain comprises the amino acid sequence of SEQ ID NO: 60, and the light chain comprises the amino acid sequence of SEQ ID NO: 141; or
   the heavy chain comprises the amino acid sequence of SEQ ID NO: 138, and the light chain comprises the amino acid sequence of SEQ ID NO: 141;
b. the heavy chain comprises the amino acid sequence of SEQ ID NO: 56, and the light chain comprises the amino acid sequence of SEQ ID NO: 57; or
   the heavy chain comprises the amino acid sequence of SEQ ID NO: 109, and the light chain comprises the amino acid sequence of SEQ ID NO: 110;
c. the heavy chain comprises the amino acid sequence of SEQ ID NO: 79, and the light chain comprises the amino acid sequence of SEQ ID NO: 80; or
   the heavy chain comprises the amino acid sequence of SEQ ID NO: 79, and the light chain comprises the amino acid sequence of SEQ ID NO: 165; or
   the heavy chain comprises the amino acid sequence of SEQ ID NO: 79, and the light chain comprises the amino acid sequence of SEQ ID NO: 170.

The "at least 80% sequence identity" described above includes at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity.

In some embodiments, provided is the anti-TF antibody according to any one of the above, which comprises a heavy chain and a light chain shown below:
a. a heavy chain having at least 85% sequence identity to SEQ ID NO: 69, and/or a light chain having at least 85% sequence identity to SEQ ID NO: 70; or
   a heavy chain having at least 85% sequence identity to SEQ ID NO: 69, and/or a light chain having at least 85% sequence identity to SEQ ID NO: 82; or
   a heavy chain having at least 85% sequence identity to SEQ ID NO: 60, and/or a light chain having at least 85% sequence identity to SEQ ID NO: 61; or
   a heavy chain having at least 85% sequence identity to SEQ ID NO: 107, and/or a light chain having at least 85% sequence identity to SEQ ID NO: 108; or
   a heavy chain having at least 85% sequence identity to SEQ ID NO: 136, and/or a light chain having at least 85% sequence identity to SEQ ID NO: 139; or
   a heavy chain having at least 85% sequence identity to SEQ ID NO: 137, and/or a light chain having at least 85% sequence identity to SEQ ID NO: 139; or
   a heavy chain having at least 85% sequence identity to SEQ ID NO: 60, and/or a light chain having at least 85% sequence identity to SEQ ID NO: 139; or
   a heavy chain having at least 85% sequence identity to SEQ ID NO: 138, and/or a light chain having at least 85% sequence identity to SEQ ID NO: 139; or
   a heavy chain having at least 85% sequence identity to SEQ ID NO: 136, and/or a light chain having at least 85% sequence identity to SEQ ID NO: 140; or
   a heavy chain having at least 85% sequence identity to SEQ ID NO: 137, and/or a light chain having at least 85% sequence identity to SEQ ID NO: 140; or
   a heavy chain having at least 85% sequence identity to SEQ ID NO: 60, and/or a light chain having at least 85% sequence identity to SEQ ID NO: 140; or
   a heavy chain having at least 85% sequence identity to SEQ ID NO: 138, and/or a light chain having at least 85% sequence identity to SEQ ID NO: 140; or
   a heavy chain having at least 85% sequence identity to SEQ ID NO: 136, and/or a light chain having at least 85% sequence identity to SEQ ID NO: 61; or
   a heavy chain having at least 85% sequence identity to SEQ ID NO: 137, and/or a light chain having at least 85% sequence identity to SEQ ID NO: 61; or
   a heavy chain having at least 85% sequence identity to SEQ ID NO: 138, and/or a light chain having at least 85% sequence identity to SEQ ID NO: 61; or
   a heavy chain having at least 85% sequence identity to SEQ ID NO: 136, and/or a light chain having at least 85% sequence identity to SEQ ID NO: 141; or
   a heavy chain having at least 85% sequence identity to SEQ ID NO: 137, and/or a light chain having at least 85% sequence identity to SEQ ID NO: 141; or
   a heavy chain having at least 85% sequence identity to SEQ ID NO: 60, and/or a light chain having at least 85% sequence identity to SEQ ID NO: 141; or
   a heavy chain having at least 85% sequence identity to SEQ ID NO: 138, and/or a light chain having at least 85% sequence identity to SEQ ID NO: 141;
b. a heavy chain having at least 85% sequence identity to SEQ ID NO: 56, and/or a light chain having at least 85% sequence identity to SEQ ID NO: 57; or
   a heavy chain having at least 85% sequence identity to SEQ ID NO: 109, and/or a light chain having at least 85% sequence identity to SEQ ID NO: 110;
c. a heavy chain having at least 85% sequence identity to SEQ ID NO: 79, and/or a light chain having at least 85% sequence identity to SEQ ID NO: 80; or
   a heavy chain having at least 85% sequence identity to SEQ ID NO: 79, and/or a light chain having at least 85% sequence identity to SEQ ID NO: 165; or
   a heavy chain having at least 85% sequence identity to SEQ ID NO: 79, and/or a light chain having at least 85% sequence identity to SEQ ID NO: 170.

The "at least 85% sequence identity" described above includes at least 85%, 90%, 91%, 92%, 93%, 94%, 95%, 96%, 97%, 98%, 99%, or 100% sequence identity.

In some embodiments, provided is the anti-TF antibody according to any one of the above, which is selected from the group consisting of antibodies comprising a heavy chain and a light chain according to any one of the following:
a. the heavy chain is set forth in SEQ ID NO: 69, and the light chain is set forth in SEQ ID NO: 70; or
   the heavy chain is set forth in SEQ ID NO: 69, and the light chain is set forth in SEQ ID NO: 82; or
   the heavy chain is set forth in SEQ ID NO: 60, and the light chain is set forth in SEQ ID NO: 61; or
   the heavy chain is set forth in SEQ ID NO: 107, and the light chain is set forth in SEQ ID NO: 108; or
   the heavy chain is set forth in SEQ ID NO: 136, and the light chain is set forth in SEQ ID NO: 139; or
   the heavy chain is set forth in SEQ ID NO: 137, and the light chain is set forth in SEQ ID NO: 139; or
   the heavy chain is set forth in SEQ ID NO: 60, and the light chain is set forth in SEQ ID NO: 139; or
   the heavy chain is set forth in SEQ ID NO: 138, and the light chain is set forth in SEQ ID NO: 139; or
   the heavy chain is set forth in SEQ ID NO: 136, and the light chain is set forth in SEQ ID NO: 140; or
   the heavy chain is set forth in SEQ ID NO: 137, and the light chain is set forth in SEQ ID NO: 140; or
   the heavy chain is set forth in SEQ ID NO: 60, and the light chain is set forth in SEQ ID NO: 140; or
   the heavy chain is set forth in SEQ ID NO: 138, and the light chain is set forth in SEQ ID NO: 140; or
   the heavy chain is set forth in SEQ ID NO: 136, and the light chain is set forth in SEQ ID NO: 61; or
   the heavy chain is set forth in SEQ ID NO: 137, and the light chain is set forth in SEQ ID NO: 61; or
   the heavy chain is set forth in SEQ ID NO: 138, and the light chain is set forth in SEQ ID NO: 61; or
   the heavy chain is set forth in SEQ ID NO: 136, and the light chain is set forth in SEQ ID NO: 141; or
   the heavy chain is set forth in SEQ ID NO: 137, and the light chain is set forth in SEQ ID NO: 141; or
   the heavy chain is set forth in SEQ ID NO: 60, and the light chain is set forth in SEQ ID NO: 141; or
   the heavy chain is set forth in SEQ ID NO: 138, and the light chain is set forth in SEQ ID NO: 141;
b. the heavy chain is set forth in SEQ ID NO: 56, and the light chain is set forth in SEQ ID NO: 57; or
   the heavy chain is set forth in SEQ ID NO: 109, and the light chain is set forth in SEQ ID NO: 110;
c. the heavy chain is set forth in SEQ ID NO: 79; and the light chain is set forth in SEQ ID NO: 80; or
   the heavy chain is set forth in SEQ ID NO: 79, and the light chain is set forth in SEQ ID NO: 165; or
   the heavy chain is set forth in SEQ ID NO: 79, and the light chain is set forth in SEQ ID NO: 170.

In some embodiments, provided is the anti-TF antibody according to any one of the above, which comprises a heavy chain and a light chain:
a. the heavy chain is set forth in SEQ ID NO: 69, and the light chain is set forth in SEQ ID NO: 70;
b. the heavy chain is set forth in SEQ ID NO: 56, and the light chain is set forth in SEQ ID NO: 57;
c. the heavy chain is set forth in SEQ ID NO: 79; and the light chain is set forth in SEQ ID NO: 80.

In some embodiments, the present disclosure further provides a nucleic acid molecule encoding the anti-TF antibody according to any one of the above.

In some embodiments, the present disclosure further provides a host cell comprising the nucleic acid molecule described above.

In some embodiments, the present disclosure further provides an immunoconjugate comprising the anti-TF antibody according to any one of the above and an effector molecule, wherein the effector molecule is conjugated to the anti-TF antibody according to any one of the above; preferably, the effector molecule is selected from the group consisting of an anti-tumor agent, an immunomodulator, a biological response modifier, a lectin, a cytotoxic drug, a chromophore, a fluorophore, a chemiluminescent compound, an enzyme, a metal ion, and any combination thereof.

In some embodiments, the present disclosure further provides a method for immunodetection or determination of TF *in vivo* and/or *in vitro,* and the method comprises a step of allowing the anti-TF antibody according to any one of the above to be in contact with a subject or a sample from the subject.

In some embodiments, the present disclosure further provides an antibody-drug conjugate of general formula (Pc-L-Y-D) or a pharmaceutically acceptable salt thereof: 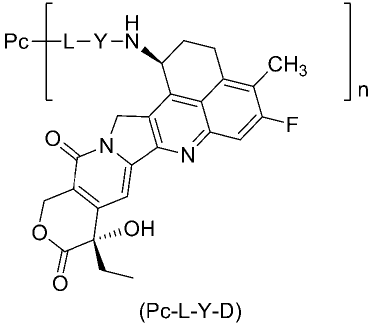 wherein:
Y is -O-(CR^{a}R^{b})ₘ-CR¹R²-C(O)-;
wherein R^{a} and R^{b} are identical or different and are each independently selected from the group consisting of a hydrogen atom, a deuterium atom, halogen, and C₁₋₆ alkyl;
R¹ is C₃₋₆ cycloalkyl-C₁₋₆ alkyl or C₃₋₆ cycloalkyl;
R² is selected from the group consisting of a hydrogen atom, C₁₋₆ haloalkyl, and C₃₋₆ cycloalkyl; or
R¹ and R², together with the carbon atom to which they are attached, form C₃₋₆ cycloalkyl; m is an integer from 0 to 4;
L is a linker unit;
n is 1 to 10; preferably, n is 1 to 8; more preferably, n is 2 to 8; most preferably, n is 4 to 8;
Pc is the anti-TF antibody according to any one of the above.

In some embodiments, n is an average value from 0-10 (including decimals and integers), preferably 1-10, more preferably 1-8, or 2-8, or 2-7, or 2-6, or 2-5, or 2-4, or 3-8, or 3-7, or 3-6, or 4-8, or 4-7, or 4-6, or 4-5; in some embodiments, n is an average value, such as 1, 2, 3, 4, 5, 6, 7, 8, 9, or 10.

In some embodiments, R^{a} and R^{b} are identical or different and are each independently selected from the group consisting of a hydrogen atom, halogen, and C₁₋₆ alkyl; preferably, R^{a} and R^{b} are identical or different and are each independently a hydrogen atom or C₁₋₆ alkyl.

In some embodiments, R¹ is C₃₋₆ cycloalkyl; preferably, R¹ is cyclopropyl.

In some embodiments, R² is a hydrogen atom or C₁₋₆ haloalkyl; preferably, R² is a hydrogen atom.

In some embodiments, R¹ and R², together with the carbon atom to which they are attached, form cyclopropyl.

In some embodiments, R¹ is C₃₋₆ cycloalkyl, and R² is a hydrogen atom or C₁₋₆ haloalkyl; preferably, R¹ is cyclopropyl, and R² is a hydrogen atom.

In some embodiments, provided is the antibody-drug conjugate of general formula (Pc-L-Y-D) or the pharmaceutically acceptable salt thereof described above, wherein the linker unit -L- is -L¹-L²-L³-L⁴-, wherein
L¹ is selected from the group consisting of -(succinimid-3-yl-*N*)-W-C(O)-, -CH₂-C(O)-NR³-W-C(O)-, and -C(O)-W-C(O)-, wherein W is selected from the group consisting of C₁₋₆ alkylene and C₁₋₆ alkylene-C₃₋₆ cycloalkyl, wherein the C₁₋₆ alkylene or C₁₋₆ alkylene-C₃₋₆ cycloalkyl is each independently optionally substituted with one or more substituents selected from the group consisting of halogen, hydroxy, cyano, amino, C₁₋₆ alkyl, C₁₋₆ haloalkyl, deuterated C₁₋₆ alkyl, C₁₋₆ alkoxy, and C₃₋₆ cycloalkyl;
L² is selected from the group consisting of -NR⁴(CH₂CH₂O)p¹CH₂CH₂C(O)-, - NR⁴(CH₂CH₂O)p^{l}CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, chloroalkyl, deuterated alkyl, alkoxy, and cycloalkyl;
L⁴ is selected from the group consisting of -NR⁵(CR⁶R⁷)ₜ-, -C(O)NR⁵-, -C(O)NR⁵(CH₂)ₜ-, and a chemical bond, wherein t is an integer from 1 to 6;
R³, R⁴, and R⁵ are identical or different and are each independently selected from the group consisting of a hydrogen atom, C₁₋₆ alkyl, C₁₋₆ haloalkyl, deuterated C₁₋₆ alkyl, and C₁₋₆ hydroxyalkyl;
R⁶ and R⁷ are identical or different and are each independently selected from the group consisting of a hydrogen atom, halogen, C₁₋₆ alkyl, C₁₋₆ haloalkyl, deuterated C₁₋₆ alkyl, and C₁₋₆ hydroxyalkyl.

In some embodiments, L¹ is selected from the group consisting of -(succinimid-3-yl-*N*)-W-C(O)-, wherein W is selected from the group consisting of C₁₋₆ alkylene and C₁₋₆ alkylene-C₃₋₆ cycloalkyl; preferably, L¹ is 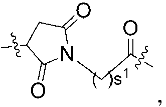 wherein s¹ is an integer from 2 to 8; more preferably, L¹ is 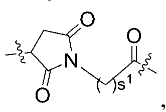 wherein s¹ is 5.

In some embodiments, L ² is a chemical bond.

In some embodiments, L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid; preferably, L³ is a tetrapeptide residue; more preferably, L³ is a tetrapeptide residue of GGFG (SEQ ID NO: 105).

In some embodiments, L⁴ is selected from the group consisting of -NR⁵(CR⁶R⁷)ₜ-, wherein t is an integer from 1 to 6; R⁵, R⁶, and R⁷ are identical or different and are each independently a hydrogen atom or C₁₋₆ alkyl; preferably, L⁴ is -NR⁵ (CR ⁶R⁷)ₜ-, wherein R⁵, R⁶, and R⁷ are identical or different and are each independently a hydrogen atom or C₁₋₆ alkyl, and t is 1 or 2.

In some embodiments, provided is the antibody-drug conjugate of general formula (Pc-L-Y-D) or the pharmaceutically acceptable salt thereof described above, wherein the linker unit -L- is -L¹-L²-L³-L⁴-, wherein
L¹ is 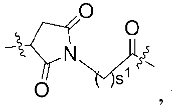 wherein s¹ is an integer from 2 to 8;
L² is a chemical bond;
L³ is a tetrapeptide residue; preferably, L³ is a tetrapeptide residue of GGFG (SEQ ID NO: 105);
L⁴ is -NR⁵(CR⁶R⁷)ₜ-, wherein R⁵, R⁶, and R⁷ are identical or different and are each independently a hydrogen atom or C₁₋₆ alkyl, and t is 1 or 2;
the L¹ terminus of -L- is connected to Pc, and the L⁴ terminus is connected to Y.

In some embodiments, provided is the antibody-drug conjugate of general formula (Pc-L-Y-D) or the pharmaceutically acceptable salt thereof according to any one of the above, which is an antibody-drug conjugate of general formula (Pc-Lₐ-Y-D) or a pharmaceutically acceptable salt thereof: 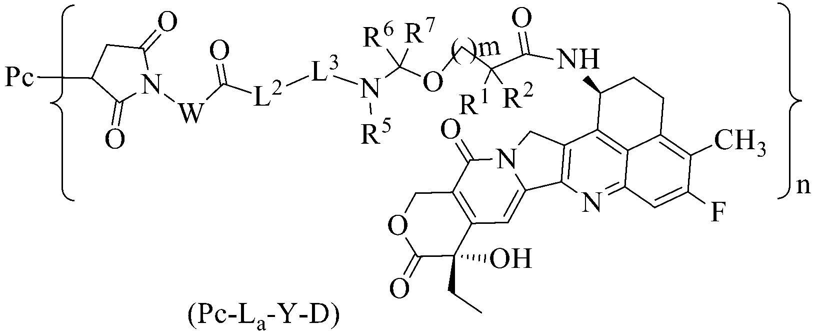 wherein:
Pc is the anti-TF antibody according to any one of the above;
m is an integer from 0 to 4;
n is 1 to 10;
R¹ is C₃₋₆ cycloalkyl-C₁₋₆ alkyl or C₃₋₆ cycloalkyl;
R² is selected from the group consisting of a hydrogen atom, C₁₋₆ haloalkyl, and C₃₋₆ cycloalkyl; or
R¹ and R², together with the carbon atom to which they are attached, form C₃₋₆ cycloalkyl; W is selected from the group consisting of C₁₋₆ alkylene and C₁₋₆ alkylene-C₃₋₆ cycloalkyl, wherein the C₁₋₆ alkylene and C₁₋₆ alkylene-C₃₋₆ cycloalkyl are each independently optionally substituted with one or more substituents selected from the group consisting of halogen, hydroxy, cyano, amino, alkyl, chloroalkyl, deuterated alkyl, alkoxy, and cycloalkyl;
L² is selected from the group consisting of -NR⁴(CH₂CH₂O)p¹CH₂CH₂C(O)-, - NR⁴(CH₂CH₂O)p^{l}CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, chloroalkyl, deuterated alkyl, alkoxy, and cycloalkyl;
R⁴ and R⁵ are selected from the group consisting of a hydrogen atom, alkyl, haloalkyl, deuterated alkyl, and hydroxyalkyl;
R⁶ and R⁷ are identical or different and are each independently selected from the group consisting of a hydrogen atom, halogen, alkyl, haloalkyl, deuterated alkyl, and hydroxyalkyl.

In some embodiments, provided is the antibody-drug conjugate of general formula (Pc-L-Y-D) or the pharmaceutically acceptable salt thereof according to any one of the above, which is an antibody-drug conjugate of general formula (Pc-9-A) or a pharmaceutically acceptable salt thereof: 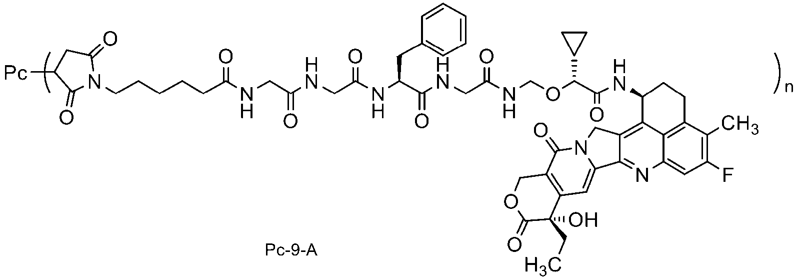 wherein:
n is 1 to 8;
Pc is the anti-TF antibody according to any one of the above;
preferably, the Pc is selected from the group consisting of anti-TF antibodies comprising a heavy chain variable region and a light chain variable region according to any one of the following:
   a. the heavy chain variable region comprises a HCDR1, a HCDR2, and a HCDR3 set forth in SEQ ID NO: 40, SEQ ID NO: 64, and SEQ ID NO: 65, respectively; the light chain variable region comprises a LCDR1, a LCDR2, and a LCDR3 set forth in SEQ ID NO: 66, SEQ ID NO: 67, and SEQ ID NO: 68, respectively; or
      the heavy chain variable region comprises a HCDR1, a HCDR2, and a HCDR3 set forth in SEQ ID NO: 40, SEQ ID NO: 64, and SEQ ID NO: 65, respectively; the light chain variable region comprises a LCDR1, a LCDR2, and a LCDR3 set forth in SEQ ID NO: 66, SEQ ID NO: 67, and SEQ ID NO: 83, respectively; or
      the heavy chain variable region comprises a HCDR1, a HCDR2, and a HCDR3 set forth in SEQ ID NO: 40, SEQ ID NO: 41, and SEQ ID NO: 42, respectively; the light chain variable region comprises a LCDR1, a LCDR2, and a LCDR3 set forth in SEQ ID NO: 43, SEQ ID NO: 44, and SEQ ID NO: 45, respectively;
   b. the heavy chain variable region comprises a HCDR1, a HCDR2, and a HCDR3 set forth in SEQ ID NO: 46, SEQ ID NO: 47, and SEQ ID NO: 48, respectively; the light chain variable region comprises a LCDR1, a LCDR2, and a LCDR3 set forth in SEQ ID NO: 49, SEQ ID NO: 50, and SEQ ID NO: 51, respectively;
   c. the heavy chain variable region comprises a HCDR1, a HCDR2, and a HCDR3 set forth in SEQ ID NO: 73, SEQ ID NO: 74, and SEQ ID NO: 75, respectively; the light chain variable region comprises a LCDR1, a LCDR2, and a LCDR3 set forth in SEQ ID NO: 76, SEQ ID NO: 77, and SEQ ID NO: 78, respectively;
more preferably, the Pc is selected from the group consisting of anti-TF antibodies comprising a heavy chain variable region and a light chain variable region according to any one of the following:
   a. the heavy chain variable region is set forth in SEQ ID NO: 62, and the light chain variable region is set forth in SEQ ID NO: 63; or
      the heavy chain variable region is set forth in SEQ ID NO: 62, and the light chain variable region is set forth in SEQ ID NO: 81; or
      the heavy chain variable region is set forth in SEQ ID NO: 58, and the light chain variable region is set forth in SEQ ID NO: 59; or
      the heavy chain variable region is set forth in SEQ ID NO: 36, and the light chain variable region is set forth in SEQ ID NO: 37;
   b. the heavy chain variable region is set forth in SEQ ID NO: 52, and the light chain variable region is set forth in SEQ ID NO: 53; or
      the heavy chain variable region is set forth in SEQ ID NO: 38, and the light chain variable region is set forth in SEQ ID NO: 39;
   c. the heavy chain variable region is set forth in SEQ ID NO: 71, and the light chain variable region is set forth in SEQ ID NO: 72;
most preferably, the Pc is selected from the group consisting of antibodies comprising a heavy chain and a light chain according to any one of the following:
   a. the heavy chain is set forth in SEQ ID NO: 69, and the light chain is set forth in SEQ ID NO: 70; or
      the heavy chain is set forth in SEQ ID NO: 69, and the light chain is set forth in SEQ ID NO: 82; or
      the heavy chain is set forth in SEQ ID NO: 60, and the light chain is set forth in SEQ ID NO: 61;
   b. the heavy chain is set forth in SEQ ID NO: 56, and the light chain is set forth in SEQ ID NO: 57;
   c. the heavy chain is set forth in SEQ ID NO: 79; and the light chain is set forth in SEQ ID NO: 80.

In some embodiments, provided is the antibody-drug conjugate of general formula (Pc-L-Y-D) or the pharmaceutically acceptable salt thereof according to any one of the above, which is an antibody-drug conjugate of general formula (Pc-9-A) or a pharmaceutically acceptable salt thereof: 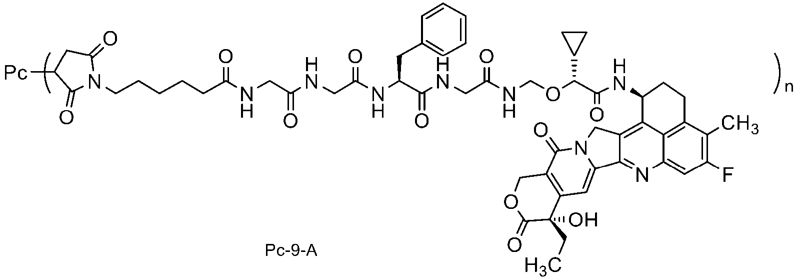 wherein:
n is 1 to 8; preferably, n is 2 to 8; more preferably, n is 4 to 8;
Pc is the anti-TF antibody according to any one of the above;
preferably, the Pc is selected from the group consisting of anti-TF antibodies comprising a heavy chain variable region and a light chain variable region according to any one of the following:
   a. the heavy chain variable region comprises a HCDR1, a HCDR2, and a HCDR3 set forth in SEQ ID NO: 40, SEQ ID NO: 64, and SEQ ID NO: 65, respectively; the light chain variable region comprises a LCDR1, a LCDR2, and a LCDR3 set forth in SEQ ID NO: 66, SEQ ID NO: 67, and SEQ ID NO: 68, respectively; or
   b. the heavy chain variable region comprises a HCDR1, a HCDR2, and a HCDR3 set forth in SEQ ID NO: 46, SEQ ID NO: 47, and SEQ ID NO: 48, respectively; the light chain variable region comprises a LCDR1, a LCDR2, and a LCDR3 set forth in SEQ ID NO: 49, SEQ ID NO: 50, and SEQ ID NO: 51, respectively; or
   c. the heavy chain variable region comprises a HCDR1, a HCDR2, and a HCDR3 set forth in SEQ ID NO: 73, SEQ ID NO: 74, and SEQ ID NO: 75, respectively; the light chain variable region comprises a LCDR1, a LCDR2, and a LCDR3 set forth in SEQ ID NO: 76, SEQ ID NO: 77, and SEQ ID NO: 78, respectively; or
more preferably, the Pc is selected from the group consisting of anti-TF antibodies comprising a heavy chain variable region and a light chain variable region according to any one of the following:
   a. the heavy chain variable region is set forth in SEQ ID NO: 62, and the light chain variable region is set forth in SEQ ID NO: 63;
   b. the heavy chain variable region is set forth in SEQ ID NO: 52, and the light chain variable region is set forth in SEQ ID NO: 53; and
   c. the heavy chain variable region is set forth in SEQ ID NO: 71, and the light chain variable region is set forth in SEQ ID NO: 72;
most preferably, the Pc is selected from the group consisting of antibodies comprising a heavy chain and a light chain according to any one of the following:
   a. the heavy chain is set forth in SEQ ID NO: 69, and the light chain is set forth in SEQ ID NO: 70; or
   b. the heavy chain is set forth in SEQ ID NO: 56, and the light chain is set forth in SEQ ID NO: 57; or
   c. the heavy chain is set forth in SEQ ID NO: 79; and the light chain is set forth in SEQ ID NO: 80.

In some embodiments, provided is the antibody-drug conjugate of general formula (Pc-L-Y-D) or the pharmaceutically acceptable salt thereof according to any one of the above, which is an antibody-drug conjugate of general formula (Pc-9-A) or a pharmaceutically acceptable salt thereof: 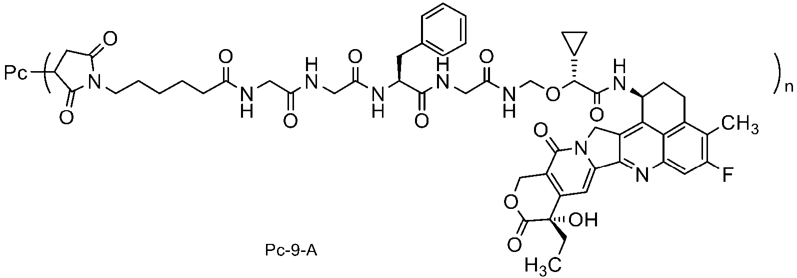 wherein:
n is 1 to 8; preferably, n is 2 to 8; more preferably, n is 4 to 8;
Pc is an anti-TF antibody comprising a heavy chain variable region and a light chain variable region, wherein the heavy chain variable region comprises a HCDR1, a HCDR2, and a HCDR3 set forth in SEQ ID NO: 40, SEQ ID NO: 64, and SEQ ID NO: 65, respectively, and the light chain variable region comprises a LCDR1, a LCDR2, and a LCDR3 set forth in SEQ ID NO: 66, SEQ ID NO: 67, and SEQ ID NO: 68, respectively.

In some embodiments, provided is the antibody-drug conjugate of general formula (Pc-L-Y-D) or the pharmaceutically acceptable salt thereof according to any one of the above, which is an antibody-drug conjugate of general formula (Pc-9-A) or a pharmaceutically acceptable salt thereof: 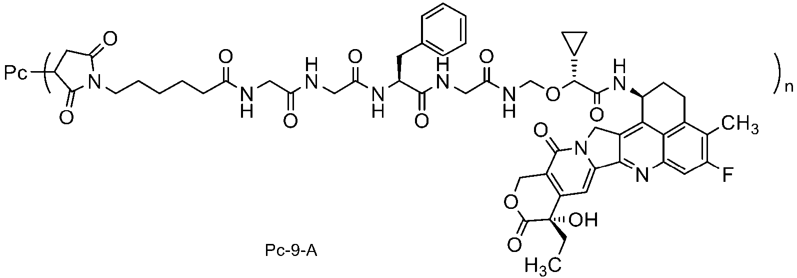 wherein:
n is 1 to 8; preferably, n is 2 to 8; more preferably, n is 4 to 8;
Pc is an anti-TF antibody comprising a heavy chain variable region and a light chain variable region, wherein the heavy chain variable region is set forth in SEQ ID NO: 62, and the light chain variable region is set forth in SEQ ID NO: 63.

In some embodiments, provided is the antibody-drug conjugate of general formula (Pc-L-Y-D) or the pharmaceutically acceptable salt thereof according to any one of the above, which is an antibody-drug conjugate of general formula (Pc-9-A) or a pharmaceutically acceptable salt thereof: 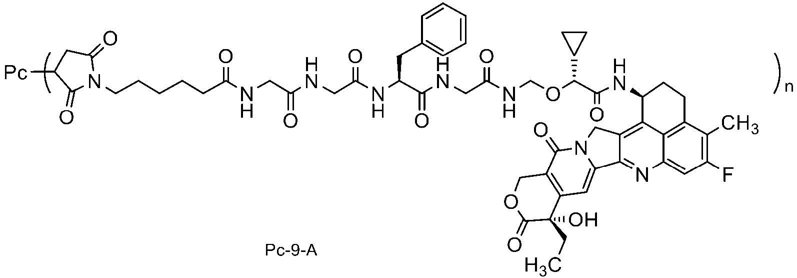 wherein:
n is 1 to 8; preferably, n is 2 to 8; more preferably, n is 4 to 8;
Pc is an anti-TF antibody comprising a heavy chain and a light chain, wherein the heavy chain is set forth in SEQ ID NO: 69, and the light chain is set forth in SEQ ID NO: 70. In some embodiments, the present disclosure further provides a method for preparing an antibody-drug conjugate of general formula (Pc-Lₐ-Y-D) or a pharmaceutically acceptable salt thereof, which comprises the following step: 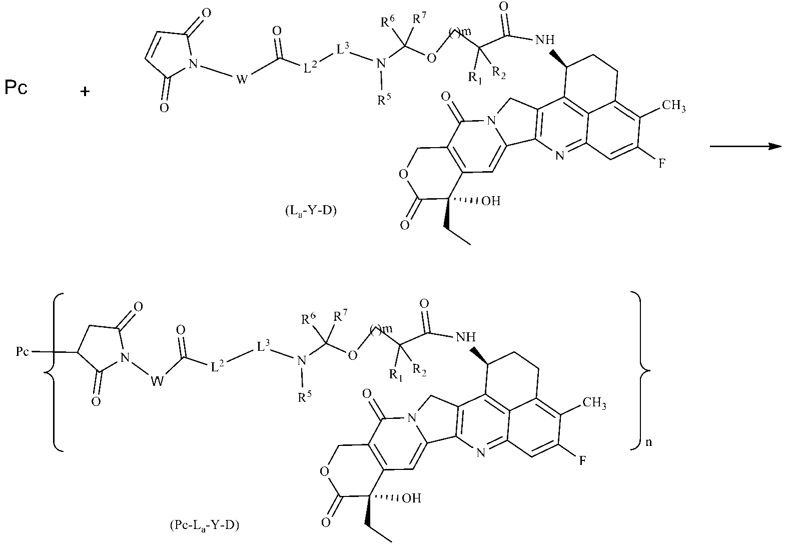 subjecting reduced Pc to a coupling reaction with a compound of general formula (Lₐ-Y-D) to give the antibody-drug conjugate of general formula (Pc-Lₐ-Y-D) or the pharmaceutically acceptable salt thereof;
wherein:
   Pc is the anti-TF antibody according to any one of the above;
   m is an integer from 0 to 4;
   n is 1 to 10;
   R¹ is C₃₋₆ cycloalkyl-C₁₋₆ alkyl or C₃₋₆ cycloalkyl;
   R² is selected from the group consisting of a hydrogen atom, C₁₋₆ haloalkyl, and C₃₋₆ cycloalkyl; or
   R¹ and R², together with the carbon atom to which they are attached, form C₃₋₆ cycloalkyl; W is selected from the group consisting of C₁₋₆ alkylene and C₁₋₆ alkylene-C₃₋₆ cycloalkyl, wherein the C₁₋₆ alkylene and C₁₋₆ alkylene-C₃₋₆ cycloalkyl are each independently optionally substituted with one or more substituents selected from the group consisting of halogen, hydroxy, cyano, amino, alkyl, chloroalkyl, deuterated alkyl, alkoxy, and cycloalkyl;
   L² is selected from the group consisting of -NR⁴(CH₂CH₂O)p¹CH₂CH₂C(O)-, - NR⁴(CH₂CH₂O)p^{l}CH₂C(O)-, -S(CH₂)p¹C(O)-, and a chemical bond, wherein p¹ is an integer from 1 to 20;
   L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, chloroalkyl, deuterated alkyl, alkoxy, and cycloalkyl;
   R⁴ and R⁵ are selected from the group consisting of a hydrogen atom, alkyl, haloalkyl, deuterated alkyl, and hydroxyalkyl;
   R⁶ and R⁷ are identical or different and are each independently selected from the group consisting of a hydrogen atom, halogen, alkyl, haloalkyl, deuterated alkyl, and hydroxyalkyl.

In some embodiments, the present disclosure further provides a method for preparing an antibody-drug conjugate of general formula (Pc-9-A) or a pharmaceutically acceptable salt thereof, which comprises the following step: 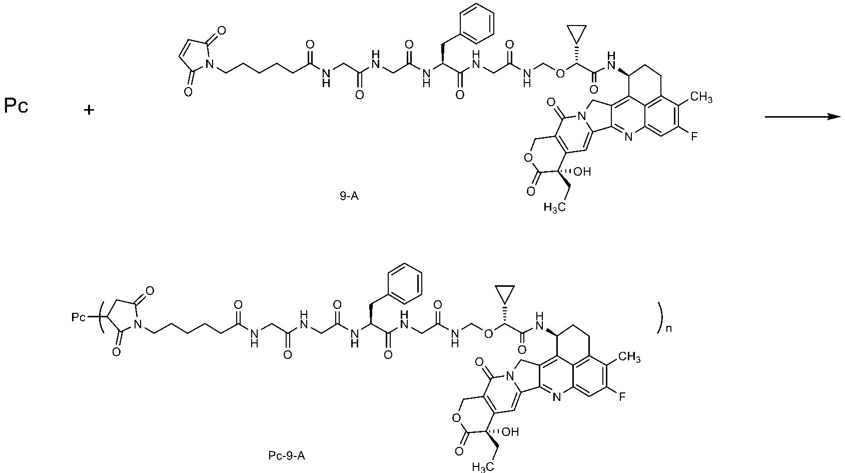
subjecting reduced Pc to a coupling reaction with a compound 9-A to give the antibody-drug conjugate of general formula (Pc-9-A) or the pharmaceutically acceptable salt thereof;
wherein:
   n is 1 to 8; preferably, n is 2 to 8; more preferably, n is 4 to 8;
   Pc is the anti-TF antibody according to any one of the above.

In some embodiments, the present disclosure further provides a pharmaceutical composition, which comprises the anti-TF antibody according to any one of the above, or the nucleic acid molecule described above, or the antibody-drug conjugate or the pharmaceutically acceptable salt thereof according to any one of the above, and one or more pharmaceutically acceptable excipients, diluents, or carriers.

In some embodiments, the present disclosure further provides use of the anti-TF antibody according to any one of the above, or the nucleic acid molecule described above, or the antibody-drug conjugate or the pharmaceutically acceptable salt thereof according to any one of the above, or the pharmaceutical composition described above, in the manufacture of a drug for treating a TF-mediated disease or disorder.

In some embodiments, the present disclosure further provides use of the anti-TF antibody according to any one of the above, or the nucleic acid molecule described above, or the antibody-drug conjugate or the pharmaceutically acceptable salt thereof according to any one of the above, or the pharmaceutical composition described above, in the manufacture of a drug for treating a tumor or cancer.

In some embodiments, the present disclosure further provides use of the anti-TF antibody according to any one of the above, or the nucleic acid molecule described above, or the antibody-drug conjugate or the pharmaceutically acceptable salt thereof according to any one of the above, or the pharmaceutical composition described above, in the manufacture of a drug for treating a tumor or cancer; preferably, the tumor or cancer is selected from the group consisting of breast cancer, pancreatic cancer, lung cancer (including non-small cell lung cancer and small cell lung cancer), esophageal cancer, ovarian cancer, cervical cancer, endometrial cancer, prostate cancer, bladder cancer, fallopian tube cancer, peritoneal cancer, colorectal cancer (including colon cancer and rectal cancer), head and neck cancer, and squamous cell carcinoma.

In some embodiments, the present disclosure further provides a kit, which comprises the anti-TF antibody according to any one of the above, or the nucleic acid molecule described above, or the antibody-drug conjugate or the pharmaceutically acceptable salt thereof according to any one of the above, or the pharmaceutical composition described above.

In some embodiments, in the antibody-drug conjugate of general formula (Pc-L-Y-D) or the pharmaceutically acceptable salt thereof according to any one of the above, m is selected from the group consisting of, but is not limited to, 0, 1, 2, 3, and 4.

In some embodiments, the present disclosure further provides a method for preventing or treating a disease or disorder, which comprises administering to a subject a therapeutically effective amount of the anti-TF antibody according to any one of the above, or the nucleic acid molecule according to any one of the above, or the antibody-drug conjugate or the pharmaceutically acceptable salt thereof according to any one of the above, or the pharmaceutical composition according to any one of the above. In some embodiments, the disease or disorder is a tumor or cancer; preferably, the disease or disorder is a disease or disorder associated with TF. In some embodiments, the tumor or cancer is selected from the group consisting of breast cancer, pancreatic cancer, lung cancer (including non-small cell lung cancer and small cell lung cancer), esophageal cancer, ovarian cancer, cervical cancer, endometrial cancer, prostate cancer, bladder cancer, fallopian tube cancer, peritoneal cancer, colorectal cancer (including colon cancer and rectal cancer), head and neck cancer, and squamous cell carcinoma.

In another aspect, the present disclosure provides a pharmaceutical composition, which comprises the anti-TF antibody, the antibody-drug conjugate or the pharmaceutically acceptable salt thereof according to any one of the above, and one or more pharmaceutically acceptable excipients, diluents, or carriers. In some embodiments, the pharmaceutical composition in a unit dose comprises 0.1-3000 mg or 1-1000 mg of the anti-TF antibody described above or the antibody-drug conjugate described above.

In another aspect, the present disclosure provides the antibody-drug conjugate or the pharmaceutically acceptable salt thereof or the pharmaceutical composition comprising the same according to any one of the above for use as a drug.

In another aspect, the present disclosure provides use of the antibody-drug conjugate or the pharmaceutically acceptable salt thereof or the pharmaceutical composition comprising the same according to any one of the above in a drug for treating a TF-mediated disease or disorder; in some embodiments, the TF-mediated disease or disorder is a cancer with high, moderate, or low TF expression. In some embodiments, the antibody-drug conjugate or the pharmaceutically acceptable salt thereof or the pharmaceutical composition comprising the same according to any one of the above is used for treating a tumor or cancer.

In another aspect, the present disclosure further relates to a method for treating and/or preventing a tumor or cancer, which comprises administering to a subject in need thereof a therapeutically effective dose of the antibody-drug conjugate or the pharmaceutically acceptable salt thereof or the pharmaceutical composition comprising the same according to any one of the above; in some embodiments, the tumor or cancer is a cancer associated with high, moderate, or low TF expression.

The active compound may be made into a form suitable for administration by any suitable route. The active compound may be in the form of a unit dose, or in the form of a single dose that can be self-administered by a subject. The unit dose of the compound or composition of the present disclosure may be expressed in the form of a tablet, a capsule, a cachet, a vial, a powder, a granule, a lozenge, a suppository, a regenerating powder, or a liquid formulation.

The dose of the compound or composition used in the treatment method of the present disclosure generally varies depending on the severity of the disease, the weight of the subject, and the relative efficacy of the compound. However, as a general instruction, a suitable unit dose may be 0.1 mg to 1000 mg.

The pharmaceutical composition of the present disclosure may comprise, in addition to the active compound, one or more auxiliary materials selected from the group consisting of a filler, a diluent, a binder, a wetting agent, a disintegrant, an excipient, and the like. Depending on the method of administration, the composition may comprise 0.1-99 wt.% of the active compound.

The anti-TF antibody provided by the present disclosure exhibits a good affinity for a cell surface antigen and good endocytosis efficiency and basically has no effect on blood coagulation; the anti-TF antibody-drug conjugate provided by the present disclosure exhibits very high tumor-inhibiting efficiency and shows better efficacy and lower toxic and side effects in animals.

Epitope analysis was performed through FACS binding experiments. The antibodies of the present disclosure bind to a specific epitope of TF. In particular, the antibody hu67Y116 binds to an epitope of TF different from the epitope of TF bound by antibodies in the prior art.

### Detailed Description of the Invention

### Terminology

Unless otherwise defined, all technical and scientific terms used herein have the same meaning as commonly understood by those of ordinary skill in the art to which the present disclosure belongs. Although any methods and materials similar or equivalent to those described herein can also be used to implement or test the present disclosure, preferred methods and materials are described herein. In describing and claiming the present disclosure, the following terms are used in accordance with the definitions below. When a trade name is used in the present disclosure, it is intended to include the formulation of the commercial product under the trade name, and the drug and active drug component of the commercial product under the trade name.

Unless otherwise stated, the terms used in the specification and claims have the following meanings.

The three-letter and single-letter codes for amino acids used herein are as described in J. biol. chem, 243, p3558 (1968).

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a structure different from the general chemical structure of amino acids, but function in a manner similar to naturally occurring amino acids.

The term "antibody" is used herein in the broadest sense and encompasses a variety of antibody structures, including but not limited to monoclonal antibodies, polyclonal antibodies, multispecific antibodies (e.g., bispecific antibodies), and full-length antibodies, and antibody fragments (or antigen-binding fragments, or antigen-binding moieties) so long as they exhibit the desired antigen binding activity.

"Native antibody" refers to a naturally occurring immunoglobulin molecule. For example, a natural IgG antibody is a heterotetrameric glycoprotein of about 150,000 Daltons composed of two identical light chains and two identical heavy chains linked by a disulfide bond. From N-terminus to C-terminus, each heavy chain has one variable region (VH), also known as variable heavy domain or heavy chain variable domain, followed by three constant domains (CH1, CH2, and CH3). Similarly, from the N-terminus to the C-terminus, each light chain has one variable region (VL), also known as variable light domain or light chain variable domain, followed by one constant light domain (also known as a light chain constant region, CL).

The term "full-length antibody", "intact antibody", and "whole antibody" are used herein interchangeably and refer to an antibody comprising a substantially similar structure to a native antibody structure or whose heavy chains have an Fc region as defined herein. An "isolated" antibody is one that has been separated from components of its natural environment. In the present disclosure, in some embodiments, the antibody may be purified to a purity of greater than 90% or a purity of 99%. In some embodiments, the antibody is purified and assayed by methods such as electrophoresis (e.g., SDS-PAGE, isoelectric focusing (IEF), or capillary electrophoresis) or chromatography (e.g., ion exchange or reversed-phase HPLC).

The term "variable region" or "variable domain" refers to a domain in antibody heavy and/or light chains that is involved in the binding of the antibody to an antigen. The VH and VL of a native IgG antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues in variable domains. A VH comprises 3 CDRs: HCDR1, HCDR2, and HCDR3; a VL comprises 3 CDRs: LCDR1, LCDR2, and LCDR3. Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships among the various numbering schemes are well known to those skilled in the art and are, illustratively, as shown in Table 1 below.

**Table 1. Relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The term "light chain" includes the variable region domain VL and the constant region domain CL. VL is at the amino terminus of a light chain. Light chains include κ and λ chains.

The term "heavy chain" includes the variable region domain VH and the three constant region domains CH1, CH2, and CH3. VH is at the amino terminus of a heavy chain and the CH domains are at the carboxyl terminus, with CH3 being closest to the carboxyl terminus of a polypeptide. A heavy chain may be of any isotype, including IgG (including IgG1, IgG2, IgG3, and IgG4 subtypes), IgA (including IgA1 and IgA2 subtypes), IgM, and IgE.

The term "antibody fragment" refers to a molecule different from an intact antibody, which comprises a moiety of an intact antibody that binds to an antigen to which the intact antibody binds. Antibody fragments include, but are not limited to, Fv, Fab, Fab', Fab'-SH, F(ab')₂, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native sequence Fc regions and modified Fc regions. In some embodiments, the Fc region of the human IgG heavy chain is defined as extending from the amino acid residue at position Cys226 or from Pro230 to its carboxyl terminus. The boundaries of the Fc region of the heavy chain of an antibody may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Thus, in some embodiments, a composition of intact antibodies may comprise antibody populations with all K447 residues and/or G446 + K447 residues removed. In some embodiments, a composition of intact antibodies may comprise antibody populations with K447 residues and/or G446 + K447 residues unremoved. In some embodiments, a composition of intact antibodies comprises antibody populations having a mixture of antibodies with and without a K447 residue and/or G446 + K447 residues. Suitable native sequence Fc regions for the antibodies described herein include human IgG1, IgG2 (IgG2A and IgG2B), IgG3, and IgG4. Unless otherwise specified herein, the numbering of amino acid residues in the Fc region or constant region conforms to the EU numbering scheme, also known as the EU index, as described in Kabat et al., Sequences of Proteins of Immunological Interest, 5th edition, Public Health Service, National Institutes of Health, Bethesda, MD, 1991.

The term "chimeric antibody" refers to an antibody in which a portion of the heavy and/or light chains in the antibody is derived from a particular source or species, while the remainder of the heavy and/or light chains is derived from a different source or species. The term "humanized antibody" is an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions).

The term "human antibody" refers to an antibody in which the variable and constant regions are human sequences. The term encompasses antibodies that are derived from human genes but have, for example, sequences that have been altered to, e.g., reduce possible immunogenicity, increase affinity, and eliminate cysteines or glycosylation sites that may cause undesired folding. The term encompasses antibodies recombinantly produced in non-human cells (that may confer glycosylation not characteristic of human cells). The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "affinity-matured" antibody refers to an antibody with one or more alterations in one or more CDRs thereof which result in an improvement in the affinity of the antibody for antigens, compared to the parent antibody. In some embodiments, an affinity-matured antibody has nanomolar or even picomolar affinity for the target antigen. Affinity-matured antibodies can be produced using methods known in the art. Marks et al., Bio/Technology 10:779-783 (1992) described affinity maturation by VH and VL domain shuffling. The following documents describe random mutagenesis of CDR and/or framework residues: Barbas et al., PNAS, 91:3809-3813 (1994); Schier et al., Gene 169:147-155 (1995); Yelton et al., J.Immunol. 155:1994-2004 (1995); Jackson et al., J.Immunol. 154 (7):3310-9 (1995) and Hawkins et al., J. Mol. Biol. 226:889-896 (1992). The term "monoclonal antibody" refers to a population of substantially homogeneous antibodies, that is, the amino acid sequences of the antibody molecules comprised in the population are identical, except for a small number of natural mutations that may exist. In contrast, a polyclonal antibody formulation generally comprises several different antibodies having different amino acid sequences in their variable domains, which are generally specific for different epitopes. "Monoclonal" refers to the characteristics of an antibody obtained from a substantially homogeneous antibody population and should not be construed as requiring the production of the antibody by any particular method. For example, monoclonal antibodies can be prepared by the hybridoma method described in Kohler et al., (1975) Nature 256:495, or by recombinant DNA methods (see, e.g., U.S. Pat. No. 4,816,567). Alternatively, "monoclonal antibodies" can be isolated from phage antibody libraries using the techniques described in Clackson et al., (1991) Nature 352: 624-628 and Marks et al., (1991) J. Mol. Biol. 222:581-597. Reference may also be made to Presta (2005) J. Allergy Clin. Immunol. 116:731, or "monoclonal antibodies" can be prepared using methods utilizing transgenic animals containing all or part of the human immunoglobulin loci.

The term "antigen" refers to a molecule or a molecular moiety that is capable of being selectively bound by an antibody and is capable of being used in an animal to produce an antibody that is capable of binding to the antigen. An antigen may have one or more epitopes capable of interacting with different antibodies.

The term "epitope" refers to an area or region on an antigen that is capable of specifically binding to an antibody. Epitopes can be formed from contiguous strings of amino acids (linear epitope) or comprise non-contiguous amino acids (conformational epitope), e.g., coming in spatial proximity due to the folding of the antigen (i.e., by the tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation.

Screening for antibodies that bind to particular epitopes (i.e., those that bind to identical epitopes) can be performed using methods well known in the art, including but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

"An antibody that binds to the same epitope" as a reference antibody or "an antibody that competes for binding with" a reference antibody refers to an antibody that blocks binding of the reference antibody to an antigen by 50% or more, or an antibody whose binding to an antigen is blocked by 50% or more by the reference antibody, in a competition assay. For example, to determine whether the test antibody binds to the same epitope as the reference antibody, the reference antibody is allowed to bind to the antigen under saturating conditions. After removal of excess reference antibody, the ability of the test antibody to bind to the antigen is assessed. To confirm whether the test antibody binds to the same epitope or is just hampered from binding by steric reasons, conventional experimentation can be used (e.g., peptide mutation and binding analyses using ELISA, RIA, surface plasmon resonance, flow cytometry or any other quantitative or qualitative antibody-binding assay available in the art). This assay should be carried out in two set-ups, i.e. with both of the antibodies being saturating antibodies. If, in both set-ups, only the first (saturating) antibody is capable of binding to the antigen, then it can be concluded that the test antibody and the reference antibody compete for binding to the antigen.

In some embodiments, two antibodies are considered to bind to the same epitope or an overlapping epitope if a 1-, 5-, 10-, 20-, or 100-fold excess of one antibody inhibits binding of the other by at least 50%, at least 75%, at least 90% or even 99% or more as measured in a competitive binding assay (see, e.g., Junghans et al., Cancer Res. 50 (1990) 1495-1502).

In some embodiments, two antibodies are considered to bind to the "same epitope" if essentially all amino acid mutations in the antigen that reduce or eliminate binding of one antibody also reduce or eliminate binding of the other. Two antibodies are considered to have "overlapping epitopes" if only some of the mutations reduce or eliminate binding of the other.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the 1:1 interaction between members of a binding pair (e.g., an antibody and an antigen). The affinity of molecule X for its ligand Y can be generally expressed by the equilibrium dissociation constant (KD). Affinity can be determined by conventional methods known in the art, including those described herein. As used herein, the term "kassoc" or "ka" refers to the association rate of a particular antibody-antigen interaction, while the term "kdis" or "kd" refers to the dissociation rate of a particular antibody-antigen interaction. The term "KD" refers to the equilibrium dissociation constant, which is obtained from the ratio of kd to ka (i.e., kd/ka) and denoted by a molar concentration (M). The KD value of an antibody can be determined using methods well known in the art. For example, surface plasmon resonance is determined using a biosensing system such as a system, or affinity in a solution is determined by solution equilibrium titration (SET).

The term "specifically bind", "specific binding", or "bind" refers to antibody binding to an antigen or an epitope within the antigen with greater affinity than to other antigens or epitopes. Typically, the antibody binds to the antigen or the epitope within the antigen with an equilibrium dissociation constant (KD) of about 1 × 10⁻⁷ M or less (e.g., about 1 × 10⁻⁸ M or less, or about 1 × 10⁻⁹ M or less). In some embodiments, the KD for the binding of an antibody to an antigen is 10% or less (e.g., 1%) of the KD for the binding of the antibody to a non-specific antigen (e.g., BSA or casein). KD may be determined using known methods in the art, for example, by a BIACORE^{®} surface plasmon resonance assay. However, an antibody that specifically binds to an antigen or an epitope within the antigen may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The terms "anti-TF antibody" and "antibody that binds to TF" refer to an antibody that is capable of binding to TF with sufficient affinity. In certain embodiments, the antibody that binds to an anti-TF antibody has an equilibrium dissociation constant (KD) of < about 1 µM, < about 100 nM, < about 10 nM, < about 1 nM, < about 0.1 nM, < about 0.01 nM, or < about 0.001 nM (e.g., 10⁻⁸ M or less, e.g., 10⁻⁸ M to 10⁻⁹ M, e.g., 10⁻⁹ M or less). In certain embodiments, the anti-TF antibody binds to a conserved TF epitope of TF from different species.

The term "antibody-dependent cell-mediated cytotoxicity" or "ADCC" refers to a form of cytotoxicity in which secreted Ig bound onto Fc receptors (FcRs) present on certain cytotoxic cells (e.g., natural killer (NK) cells, neutrophils, and macrophages) enables these cytotoxic effector cells to specifically bind to antigen-bearing target cells and subsequently kill the target cells with cytotoxins. The antibodies "arm" the cytotoxic cells and are essential for such killing. The primary cells for mediating ADCC, NK cells, express FcγRIII only, whereas monocytes express FcγRI, FcγRII, and FcγRIII. FcR expression on hematopoietic cells is summarized in Table 3 on page 464 of Ravetch and Kinet, Annu. Rev. Immunol., 9:457-92 (1991). To assess the ADCC activity of a target molecule, an *in vitro* ADCC assay, such as that described in U.S. Pat. No. 5,500,362 or 5,821,337 may be performed. Useful effector cells for such assays include peripheral blood monocytes (PBMCs) and natural killer (NK) cells. Alternatively, the ADCC activity of the target antibody may be assessed *in vivo,* e.g., in an animal model (such as that disclosed in Clynes et al., (USA) 95:652-656 (1998)).

The term "antibody-dependent cellular phagocytosis" ("ADCP") refers to a mechanism by which antibody-coated target cells are eliminated by internalization of phagocytic cells (such as macrophages or dendritic cells).

The term "complement-dependent cytotoxicity" or "CDC" refers to a mechanism for inducing cell death in which the Fc effector domain of a target-binding antibody binds to and activates the complement component C1q, and C1q then activates the complement cascade, resulting in the death of the target cell. Activation of a complement may also result in the deposition of complement components on the surface of target cells, and these complement components promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized similarly to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. "Nucleic acid encoding the anti-TF antibody" refers to one or more nucleic acid molecules encoding the antibody heavy and light chains (or fragments thereof).

Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues (Batzer et al., Nucleic Acid Res.19:5081, 1991; Ohtsuka et al., J. Biol. Chem. 260:2605-2608, 1985; and Rossolini et al., Mol. Cell. Probes8:91-98, 1994).

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers.

The term "naked antibody" refers to an antibody that is not conjugated to a heterologous module (e.g., a cytotoxic module) or a radioactive label. A naked antibody may be present in a pharmaceutical formulation.

The term "identity", "sequence identity", or "amino acid sequence identity" refers to the degree (percentage) to which the amino acids/nucleic acids of two sequences are identical at equivalent positions, when the two sequences are optimally aligned, with gaps introduced as necessary to achieve the maximum percent sequence identity, and without considering any conservative substitutions as part of the sequence identity. To determine percent sequence identity, alignment can be achieved using methods that are well known in the art, such as BLAST, BLAST-2, ALIGN, ALIGN-2, or Megalign (DNASTAR). Those skilled in the art can determine parameters suitable for measuring alignment, including any algorithms required to achieve maximum alignment of the full length of the aligned sequences.

The term "conservatively modified variant" or "conservative substitution" refers to substitutions of amino acids in a protein with other amino acids having similar characteristics (e.g. charge, side-chain size, hydrophobicity/hydrophilicity, backbone conformation, and rigidity), such that the changes can be often made without altering the biological activity of the protein. Those skilled in the art recognize that, in general, single amino acid substitutions in non-essential regions of a polypeptide do not substantially alter biological activity (see, e.g., Watson et al., (1987) Molecular Biology of the Gene, The Benjamin/Cummings Pub. Co., p. 224 (4th Ed.)). The term "conservatively modified variant", when applied to nucleic acid sequences, refers to those nucleic acids which encode identical or essentially identical amino acid sequences, or where the nucleic acid does not encode an amino acid sequence, refers to essentially identical sequences. Because of the degeneracy of the genetic code, a number of functionally identical nucleic acids encode any given protein. For example, the codons GCA, GCC, GCG, and GCU all encode the amino acid alanine. Thus, at every position where an alanine is specified by a codon, the codon can be altered to any of the corresponding codons described without altering the encoded polypeptide. Such nucleic acid variations are "silent variations", which are one species of conservatively modified variations. Every nucleic acid sequence herein which encodes a polypeptide also describes every possible silent variation of the nucleic acid. One of skill will recognize that each codon in a nucleic acid (except AUG, which is normally the only codon for methionine, and TGG, which is normally the only codon for tryptophan) can be modified to produce a functionally identical molecule. Thus, each silent variation of a nucleic acid that encodes a polypeptide is implicit in each sequence described.

The term "expression vector" or "expression construct" refers to a vector that is applied to transform a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

As used herein, "operably linked" means that the components to which the term is applied are in a relationship that allows them to carry out their inherent functions under suitable conditions. For example, a control sequence in an expression vector that is "operably linked" to a protein-coding sequence is ligated thereto so that expression of the protein-coding sequence is achieved under conditions compatible with the transcriptional activity of the control sequence.

The terms "host cell", "host cell line", and "host cell culture" are used interchangeably and refer to cells into which exogenous nucleic acids have been introduced, including progeny of such cells. Host cells include "transformants" and "transformed cells", which include primary transformed cells and progeny derived therefrom, regardless of the number of passages. Progeny may not be exactly the same as parent cells in terms of nucleic acid content and may contain mutations. Mutant progenies that have the same function or biological activity as the cells screened or selected from the initially transformed cells are included herein. Host cells include prokaryotic and eukaryotic host cells, wherein the eukaryotic host cells include, but are not limited to, mammalian cells, insect cell lines, plant cells, and fungal cells. Exemplary host cells are as follows: Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells and HEK-293 cells, *Pichiapastoris, Pichia finlandica, Candida albicans, Aspergillus niger, Aspergillus oryzae,* and *Trichoderma reesei.*

As used in the present disclosure, the expressions "cell", "cell line", and "cell culture" are used interchangeably and include the progeny of such cells. Thus, the words "transformant" and "transformed cell" include the primary subject cell and cultures derived therefrom, regardless of the number of passages. It is also understood that not all progeny have precisely identical DNA contents due to sense or nonsense mutations. Mutant progeny that have the identical function or biological activity as the original transformed cell from which they were selected are included.

Methods for producing and purifying antibodies and antigen-binding fragments are well known in the art, for example, those described in chapters 5-8 and 15 of Antibodies: A Laboratory Manual, Cold Spring Harbor Press. The antibody or the antigen-binding fragment described herein is genetically engineered to contain one or more human FRs in the non-human CDRs. Human FR species sequences can be obtained at the website http://imgt.cines.fr of ImMunoGeneTics (IMGT) or from the immunoglobulin journal, 2001ISBN012441351, by alignment with the IMGT human antibody variable region germline gene database through the MOE software.

The engineered antibody or antigen-binding fragment of the present disclosure can be prepared and purified by conventional methods. For example, cDNA sequences encoding heavy and light chains can be cloned and recombined into an expression vector. Recombinant immunoglobulin expression vectors can be stably transfected into host cells. As a more recommended prior art, mammalian expression systems will result in glycosylation of the antibody, particularly at the N-terminal site of the Fc region. Stable clones are obtained by expression of the antibody that specifically binds to human TF. Positive clones are expanded in a culture medium in a bioreactor to produce antibodies. The culture solution with the secreted antibody can be purified by conventional techniques, for example, using an A or G Sepharose FF column. Non-specifically bound fractions are washed away. The bound antibody is eluted by the pH gradient method, and the antibody fragments are detected by SDS-PAGE and collected. The antibodies can be filtered and concentrated by conventional methods. Soluble mixtures and polymers can also be removed by conventional methods, such as molecular sieves and ion exchange. The resulting products need to be immediately frozen, such as at -70 °C, or lyophilized. "Isolated" refers to a purified state, and in this case means that the designated molecule is substantially free of other biomolecules, such as nucleic acids, proteins, lipids, carbohydrates, or other materials (such as cell debris and growth culture medium). Generally, the term "isolated" does not mean the complete absence of such materials or the absence of water, buffers, or salts, unless they are present in amounts that will significantly interfere with the experimental or therapeutic use of the compounds described herein.

The term "drug" refers to a chemical substance that can alter or ascertain an organism's physiology and pathological state and can be used for the prevention, diagnosis, and treatment of diseases. The drug includes a cytotoxic drug. There is no clear boundary between a drug and a toxic substance. The toxic substance refers to a chemical substance that has a toxic effect on organisms and can cause damage to human health even in small doses. Any drug in large doses may induce toxic reactions.

The term "cytotoxic drug" refers to a substance that inhibits or prevents cell functions and/or causes cell death or cell destruction. The cytotoxic drug can kill tumor cells in principle at a sufficiently high concentration; however, due to lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant, or animal origin, radioisotopes (e.g., At²¹¹, I¹³¹, I¹²⁵, Y⁹⁰, Re¹⁸⁶ , Re¹⁸⁸, Sm¹⁵³, Bi²¹², P³², and radioisotopes of Lu), chemotherapeutic drugs, antibiotics, and nucleolytic enzymes.

The term "alkyl" refers to a saturated aliphatic hydrocarbon group. It is a linear or branched group containing 1 to 20 carbon atoms, preferably alkyl containing 1 to 12 (e.g., 1, 2, 3, 4, 5, 6, 7, 8, 9, 10, 11, and 12) carbon atoms, and more preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various side-chain isomers thereof, and the like. Lower alkyl groups having 1 to 6 carbon atoms are more preferred, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted. When it is substituted, the substituent may be substituted at any available point of attachment, and the substituent is preferably one or more substituents independently optionally selected from the group consisting of D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted. When it is substituted, the substituent is preferably one or more groups independently selected from the group consisting of D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a saturated linear or branched aliphatic hydrocarbon group. It is a residue derived from the parent alkane by removal of two hydrogen atoms from the same carbon atom or two different carbon atoms. It is a linear or branched group containing 1 to 20 carbon atoms, preferably alkylene containing 1 to 12 (e.g., 1, 2, 3, 4, 5, 6, 7, 8, 9, 10, 11, and 12) carbon atoms, and more preferably alkylene containing 1 to 6 carbon atoms. Non-limiting examples of alkylene include, but are not limited to, methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. Alkylene may be substituted or unsubstituted. When it is substituted, the substituent may be substituted at any available point of attachment, and the substituent is preferably one or more substituents independently optionally selected from the group consisting of alkenyl, alkynyl, alkoxy, haloalkoxy, cycloalkyloxy, heterocyclyloxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "alkenyl" refers to an alkyl compound having at least one carbon-carbon double bond in the molecule, wherein the alkyl is as defined above. Preferred is alkenyl containing 2 to 12 (e.g., 2, 3, 4, 5, 6, 7, 8, 9, 10, 11, and 12) carbon atoms and more preferred is alkenyl containing 2 to 6 carbon atoms. Alkenyl may be substituted or unsubstituted. When it is substituted, the substituent is preferably one or more groups independently selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl compound having at least one carbon-carbon triple bond in the molecule, wherein the alkyl is as defined above. Preferred is alkynyl containing 2 to 12 (e.g., 2, 3, 4, 5, 6, 7, 8, 9, 10, 11, and 12) carbon atoms and more preferred is alkynyl containing 2 to 6 carbon atoms. Alkynyl may be substituted or unsubstituted. When it is substituted, the substituent is preferably one or more groups independently selected from the group consisting of alkyl, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 12 (e.g., 3, 4, 5, 6, 7, 8, 9, 10, 11, and 12) carbon atoms, more preferably 3 to 8 carbon atoms, and even more preferably 3 to 6 carbon atoms (e.g., C₃₋₆ cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl.

The term "spirocycloalkyl" refers to a 5-20 membered polycyclic group in which monocyclic rings share one carbon atom (referred to as the spiro atom), and the group may contain one or more double bonds. It is preferably 6-14 (e.g., 6, 7, 8, 9, 10, 11, 12, 13, and 14) membered and more preferably 7-10 (e.g., 7, 8, 9, or 10) membered. According to the number of spiro atoms shared between rings, spirocycloalkyl may be monospirocycloalkyl, bispirocycloalkyl, or polyspirocycloalkyl, preferably monospirocycloalkyl and bispirocycloalkyl, more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered monospirocycloalkyl. Non-limiting examples of spirocycloalkyl include:

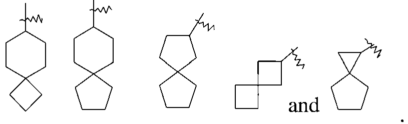

The term "fused cycloalkyl" refers to a 5-20 membered all-carbon polycyclic group in which each ring shares a pair of adjacent carbon atoms with the other rings in the system, wherein one or more rings may contain one or more double bonds. It is preferably 6-14 (e.g., 6, 7, 8, 9, 10, 11, 12, 13, and 14) membered and more preferably 7-10 (e.g., 7, 8, 9, or 10) membered. According to the number of constituent rings, it may be bicyclic, tricyclic, tetracyclic, or polycyclic fused cycloalkyl, preferably bicyclic or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic cycloalkyl. Non-limiting examples of fused cycloalkyl include:

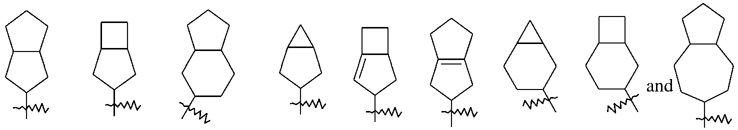

The term "bridged cycloalkyl" refers to a 5-20 membered all-carbon polycyclic group in which any two rings share two carbon atoms that are not directly connected, and the group may contain one or more double bonds. It is preferably 6-14 (e.g., 6, 7, 8, 9, 10, 11, 12, 13, and 14) membered and more preferably 7-10 (e.g., 7, 8, 9, or 10) membered. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. Non-limiting examples of bridged cycloalkyl include:

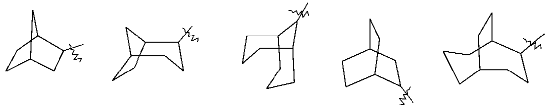

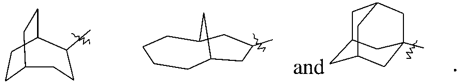

The cycloalkyl ring includes those in which the cycloalkyl described above (including monocyclic, spiro, fused, and bridged ones) is fused to an aryl, heteroaryl, or heterocycloalkyl ring, wherein the ring attached to the parent structure is cycloalkyl. Non-limiting examples include indanyl, tetrahydronaphthyl, benzocycloheptanyl, and the like, and preferably indanyl and tetrahydronaphthyl.

Cycloalkyl may be substituted or unsubstituted. When it is substituted, the substituent may be substituted at any available point of attachment, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heterocyclyl" refers to a saturated or partially unsaturated monocyclic or polycyclic substituent containing 3 to 20 ring atoms, wherein one or more of the ring atoms are heteroatoms selected from the group consisting of nitrogen, oxygen, sulfur, S(O), and S(O)₂, excluding a cyclic portion of -O-O-, -O-S-, or -S-S-, and the other ring atoms are carbon atoms. It preferably contains 3 to 12 (e.g., 3, 4, 5, 6, 7, 8, 9, 10, 11, and 12) ring atoms, of which 1 to 4 (e.g., 1, 2, 3, and 4) are heteroatoms; more preferably 3 to 8 (e.g., 3, 4, 5, 6, 7, and 8) ring atoms, of which 1 to 3 (e.g., 1, 2, and 3) are heteroatoms; more preferably 3 to 6 ring atoms, of which 1 to 3 are heteroatoms; most preferably 5 or 6 ring atoms, of which 1 to 3 are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiroheterocyclyl, fused heterocyclyl, and bridged heterocyclyl.

The term "spiroheterocyclyl" refers to a 5-20 membered polycyclic heterocyclyl group in which monocyclic rings share one atom (referred to as the spiro atom), wherein one or more ring atoms are heteroatoms selected from the group consisting of nitrogen, oxygen, sulfur, S(O) and S(O)₂, and the other ring atoms are carbon atoms. It may contain one or more double bonds. It is preferably 6-14 membered, and is more preferably 7-10 membered (e.g., 7-, 8-, 9-, or 10-membered). According to the number of spiro atoms shared between rings, spiroheterocyclyl may be monospiroheterocyclyl, bispiroheterocyclyl, or polyspiroheterocyclyl, preferably, monospiroheterocyclyl and bispiroheterocyclyl, and more preferably 4-membered/4-membered, 4-membered/5-membered, 4-membered/6-membered, 5-membered/5-membered, 5-membered/6-membered, or 6-membered/6-membered monospiroheterocyclyl. Non-limiting examples of spiroheterocyclyl include:

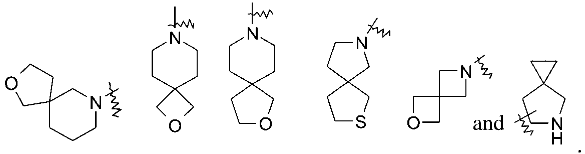

The term "fused heterocyclyl" refers to a 5-20 membered polycyclic heterocyclyl group in which each ring shares a pair of adjacent atoms with the other rings in the system, wherein one or more of the rings may contain one or more double bonds. In the fused heterocyclyl, one or more of the ring atoms are heteroatoms selected from the group consisting of nitrogen, oxygen, sulfur, S(O) and S(O)₂, and the other ring atoms are carbon atoms. It is preferably 6-14 membered, and is more preferably 7-10 membered (e.g., 7-, 8-, 9-, or 10-membered). According to the number of constituent rings, it may be bicyclic, tricyclic, tetracyclic, or polycyclic fused heterocyclyl, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused heterocyclyl. Non-limiting examples of fused heterocyclyl include:

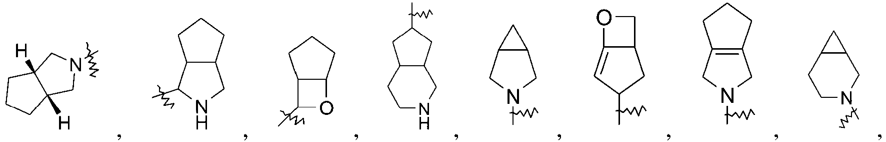

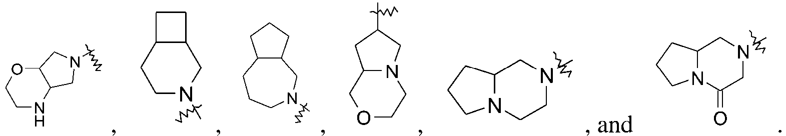

The term "bridged heterocyclyl" refers to a 5-14 membered polycyclic heterocyclyl group in which any two rings share two atoms that are not directly connected to each other, and the group may contain one or more double bonds. In the bridged heterocyclyl, one or more of the ring atoms are heteroatoms selected from the group consisting of nitrogen, oxygen, sulfur, S(O) and S(O)₂, and the other ring atoms are carbon atoms. It is preferably 6-14 membered, and is more preferably 7-10 membered (e.g., 7-, 8-, 9-, or 10-membered). According to the number of constituent rings, it may be bicyclic, tricyclic, tetracyclic, or polycyclic bridged heterocyclyl, preferably bicyclic, tricyclic, or tetracyclic, and more preferably bicyclic or tricyclic. Non-limiting examples of bridged heterocyclyl include:

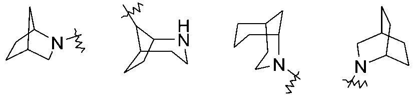

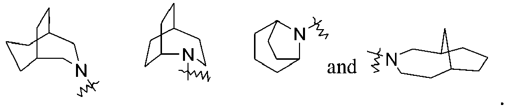

The heterocyclyl ring includes those in which the heterocyclyl described above (including monocyclic, spiroheterocyclic, fused heterocyclic and bridged heterocyclic rings) is fused to an aryl, heteroaryl, or cycloalkyl ring, wherein the ring attached to the parent structure is heterocyclyl; its non-limiting examples include: 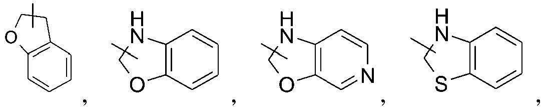 and the like.

Heterocyclyl may be substituted or unsubstituted. When it is substituted, the substituent may be substituted at any available point of attachment, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "aryl" refers to a 6-14 membered, preferably 6-10 membered, all-carbon monocyclic or fused polycyclic (which refers to rings sharing a pair of adjacent carbon atoms) group having a conjugated π-electron system, such as phenyl and naphthyl. The aryl ring includes those in which the aryl ring described above is fused to a heteroaryl, heterocyclyl, or cycloalkyl ring, wherein the ring attached to the parent structure is the aryl ring; its non-limiting examples include:

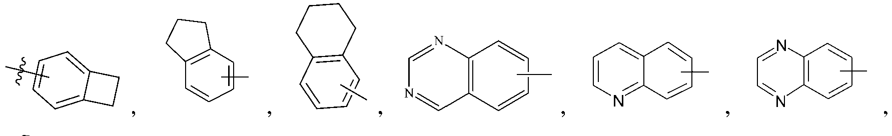

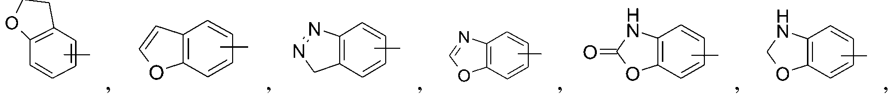

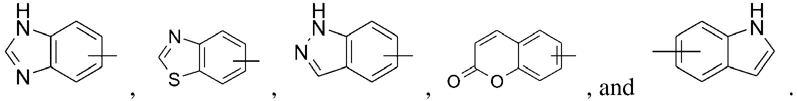

Aryl may be substituted or unsubstituted. When it is substituted, the substituent may be substituted at any available point of attachment, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisting of oxygen, sulfur, and nitrogen. Heteroaryl is preferably 5-10 membered (e.g., 5, 6, 7, 8, 9, or 10 membered) and more preferably 5-6 membered, e.g., furanyl, thienyl, pyridinyl, pyrrolyl, N-alkylpyrrolyl, pyrimidinyl, pyrazinyl, pyridazinyl, imidazolyl, pyrazolyl, triazolyl, and tetrazolyl. The heteroaryl ring includes those in which the heteroaryl ring described above is fused to an aryl, heterocyclyl, or cycloalkyl ring, wherein the ring attached to the parent structure is the heteroaryl ring; its non-limiting examples include:

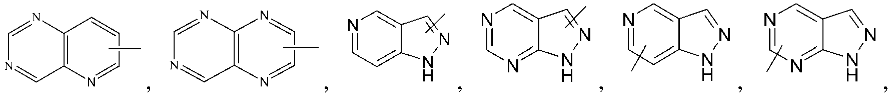

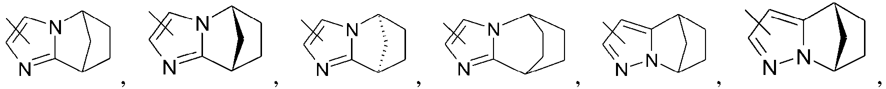

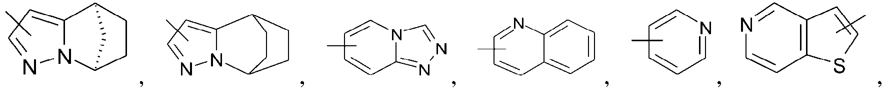

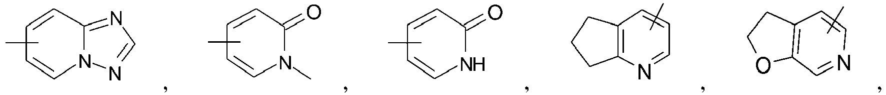

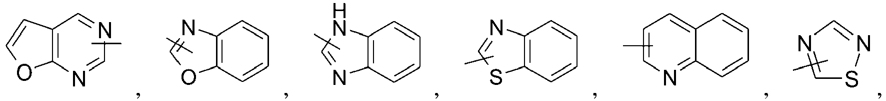

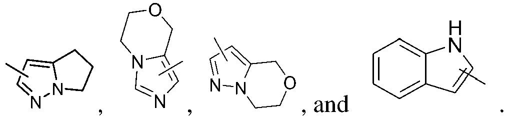

Heteroaryl may be substituted or unsubstituted. When it is substituted, the substituent may be substituted at any available point of attachment, and the substituent is preferably one or more substituents independently optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The cycloalkyl, heterocyclyl, aryl, and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene", or "heteroarylene".

The term "amino protecting group" refers to an easily removable group for protecting an amino group from being changed when a reaction is taking place elsewhere in the molecule. Non-limiting examples include (trimethylsilyl)ethoxymethyl, tetrahydropyranyl, tert-butoxycarbonyl (Boc), acetyl, benzyl, allyl, p-methoxybenzyl, tert-butyldimethylsilyl (TBS), and the like. These groups may be optionally substituted with 1-3 substituents selected from the group consisting of halogen, alkoxy, and nitro. The term "hydroxy protecting group" refers to a hydroxy derivative commonly used to block or protect hydroxy when a reaction is taking place on another functional group of the compound. By way of example, preferably, the hydroxy protecting group may be: (C₁₋₁₀ alkyl or aryl)₃silyl, e.g., triethylsilyl, triisopropylsilyl, tert-butyldimethylsilyl (TBS), and tert-butyldiphenylsilyl; C₁₋₁₀ alkyl or substituted alkyl, preferably alkoxy or aryl-substituted alkyl, more preferably C₁₋₆ alkoxy-substituted C₁₋₆ alkyl or phenyl-substituted C₁₋₆ alkyl, and most preferably C₁₋₄ alkoxy-substituted C₁₋₄ alkyl, e.g., methyl, tert-butyl, allyl, benzyl, methoxymethyl (MOM), ethoxyethyl, and 2-tetrahydropyranyl (THP); (C₁₋₁₀ alkyl or aryl)acyl, e.g., formyl, acetyl, benzoyl, and p-nitrobenzoyl; (C₁₋₆ alkyl or 6-10 membered aryl)sulfonyl; or (C₁₋₆ alkoxy or 6-10 membered aryloxy)carbonyl.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

The term "antibody-drug conjugate (ADC)" means that an antibody is linked to a biologically active cytotoxin or a small-molecule drug with cell killing activity by a linker unit.

The antibody or the antibody fragment described herein may be conjugated to an effector molecule by any means. For example, the antibody or the antibody fragment may be chemically or recombinantly attached to the cytotoxic drug. Chemical means for preparing fusions or conjugates are known in the art and can be used to prepare immunoconjugates. The method for conjugating the antibody or the antibody fragment and the drug must be capable of linking the antibody to the cytotoxic drug without interfering with the ability of the antibody or the antibody fragment to bind to the target molecule.

In one embodiment, both the antibody and cytotoxic drug are proteins and can be conjugated using techniques well known in the art. There are hundreds of cross-linking agents disclosed in the art that can conjugate two proteins. The cross-linking agent is generally selected based on reactive functional groups available or inserted on the antibody or cytotoxic drug. Alternatively, if no reactive groups are present, a photo-activatable cross-linking agent may be used. In some cases, it may be desirable to include a spacer between the antibody and the cytotoxic drug. Cross-linking agents known in the art include homobifunctional agents: glutaraldehyde, dimethyl adipimidate and bis(diazobenzidine), and heterobifunctional agents: m-maleimidobenzoyl-N-hydroxysuccinimide and sulfo-m-maleimidobenzoyl-N-hydroxysuccinimide. Cross-linking agents that can be used to conjugate an effector molecule to an antibody fragment include, for example, TPCH (S-(2-thiopyridyl)-L-cysteine hydrazide) and TPMPH (S-(2-thiopyridyl)sulfhydryl-propionhydrazide). TPCH and TPMPH react on the carbohydrate moiety of the glycoprotein that had previously been oxidized by mild periodate treatment, thereby forming a hydrazone bond between the hydrazide moiety of the cross-linking agent and the aldehyde generated by periodate. The heterobifunctional cross-linking agents GMBS (N-(γ-maleimidobutyryloxy)-succinimide) and SMCC (succinimidyl 4-(N-maleimido-methyl)cyclohexane) are reacted with a primary amine, thereby introducing a maleimido group onto the component. This maleimido group may then react with a sulfhydryl group on another component which may be introduced by a cross-linking agent, thereby forming a stable thioether bond between the components. If steric hindrance between the components interferes with the activity of either component, a cross-linking agent may be used to introduce a long spacer between the components, such as N-succinimidyl 3-(2-pyridyldithio)propionate (SPDP). Thus, there are many suitable cross-linking agents that can be used and selected individually depending on their effect on the yield of the optimal immunoconjugate.

The term "drug loading", also referred to as drug-to-antibody ratio (DAR), refers to the average number of drugs conjugated to each antibody in an ADC. It may range, for example, from about 1 to about 10 drugs conjugated to each antibody, and in certain embodiments, from about 1 to about 8 drugs conjugated to each antibody, preferably selected from the group consisting of 2-8, 2-7, 2-6, 2-5, 2-4, 3-4, 3-5, 4-8, 5-6, 5-7, 5-8, and 6-8 drugs conjugated to each antibody. Illustratively, the drug loading may be an average of 1, 2, 3, 4, 5, 6, 7, 8, 9, or 10. The ADC general formulas of the present disclosure include a set of antibody-drug conjugates within a certain range as described above. In the embodiments of the present disclosure, drug loading may be represented by n, which is a decimal or an integer. Drug loading can be determined by conventional methods such as UV/visible spectroscopy, mass spectrometry, ELISA assay, and HPLC. The term "linker unit" or "linker fragment" refers to a chemical structure fragment or bond that is linked to an antibody or an antigen-binding fragment thereof at one end and to a drug at the other end, and it may also be linked to a drug after being linked to another linker.

The term "linker" may comprise one or more linker components. Exemplary linker components include 6-maleimidocaproyl ("MC"), maleimidopropionyl ("MP"), valine-citrulline ("val-cit" or "vc"), alanine-phenylalanine ("ala-phe"), p-aminobenzyloxycarbonyl ("PAB"), and those derived from coupling to a linker reagent: N-succinimidyl 4-(2-pyridylthio)pentanoate ("SPP"), N-succinimidyl 4-(N-maleimidomethyl)cyclohexane-1 carboxylate ("SMCC", also referred to herein as "MCC"), and N-succinimidyl(4-iodo-acetyl)aminobenzoate ("SIAB"). The linker may include stretcher units, spacer units, and amino acid units, and may be synthesized using methods known in the art, such as those described in US2005-0238649A1. The linker may be a "cleavable linker" favorable for the release of drugs in cells. For example, acid-labile linkers (e.g., hydrazones), protease-sensitive (e.g., peptidase-sensitive) linkers, photolabile linkers, dimethyl linkers, or disulfide-containing linkers may be used (Chari et al., Cancer Research, 52: 127-131(1992); U.S. Patent No. 5,208,020).

Linker components include, but are not limited to:
MC = 6-maleimidocaproyl, whose structure is shown below: 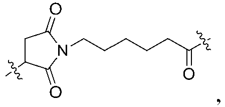
Val-Cit or "vc" = valine-citrulline (an exemplary dipeptide in a protease cleavable linker), citrulline = 2-amino-5-ureidopentanoic acid,
PAB group = p-aminobenzyloxycarbonyl (an example of "self-immolative" linker components), whose structure is shown below: 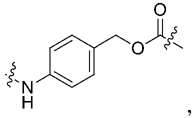
Me-Val-Cit = N-methyl-valine-citrulline (where the linker peptide bond has been modified to prevent it from being cleaved by cathepsin B),
MC(PEG)6-OH = maleimidocaproyl-polyethylene glycol (attachable to antibody cysteine),
SPP = N-succinimidyl 4-(2-pyridylthio)valerate,
SPDP = N-succinimidyl 3-(2-pyridyldithio)propionate,
SMCC = succinimidyl-4-(N-maleimidomethyl)cyclohexane-1-carboxylate, and
IT = iminothiolane.

In one embodiment of the present disclosure, the cytotoxic drug is conjugated to a sulfhydryl group of the antibody by a linker unit.

The loading of the ligand-drug conjugate can be controlled by the following non-limiting methods, including:
(1) controlling a molar ratio of a linking moiety of the compound to the monoclonal antibody,
(2) controlling reaction time and temperature, and
(3) selecting different reaction reagents.

The term "optionally" or "optional" means that the event or circumstance subsequently described may, but does not necessarily, occur, and this description includes instances where the event or circumstance occurs or does not occur. For example, "C₁₋₆ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

The term "substituted" means that one or more, preferably 1-6, and more preferably 1-3 hydrogen atoms in the group are independently substituted with a corresponding number of substituents. Those skilled in the art can determine (experimentally or theoretically) possible or impossible substitutions without undue effort. For example, it may be unstable when amino or hydroxy having a free hydrogen binds to a carbon atom having an unsaturated (e.g., olefinic) bond.

In the present disclosure, the concentration of the antibody-drug conjugate is expressed in terms of the concentration of the protein, i.e., the concentration of the antibody moiety in the antibody-drug conjugate.

For drugs or pharmacologically active agents, the term "therapeutically effective amount" refers to an amount of a drug or an agent that is sufficient to provide the desired effect but is non-toxic. The determination of the effective amount varies from person to person. It depends on the age and general condition of a subject, as well as the specific active substance used. The appropriate effective amount in a case may be determined by those skilled in the art in the light of routine tests.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. An example of the lactam-lactim equilibrium is present between A and B as shown below.

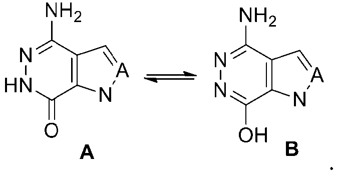

All compounds in the present disclosure can be drawn as form A or form B. All tautomeric forms are within the scope of the present disclosure. The names of the compounds do not exclude any tautomers.

The term "pharmaceutically acceptable" used herein means that those compounds, materials, compositions, and/or dosage forms are, within the scope of reasonable medical judgment, suitable for use in contact with the tissues of subjects without excessive toxicity, irritation, allergic reaction, or other problems or complications, and are commensurate with a reasonable benefit/risk ratio and effective for the intended use. For the preparation of conventional pharmaceutical compositions, refer to Chinese Pharmacopoeia.

As used herein, the singular forms "a", "an", and "the" include plural references and vice versa, unless otherwise clearly defined in the context.

"About" means that it is within an acceptable error range for a particular value as determined by one of ordinary skill in the art, which will depend in part on how the value is measured or determined, i.e., the limitations of the measurement system. In the context of a particular assay, result, or embodiment, "about" means that it is within one standard deviation according to the practice in the art, unless otherwise explicitly stated in the example or elsewhere in the specification.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes humans and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to *Macaca fascicularis.* In certain embodiments, the individual or subject is a human.

The term "excipient" is an addition, besides the main drug, to a pharmaceutical formulation. It may also be referred to as an auxiliary material. For example, binders, fillers, disintegrants, and lubricants in tablets, the matrix part in semisolid ointment and cream preparations, preservatives, antioxidants, corrigents, fragrances, cosolvents, emulsifiers, solubilizers, osmotic pressure regulators, colorants, and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus facilitate the preparation of the tablet. Examples include starch, lactose, inorganic salts of calcium, microcrystalline cellulose, and the like.

The term "pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote administration to an organism and facilitate the absorption of the active ingredient so that it can exert its biological activity.

The pharmaceutical composition may be in the form of a sterile injectable aqueous solution. Available and acceptable vehicles and solvents include water, Ringer's solution, and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer the solution and microemulsion in such a way that maintains a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic monoglycerides or diglycerides can be used. In addition, fatty acids such as oleic acid may also be used in the preparation of injections.

"Administrating" or "giving", when applied to animals, humans, experimental subjects, cells, tissues, organs, or biological fluids, refers to contact of an exogenous drug, therapeutic agent, diagnostic agent, or composition with the animals, humans, subjects, cells, tissues, organs, or biological fluids.

The term "sample" refers to a collection of similar fluids, cells, or tissues isolated from a subject, as well as fluids, cells, or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; and the like), tissue biopsy samples, fine needle punctures, surgically excised tissues, organ cultures, or cell cultures.

The term "pharmaceutically acceptable salt" refers to a salt of the antibody-drug conjugates of the present disclosure, or a salt of the compound described in the present disclosure. Such salts are safe and effective when used in the body of a mammal and possess the required biological activity. The antibody-drug conjugates of the present disclosure contain at least one amino group and therefore can form salts with acids.

"Treatment" or "treat" (and grammatical variations thereof) refer to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or alleviate damage (e.g., lung disease) caused by or associated with a disease state. In some embodiments, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. Complete treatment or prevention does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on the following factors: e.g., the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a subject.

One or more embodiments of the present disclosure are described in detail in the specification above. Other features, objects, and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

### BRIEF DESCRIPTION OF THE DRAWINGS

FIG. 1 shows the results of the binding of the antibodies of the present disclosure to the mouse TF antigen protein.
FIG. 2 shows the results of the binding of the antibodies of the present disclosure to the rat TF antigen protein.
FIG. 3 shows PT assay results of the ADC-1 of the present disclosure in a pharmacokinetic experiment on cynomolgus monkeys.
FIG. 4 shows APTT assay results of the ADC-1 of the present disclosure in a pharmacokinetic experiment on cynomolgus monkeys.

### DETAILED DESCRIPTION

The present disclosure is further described below with reference to the examples, which, however, are not intended to limit the scope of the present disclosure. Experimental methods without specific conditions indicated in the examples or test examples of the present disclosure were generally conducted under conventional conditions or conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated were commercially available conventional reagents. The application WO2020063676 A1 is incorporated herein by reference in its entirety.

### Examples

### Example 1: Clone Construction of Antigens and Antibodies

Molecular clones of antibodies (comprising light and heavy chains) and antigens were constructed using the overlap extension PCR method or the gene synthesis method well known in the art. Antibody and protein antigen sequences were constructed into pTT5 vectors, respectively, and Expi293F^{™} cells (A14528, Gibco) were transfected to obtain corresponding antibodies and antigen proteins.

### Antigen protein sequences

The human TF (hTF) protein sequence was derived from NCBI (AAA61151.1), and its extracellular domain (ECD) comprises 219 amino acids (Ser33-Glu251). The monkey TF (cyno TF, cTF) protein sequence was derived from NCBI (XP_005542723.1), and its extracellular domain (ECD) comprises 220 amino acids (Ser33-Glu252). The rat TF (rTF) protein sequence was derived from NCBI (AAA16966.1), and its extracellular domain (ECD) comprises 224 amino acids (Ala29-Glu252). The mouse TF (mTF) protein sequence was derived from UniProtKB (P20352), and its extracellular domain (ECD) comprises 223 amino acids (Ala29-Glu251). A His or human Fc region (hFc) or mouse Fc region (mFc) tag might be added to the sequence of the extracellular region of TF. Plasmids related to the proteins used for TF immunization and assays were prepared using pTT5 vectors, or were transfected into Expi293F^{™} cells using an ExpiFectamine^{™} 293 transfection kit (A14525, Gibco) to obtain the target proteins.

The antigen protein sequences are shown below:
Full sequence of human TF: 






SEQ ID NO: 1
Full sequence of Cyno TF: 
METPAWPRVPRPETAVARTLLLGWVFAQVAGASGTTNTVAAYNLTWKSTNFKTI
LEWEPKPINQVYTVQISTKSGDWKSKCFYTADTECDLTDEIVKDVKQTYLARVF
 
SYPAGHVESTGSTEEPPYENSPEFTPYLETNLGQPTIQSFEQVGTKVNVTVQDEW
TLVRRNDTFLSLRDVFGKDLIYTLYYWKSSSSGKKTAKTNTNEFLIDVDKGENY
CFSVQAVIPSRRTANRKSTDSPVECMGHEKGESREIFYIIGAVVFVVIILVIILAISL
HKCKKARVGRSWKENSPLNVA
SEQ ID NO: 2
Full sequence of rat TF: 
MAIPMRPRLLAALAPTFLGFLLLQVAVGAGTPPGKAFNLTWISTDFKTILEWQPK
PTNYTYTVQISDRSRNWKYKCTGTTDTECDLTDEIVKDVNWTYEARVLSVPWR
NSTHGKETLFGTHGEEPPFTNARKFLPYRDTKIGQPVIQKYEQGGTKLKVTVKD
SFTLVRKNGTFLTLRQVFGNDLGYILTYRKDSSTGRKTNTTHTNEFLIDVEKGVS
YCFFAQAVIFSRKTNHKSPESITKCTEQWKSVLGETLIIVGAVVFLVTVFIILLTISL
CKRRKNRAGQKRKNTPSRLA
SEQ ID NO: 3
Full sequence of mouse TF: 
MAILVRPRLLAALAPTFLGCLLLQVIAGAGIPEKAFNLTWISTDFKTILEWQPKPT
NYTYTVQISDRSRNWKNKCFSTTDTECDLTDEIVKDVTWAYEAKVLSVPRRNS
VHGDGDQLVIHGEEPPFTNAPKFLPYRDTNLGQPVIQQFEQDGRKLNVVVKDSL
TLVRKNGTFLTLRQVFGKDLGYIITYRKGSSTGKKTNITNTNEFSIDVEEGVSYC
FFVQAMIFSRKTNQNSPGSSTVCTEQWKSFLGETLIIVGAVVLLATIFIILLSISLCK
RRKNRAGQKGKNTPSRLA
SEQ ID NO: 4
Sequence of human TF ECD-His (hTF-his): 
SGTTNTVAAYNLTWKSTNFKTILEWEPKPVNQVYTVQISTKSGDWKSKCFYTT
DTECDLTDEIVKDVKQTYLARVFSYPAGNVESTGSAGEPLYENSPEFTPYLETNL
GQPTIQSFEQVGTKVNVTVEDERTLVRRNNTFLSLRDVFGKDLIYTLYYWKSSS
SGKKTAKTNTNEFLIDVDKGENYCFSVQAVIPSRTVNRKSTDSPVECMGQEKGE
FRETGHHHHHH
SEQ ID NO: 5
Sequence of human TF ECD-hFc (hTF-hFc): 
SGTTNTVAAYNLTWKSTNFKTILEWEPKPVNQVYTVQISTKSGDWKSKCFYTT
DTECDLTDEIVKDVKQTYLARVFSYPAGNVESTGSAGEPLYENSPEFTPYLETNL
GQPTIQSFEQVGTKVNVTVEDERTLVRRNNTFLSLRDVFGKDLIYTLYYWKSSS
SGKKTAKTNTNEFLIDVDKGENYCFSVQAVIPSRTVNRKSTDSPVECMGQEKGE
FREEPKSSDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 6
Sequence of cyno TF ECD-hFc (cTF-hFc): SGTTNTVAAYNLTWKSTNFKTILEWEPKPINQVYTVQISTKSGDWKSKCFYTAD 
TECDLTDEIVKDVKQTYLARVFSYPAGHVESTGSTEEPPYENSPEFTPYLETNLG
QPTIQSFEQVGTKVNVTVQDEWTLVRRNDTFLSLRDVFGKDLIYTLYYWKSSSS
GKKTAKTNTNEFLIDVDKGENYCFSVQAVIPSRRTANRKSTDSPVECMGHEKGE
SREEPKSSDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 7
Sequence of cyno TF ECD-His (cTF-his): 
SGTTNTVAAYNLTWKSTNFKTILEWEPKPINQVYTVQISTKSGDWKSKCFYTAD
TECDLTDEIVKDVKQTYLARVFSYPAGHVESTGSTEEPPYENSPEFTPYLETNLG
QPTIQSFEQVGTKVNVTVQDEWTLVRRNDTFLSLRDVFGKDLIYTLYYWKSSSS
GKKTAKTNTNEFLIDVDKGENYCFSVQAVIPSRRTANRKSTDSPVECMGHEKGE
SRETGHHHHHH
SEQ ID NO: 8
Sequence of cyno TF ECD-mFc (cTF-mFc): 
SGTTNTVAAYNLTWKSTNFKTILEWEPKPINQVYTVQISTKSGDWKSKCFYTAD
TECDLTDEIVKDVKQTYLARVFSYPAGHVESTGSTEEPPYENSPEFTPYLETNLG
QPTIQSFEQVGTKVNVTVQDEWTLVRRNDTFLSLRDVFGKDLIYTLYYWKSSSS
GKKTAKTNTNEFLIDVDKGENYCFSVQAVIPSRRTANRKSTDSPVECMGHEKGE
SREEPRGPTIKPCPPCKCPAPNLLGGPSVFIFPPKIKDVLMISLSPIVTCVVVDVSE
DDPDVQISWFVNNVEVHTAQTQTHREDYNSTLRVVSALPIQHQDWMSGKEFK
CKVNNKDLPAPIERTISKPKGSVRAPQVYVLPPPEEEMTKKQVTLTCMVTDFMP
EDIYVEWTNNGKTELNYKNTEPVLDSDGSYFMYSKLRVEKKNWVERNSYSCS
VVHEGLHNHHTTKSFSRTPGK
SEQ ID NO: 106
Sequence of rat TF ECD-His: 
AGTPPGKAFNLTWISTDFKTILEWQPKPTNYTYTVQISDRSRNWKYKCTGTTDT
ECDLTDEIVKDVNWTYEARVLSVPWRNSTHGKETLFGTHGEEPPFTNARKFLPY
RDTKIGQPVIQKYEQGGTKLKVTVKDSFTLVRKNGTFLTLRQVFGNDLGYILTY
RKDSSTGRKTNTTHTNEFLIDVEKGVSYCFFAQAVIFSRKTNHKSPESITKCTEQ
WKSVLGETGHHHHHH
SEQ ID NO: 9
Sequence of mouse TF ECD-His:

AGIPEKAFNLTWISTDFKTILEWQPKPTNYTYTVQISDRSRNWKNKCFSTTDTEC
DLTDEIVKDVTWAYEAKVLSVPRRNSVHGDGDQLVIHGEEPPFTNAPKFLPYRD
TNLGQPVIQQFEQDGRKLNVVVKDSLTLVRKNGTFLTLRQVFGKDLGYIITYRK
GSSTGKKTNITNTNEFSIDVEEGVSYCFFVQAMIFSRKTNQNSPGSSTVCTEQWK
SFLGETGHHHHHH
SEQ ID NO: 10

Amino acids 1-130 of rat TF (Metl-Alal30) and amino acids 130-245 of human TF (Pro130-Glu245) were combined, and a His tag was added to construct HuRatTF, a human-rat hybrid antigen. Individual amino acids that affect blood coagulation were replaced with amino acid Ala, and individual sites in the rat TF sequence were mutated into corresponding sequences of human TF. Its protein sequence (containing a signal peptide) is shown below:

MAIPMRPRLLAALAPTFLGFLLLQVAVGAGTPPGKAFNLTWISADFKTILAWQP
KPTNYTYTVQISTKSGDWKYKCTGTTDTECALTDEIVKDVKQTYLARVLSVPW
RNSTHGKETLFGTHGEEPPFTNAPEFTPYLETNLGAPTIQSFEQVGTKVNVTVEDE
RTLVRRNNTFLSLRDVFGKDLIYTLAYWKSSSSGAKTAATNTNEFLIDVDKGENYCFS
VQAVIPSRTVNRASTDSPVECMGQEHHHHHH
SEQ ID NO: 11

Note: The human TF sequence is italicized, the signal peptide is indicated by a dashed line, the mutation of rat TF amino acids into corresponding human TF amino acids is double-underlined, the mutation of the sites that affect blood coagulation into alanine (Ala) is underlined, and the His tag is indicated by a wavy line.

### Sequences used in construction of antigen cell strains

Plasmids related to the antigen cell strains used for TF immunization and assays were prepared with reference to the vectors in Cell, 01 Aug 2005, 122(3):473-48. PiggyBac transposon plasmids containing antigen sequences were transfected into NIH/3T3 (ATCC, CRL-1658) or/and CHO-K1 (ATCC, CCL-61) cells using Lipofectamine 3000 transfection reagent (Invitrogen, L3000015) and transposase hyPBase plasmid, and target cell strains were obtained by flow cytometry sorting (BD FACSAria^{™} Fusion Cell Sorter). The antigen sequences used in the cell strains constructed above consisted of full-length TF antigen, self-cleaving protein (2A), and green fluorescent protein (EGFP) sequences. Specifically:
PiggyBac transposon plasmid constituent elements (PB 3' LTR and PB 5' LTR, NCBI-KF658273.1):
PB 3' LTR-CMV promoter-antigen sequence-beta-globin poly(A)-PB 5' LTR -Ampicillin resisitance gene-bla promoter
Transposase hyPBase plasmid constituent element (hyPBase, NCBI-OL519599.1): EF1a promoter-hyPBase-BGH polyA-f1 origin-SV40 early promoter and origin - Neomycin resistance gene-SV40 polyA- pUC origin -Ampicillin resisitance gene- bla promoter
Sequence of human TF-2A-EGFP (hTF): 
METPAWPRVPRPETAVARTLLLGWVFAQVAGASGTTNTVAAYNLTWKSTNFKTILEWE
PKPVNQVYTVQISTKSGDWKSKCFYTTDTECDLTDEIVKDVKQTYLARVFSYPAGNV
ESTGSAGEPLYENSPEFTPYLETNLGQPTIQSFEQVGTKVNVTVEDERTLVRRNNTFL
SLRDVFGKDLIYTLYYWKSSSSGKKTAKTNTNEFLIDVDKGENYCFSVQAVIPSRTVNR
KSTDSPVECMGQEKGEFREIFYIIGAVAFVVIILVIILAISLHKCRKAGVGQSWKENSPL
NVSTSKIVAPVKQTLNFDLLKLAGDVESNPGPGSTMVSKGEELFTGVVPILVELD
 
GDVNGHKFSVSGEGEGDATYGKLTLKFICTTGKLPVPWPTLVTTLTYGVQCFSR
YPDHMKQHDFFKSAMPEGYVQERTIFFKDDGNYKTRAEVKFEGDTLVNRIELK
GIDFKEDGNILGHKLEYNYNSHNVYIMADKQKNGIKVNFKIRHNIEDGSVQLA
DHYQQNTPIGDGPVLLPDNHYLSTQSALSKDPNEKRDHMVLLEFVTAAGITLG
MDELYK
SEQ ID NO: 12
Sequence of cyno TF-2A-EGFP (cTF):

METPAWPRVPRPETAVARTLLLGWVFAQVAGASGTTNTVAAYNLTWKSTNFKTILEWE
PKPINQVYTVQISTKSGDWKSKCFYTADTECDLTDEIVKDVKQTYLARVFSYPAGHVE
STGSTEEPPYENSPEFTPYLETNLGQPTIQSFEQVGTKVNVTVQDEWTLVRRNDTFLS
LRDVFGKDLIYTLYYWKSSSSGKKTAKTNTNEFLIDVDKGENYCFSVQAVIPSRRTANR
KSTDSPVECMGHEKGESREIFYIIGAVVFVVIILVIILAISLHKCKKARVGRSWKENSPL
NVATSKIVAPVKQTLNFDLLKLAGDVESNPGPGSTMVSKGEELFTGVVPILVELD
GDVNGHKFSVSGEGEGDATYGKLTLKFICTTGKLPVPWPTLVTTLTYGVQCFSR
YPDHMKQHDFFKSAMPEGYVQERTIFFKDDGNYKTRAEVKFEGDTLVNRIELK
GIDFKEDGNILGHKLEYNYNSHNVYIMADKQKNGIKVNFKIRHNIEDGSVQLA
DHYQQNTPIGDGPVLLPDNHYLSTQSALSKDPNEKRDHMVLLEFVTAAGITLG
MDELYK
SEQ ID NO: 13

Note: The TF sequence is italicized, the 2A sequence is double-underlined, and the EGFP sequence is indicated by a wavy line.

Referring to patent document WO2010066803A2, a total of 7 human-mouse chimeric antigens were prepared by replacing a segment of the sequence of human TF with the corresponding sequence of mouse TF: hTF1 (1-41 mm), hTF2 (42-84 mm), hTF3 (85-122 mm), hTF4 (123-137 mm), hTF7 (138-184 mm), hTF5 (185-225 mm), and hTF6 (226-250 mm). These 7 antigens were referred to as hTF1, hTF2, hTF3, hTF4, hTF7, hTF5, and hTF6 for short. Patent document WO2010066803A2 does not mention hTF7, which is a new addition in the present disclosure. The human TF antigen epitopes to which these 7 antigens correspond in the test examples are shown in Table 2.

**Table 2**

| **Human-mouse chimeric TF antigen** | **Corresponding human TF sequence replaced with mouse sequence** |
|---|---|
| hTF1(1-41mm) | METPAWPRVPRPETAVARTLLLGWVFAQVAGASGTTN TVAA (SEQ ID NO:14) |
| hTF2(42-84mm) | YNLTWKSTNFKTILEWEPKPVNQVYTVQISTKSGDW KSKCFYT (SEQ ID NO:15) |
| hTF3(85-122mm) | TDTECDLTDEIVKDVKQTYLARVFSYPAGNVESTGSA G |
| | (SEQ ID NO:16) |
| hTF4(123-137mm) | EPLYENSPEFTPYLE (SEQ ID NO: 17) |
| hTF7(138-184mm) | TNLGQPTIQSFEQVGTKVNVTVEDERTLVRRNNTFLS LRDVFGKDLI (SEQ ID NO:18) |
| hTF5(185-225mm) | YTLYYWKSSSSGKKTAKTNTNEFLIDVDKGENYCFSV QAVI (SEQ ID NO:19) |
| hTF6(226-250mm) | PSRTVNRKSTDSPVECMGQEKGEFR (SEQ ID NO:20) |

The sequences of the 7 chimeric antigens are shown below:
Sequence of hTF1 (1-41 mm): 
MAILVRPRLLAALAPTFLGCLLLQVIAGAGIPEKAYNLTWKSTNFKTILEWEPKPV
NQVYTVQISTKSGDWKSKCFYTTDTECDLTDEIVKDVKQTYLARVFSYPAGNVESTGS
AGEPLYENSPEFTPYLETNLGQPTIQSFEQVGTKVNVTVEDERTLVRRNNTFLSLRDV
FGKDLIYTLYYWKSSSSGKKTAKTNTNEFLIDVDKGENYCFSVQAVIPSRTVNRKSTDS
PVECMGQEKGEFREIFYIIGAVAFVVIILVIILAISLHKCRKAGVGQSWKENSPLNVSTS
KIVAPVKQTLNFDLLKLAGDVESNPGPGSTMVSKGEELFTGVVPILVELDGDVN
GHKFSVSGEGEGDATYGKLTLKFICTTGKLPVPWPTLVTTLTYGVQCFSRYPDH
MKQHDFFKSAMPEGYVQERTIFFKDDGNYKTRAEVKFEGDTLVNRIELKGIDFK
EDGNILGHKLEYNYNSHNVYIMADKQKNGIKVNFKIRHNIEDGSVQLADHYQQ
NTPIGDGPVLLPDNHYLSTQSALSKDPNEKRDHMVLLEFVTAAGITLGMDELYK
SEQ ID NO: 21
Sequence of hTF2 (42-84 mm): 
METPAWPRVPRPETAVARTLLLGWVFAQVAGASGTTNTVAAFNLTWISTDFKTILEW
QPKPTNYTYTVQISDRSRNWKNKCFSTTDTECDLTDEIVKDVKQTYLARVFSYPAG
NVESTGSAGEPLYENSPEFTPYLETNLGQPTIQSFEQVGTKVNVTVEDERTLVRRNNT
FLSLRDVFGKDLIYTLYYWKSSSSGKKTAKTNTNEFLIDVDKGENYCFSVQAVIPSRTV
NRKSTDSPVECMGQEKGEFREIFYIIGAVAFVVIILVIILAISLHKCRKAGVGQSWKENS
PLNVSTSKIVAPVKQTLNFDLLKLAGDVESNPGPGSTMVSKGEELFTGVVPILVE
LDGDVNGHKFSVSGEGEGDATYGKLTLKFICTTGKLPVPWPTLVTTLTYGVQCF
SRYPDHMKQHDFFKSAMPEGYVQERTIFFKDDGNYKTRAEVKFEGDTLVNRIE
LKGIDFKEDGNILGHKLEYNYNSHNVYIMADKQKNGIKVNFKIRHNIEDGSVQL
ADHYQQNTPIGDGPVLLPDNHYLSTQSALSKDPNEKRDHMVLLEFVTAAGITL
GMDELYK
SEQ ID NO: 22
hTF3 (85-122 mm): 
METPAWPRVPRPETAVARTLLLGWVFAQVAGASGTTNTVAAYNLTWKSTNFKTILEWE
PKPVNQVYTVQISTKSGDWKSKCFYTTDTECDLTDEIVKDVTWAYEAKVLSVPRR
 
NSVHGDGDQLVIHGEEPLYENSPEFTPYLETNLGQPTIQSFEQVGTKVNVTVEDERT
LVRRNNTFLSLRDVFGKDLIYTLYYWKSSSSGKKTAKTNTNEFLIDVDKGENYCFSVQ
AVIPSRTVNRKSTDSPVECMGQEKGEFREIFYIIGAVAFVVIILVIILAISLHKCRKAGVG
QSWKENSPLNVSTSKIVAPVKQTLNFDLLKLAGDVESNPGPGSTMVSKGEELFTG
VVPILVELDGDVNGHKFSVSGEGEGDATYGKLTLKFICTTGKLPVPWPTLVTTLT
YGVQCFSRYPDHMKQHDFFKSAMPEGYVQERTIFFKDDGNYKTRAEVKFEGD
TLVNRIELKGIDFKEDGNILGHKLEYNYNSHNVYIMADKQKNGIKVNFKIRHNI
EDGSVQLADHYQQNTPIGDGPVLLPDNHYLSTQSALSKDPNEKRDHMVLLEFV
TAAGITLGMDELYK
SEQ ID NO: 23
hTF4 (123-137 mm): 
METPAWPRVPRPETAVARTLLLGWVFAQVAGASGTTNTVAAYNLTWKSTNFKTILEWE
PKPVNQVYTVQISTKSGDWKSKCFYTTDTECDLTDEIVKDVKQTYLARVFSYPAGNV
ESTGSAGEPPFTNAPKFLPYRDTNLGQPTIQSFEQVGTKVNVTVEDERTLVRRNNTF
LSLRDVFGKDLIYTLYYWKSSSSGKKTAKTNTNEFLIDVDKGENYCFSVQAVIPSRTVN
RKSTDSPVECMGQEKGEFREIFYIIGAVAFVVIILVIILAISLHKCRKAGVGQSWKENSP
LNVSTSKIVAPVKQTLNFDLLKLAGDVESNPGPGSTMVSKGEELFTGVVPILVEL
DGDVNGHKFSVSGEGEGDATYGKLTLKFICTTGKLPVPWPTLVTTLTYGVQCFS
RYPDHMKQHDFFKSAMPEGYVQERTIFFKDDGNYKTRAEVKFEGDTLVNRIEL
KGIDFKEDGNILGHKLEYNYNSHNVYIMADKQKNGIKVNFKIRHNIEDGSVQL
ADHYQQNTPIGDGPVLLPDNHYLSTQSALSKDPNEKRDHMVLLEFVTAAGITL
GMDELYK
SEQ ID NO: 24
hTF7 (138-184 mm): 
METPAWPRVPRPETAVARTLLLGWVFAQVAGASGTTNTVAAYNLTWKSTNFKTILEWE
PKPVNQVYTVQISTKSGDWKSKCFYTTDTECDLTDEIVKDVKQTYLARVFSYPAGNV
ESTGSAGEPLYENSPEFTPYLETNLGQPVIQQFEQDGRKLNVVVKDSLTLVRKNG
TFLTLRQVFGKDLGYTLYYWKSSSSGKKTAKTNTNEFLIDVDKGENYCFSVQAVIPSR
TVNRKSTDSPVECMGQEKGEFREIFYIIGAVAFVVIILVIILAISLHKCRKAGVGQSWKE
NSPLNVSTSKIVAPVKQTLNFDLLKLAGDVESNPGPGSTMVSKGEELFTGVVPIL
VELDGDVNGHKFSVSGEGEGDATYGKLTLKFICTTGKLPVPWPTLVTTLTYGVQ
CFSRYPDHMKQHDFFKSAMPEGYVQERTIFFKDDGNYKTRAEVKFEGDTLVNR
IELKGIDFKEDGNILGHKLEYNYNSHNVYIMADKQKNGIKVNFKIRHNIEDGSV
QLADHYQQNTPIGDGPVLLPDNHYLSTQSALSKDPNEKRDHMVLLEFVTAAGI
TLGMDELYK
SEQ ID NO: 25
hTF5 (185-225 mm): 
METPAWPRVPRPETAVARTLLLGWVFAQVAGASGTTNTVAAYNLTWKSTNFKTILEWE
PKPVNQVYTVQISTKSGDWKSKCFYTTDTECDLTDEIVKDVKQTYLARVFSYPAGNV
ESTGSAGEPLYENSPEFTPYLETNLGQPTIQSFEQVGTKVNVTVEDERTLVRRNNTFL
SLRDVFGKDLIYIITYRKGSSTGKKTNITNTNEFSIDVEEGVSYCFFVQAMIPSRTV
 
NRKSTDSPVECMGQEKGEFREIFYIIGAVAFVVIILVIILAISLHKCRKAGVGQSWKENS
PLNVSTSKIVAPVKQTLNFDLLKLAGDVESNPGPGSTMVSKGEELFTGVVPILVE
LDGDVNGHKFSVSGEGEGDATYGKLTLKFICTTGKLPVPWPTLVTTLTYGVQCF
SRYPDHMKQHDFFKSAMPEGYVQERTIFFKDDGNYKTRAEVKFEGDTLVNRIE
LKGIDFKEDGNILGHKLEYNYNSHNVYIMADKQKNGIKVNFKIRHNIEDGSVQL
ADHYQQNTPIGDGPVLLPDNHYLSTQSALSKDPNEKRDHMVLLEFVTAAGITL
GMDELYK
SEQ ID NO: 26
hTF6 (226-250 mm):

METPAWPRVPRPETAVARTLLLGWVFAQVAGASGTTNTVAAYNLTWKSTNFKTILEWE
PKPVNQVYTVQISTKSGDWKSKCFYTTDTECDLTDEIVKDVKQTYLARVFSYPAGNV
ESTGSAGEPLYENSPEFTPYLETNLGQPTIQSFEQVGTKVNVTVEDERTLVRRNNTFL
SLRDVFGKDLIYTLYYWKSSSSGKKTAKTNTNEFLIDVDKGENYCFSVQAVIFSRKTN
QNSPGSSTVCTEQWKSFLGEIFYIIGAVAFVVIILVIILAISLHKCRKAGVGQSWKENS
PLNVSTSKIVAPVKQTLNFDLLKLAGDVESNPGPGSTMVSKGEELFTGVVPILVE
LDGDVNGHKFSVSGEGEGDATYGKLTLKFICTTGKLPVPWPTLVTTLTYGVQCF
SRYPDHMKQHDFFKSAMPEGYVQERTIFFKDDGNYKTRAEVKFEGDTLVNRIE
LKGIDFKEDGNILGHKLEYNYNSHNVYIMADKQKNGIKVNFKIRHNIEDGSVQL
ADHYQQNTPIGDGPVLLPDNHYLSTQSALSKDPNEKRDHMVLLEFVTAAGITL
GMDELYK
SEQ ID NO: 27

Note: The human TF sequence is italicized, the mouse TF sequence is underlined, the 2A sequence is double-underlined, and the EGFP sequence is indicated by a wavy line. Epitope analysis was performed through FACS binding experiments. The antibodies of the present disclosure bind to a specific epitope of TF.

### Sequences of positive control antibodies

The sequence of the positive control antibody TF011 was derived from the monoclonal antibody sequence of tisotumab vedotin, the variable region sequence of antibody H39 was derived from WO2018036117A1, and the variable region sequence of antibody 10H10 was derived from WO2012125559A1.
Sequence of heavy chain of antibody TF011 (monoclonal antibody sequence of tisotumab vedotin): 
EVQLLESGGGLVQPGGSLRLSCAASGFTFSNYAMSWVRQAPGKGLEWVSSISGS
GDYTYYTDSVKGRFTISRDNSKNTLYLQMNSLRAEDTAVYYCARSPWGYYLDS
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKRV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
 
EALHNHYTQKSLSLSPGK
SEQ ID NO: 28
Sequence of light chain of antibody TF011 (monoclonal antibody sequence of tisotumab vedotin): 
DIQMTQSPPSLSASAGDRVTITCRASQGISSRLAWYQQKPEKAPKSLIYAASSLQS
GVPSRFSGSGSGTDFTLTISSLQPEDFATYYCQQYNSYPYTFGQGTKLEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 29
Sequence of heavy chain of antibody H39 (variable region + human IgG1 constant region): 
EVQLVQSGAEVKKPGESLKISCKGSGYSFTSYWMNWVRQMPGKGLEWMGMI
YPADSETRLNQKFKDQATLSVDKSISTAYLQWSSLKASDTAMYYCAREDYGSS
DYWGQGTTVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 30
Sequence of light chain of antibody H39 (variable region + human 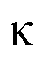 constant region): 
DIQLTQSPSSLSASVGDRVTITCSASSSVSYMNWYQQKPGKSPKIWIYGISNLAS
GVPSRFSGSGSGTDFTLTISSLQPEDFATYYCQQKSSFPWTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 31
Sequence of heavy chain of antibody 10H10 (variable region + human IgG1 constant region): 
QVHLQQSGAELMKPGASVKISCKASGYTFITYWIEWVKQRPGHGLEWIGDILP
GSGSTNYNENFKGKATFTADSSSNTAYMQLSSLTSEDSAVYYCARSGYYGNSGF
AYWGQGTLVTVSAASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 32
Sequence of light chain of antibody 10H10 (variable region + human κ constant region): 
DIVMTQSPSSLTVTAGEKVTMSCKSSQSLLSSGNQKNYLTWYQQIPGQPPKLLIY
WASTRESGVPDRFTGSGSGTDFTLTINSVQAEDLAVYYCQNDYTYPLTFGAGTK
LELKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC
SEQ ID NO: 33
Sequence of heavy chain of TF011-mIgG2a (variable region + mouse mIgG2a constant region): 
EVQLLESGGGLVQPGGSLRLSCAASGFTFSNYAMSWVRQAPGKGLEWVSSISGS
GDYTYYTDSVKGRFTISRDNSKNTLYLQMNSLRAEDTAVYYCARSPWGYYLDS
WGQGTLVTVSSAKTTAPSVYPLAPVCGDTTGSSVTLGCLVKGYFPEPVTLTWNS
GSLSSGVHTFPAVLQSDLYTLSSSVTVTSSTWPSQSITCNVAHPASSTKVDKKIEP
RGPTIKPCPPCKCPAPNLLGGPSVFIFPPKIKDVLMISLSPIVTCVVVDVSEDDPDV
QISWFVNNVEVHTAQTQTHREDYNSTLRVVSALPIQHQDWMSGKEFKCKVNN
KDLPAPIERTISKPKGSVRAPQVYVLPPPEEEMTKKQVTLTCMVTDFMPEDIYVE
WTNNGKTELNYKNTEPVLDSDGSYFMYSKLRVEKKNWVERNSYSCSVVHEGL
HNHHTTKSFSRTPGK
SEQ ID NO: 34
Sequence of light chain of TF011-mκ (variable region + mouse κ constant region):

DIQMTQSPPSLSASAGDRVTITCRASQGISSRLAWYQQKPEKAPKSLIYAASSLQS
GVPSRFSGSGSGTDFTLTISSLQPEDFATYYCQQYNSYPYTFGQGTKLEIKRADA
APTVSIFPPSSEQLTSGGASVVCFLNNFYPKDINVKWKIDGSERQNGVLNSWTD
QDSKDSTYSMSSTLTLTKDEYERHNSYTCEATHKTSTSPIVKSFNRNEC
SEQ ID NO: 35

**Negative control antibody hIgG1** (in which the VH/VL is derived from the antibody C25 of patent document US6114143A):
Amino acid sequence of heavy chain of antibody hIgG1 (C25 heavy chain variable region + human IgG1 constant region): 
QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK
SEQ ID NO: 103
Amino acid sequence of light chain of antibody hIgG1 (C25 light chain variable region + human κ constant region):

DIQMTQSPSSLSASVGDRVTMSCKSSQSLLNSGDQKNYLTWYQQKPGKAPKLLI
YWASTGESGVPSRFSGSGSGTDFTFTISSLQPEDIATYYCQNDYSYPWTFGQGTK
VEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC
SEQ ID NO: 104

Note: The variable region sequences are underlined.

### Example 2: Purification of Antigen Proteins and Antibody Proteins

The His-tagged antigens were affinity-purified on a nickel column (Ni Sepharose excel 25 mL, GE Healthcare, 17-3712-01), and the Fc-tagged antigens or antibodies were affinity-purified on ProteinA-MabSelect SuRe 25 mL (GE Healthcare, 17-5438-01). The affinity-purified proteins were then subjected to size exclusion chromatography to remove aggregates or cleavage fragments. The specific procedures were as follows: Nickel column purification: A nickel column was equilibrated by rinsing with PBS solution, and the supernatant after cell transfection was centrifuged at high speed to remove impurities and then loaded onto the column. When the nickel column was rinsed with PBS solution until the A₂₈₀ reading dropped to the baseline, the nickel column was rinsed with a PBS solution containing 10 mM imidazole to remove non-specifically bound impurity proteins, and then the target protein was eluted with a PBS solution containing 300 mM imidazole. The elution peak liquid was collected and buffer-exchanged into PBS for storage.

Size exclusion chromatography purification: An SEC column (GE, superdex75) was equilibrated by rinsing with PBS solution. The protein obtained by nickel column purification or Protein A purification was loaded onto the column (loading volume ≤ 3% column volume), and elution was performed with PBS solution as the mobile phase. The elution peaks were collected, and the fraction where the target protein was present was identified by SDS-PAGE.

Affinity chromatography for antibodies or Fc fusion proteins: The supernatant after cell transfection was centrifuged at high speed to remove impurities and then loaded onto a Protein A column. When the Protein A column was rinsed with PBS solution until the A₂₈₀ reading dropped to the baseline, the target protein was eluted with 100 mM acetic acid (pH 3.5). The eluate was neutralized with 1 M Tris-HCl (pH 8.0) and buffer-exchanged into PBS. If necessary, aggregates can be further removed subsequently using gel chromatography (Superdex200, GE).

### Example 3: Preparation of Anti-Human TF Monoclonal Antibody

### 3.1 Immunization

The anti-human TF monoclonal antibody was obtained by hybridoma technology. Laboratory SJL white mice, female, aged 6-8 weeks (Shanghai SLAC Laboratory Animal Co., Ltd., animal production license number: SCXK (Shanghai) 2017-0005). Housing environment: SPF. The purchased mice were housed in a laboratory environment for 1 week, with a 12/12-hour light/dark cycle, at a temperature of 20-25 °C with humidity at 40-60%. The acclimatized mice were immunized according to the following scheme.

### 3.2 Immunization scheme

The immunizing antigens for anti-human TF antibody 67 were hTF-NIH/3T3 cells (SEQ ID NO: 12), cTF-NIH/3T3 cells (SEQ ID NO: 13), hTF-his protein (SEQ ID NO: 5), and cTF-mFc protein (SEQ ID NO: 106), and the immunization mode was cell and protein immunization. hTF-NIH/3T3 cells were used for the first immunization. Before the cell immunization, each mouse was intraperitoneally injected with 0.1 mL of TiterMax^{®} Gold Adjuvant (Sigma, T2684) in advance, and after half an hour, each mouse was intraperitoneally injected with 0.1 mL of a cell suspension diluted to 10⁸ cells/mL with normal saline. On day 14, a second immunization was performed with cTF-NIH/3T3 cells. Each mouse was intraperitoneally injected with 0.1 mL of a cell suspension diluted to 10⁸ cells/mL with normal saline. On day 35, a third immunization was performed with hTF-his protein at 50 µg/mouse/immunization. The ratio of the antigen to the adjuvant (TiterMax^{®} Gold Adjuvant) was 1:1. The antigen and the adjuvant were fully emulsified before inoculation. On day 49, a fourth immunization was performed with hTF-his protein at 50 µg/mouse/immunization. The ratio of the antigen to the adjuvant (TiterMax^{®} Gold Adjuvant) was 1:1. The antigen and the adjuvant were fully emulsified before inoculation. Blood was collected on days 29, 47, and 61, and the antibody titer in mouse serum was determined by ELISA and FACS. After the 4th immunization, mice in which the antibody titer in the serum was high and tended to plateau were selected for splenocyte fusion. Boost immunization was performed 3 days before splenocyte fusion. An antigen protein solution of hTF-his and cTF-mFc mixed in a mass ratio of 1:1 in normal saline was injected intraperitoneally (i.p.) at 50 µg/mouse.

The immunizing antigens for anti-human TF antibody 310 were hTF-NIH/3T3 cells, cTF-NIH/3T3 cells, hTF-CHO-K1 cells, and cTF-CHO-Kl cells, and the immunization mode was cell immunization. hTF-NIH/3T3 cells were used for the first immunization. Before the cell immunization, each mouse was intraperitoneally injected with 0.1 mL of TiterMax^{®} Gold Adjuvant in advance, and after half an hour, each mouse was intraperitoneally injected with 0.1 mL of a cell suspension diluted to 10⁸ cells/mL with normal saline. On day 59, a second immunization was performed with cTF-NIH/3T3 cells. Each mouse was intraperitoneally injected with 0.1 mL of a cell suspension diluted to 10⁸ cells/mL with normal saline. On day 74, a third immunization was performed with hTF-CHO-K1 cells. Each mouse was intraperitoneally injected with 0.1 mL of a cell suspension diluted to 10⁸ cells/mL with normal saline. On day 90, a fourth immunization was performed with cTF-CHO-K1 cells. Each mouse was intraperitoneally injected with 0.1 mL of a cell suspension diluted to 10⁸ cells/mL with normal saline. On day 104, a fifth immunization was performed with hTF-CHO-K1 cells. Each mouse was intraperitoneally injected with 0.1 mL of a cell suspension diluted to 10⁸ cells/mL with normal saline. Blood was collected on days 68, 85, and 118, and the antibody titer in mouse serum was determined by ELISA and FACS. After the fifth immunization, mice in which the antibody titer in serum was high and tended to plateau were selected for splenocyte fusion. Boost immunization was performed 3 days before splenocyte fusion. An antigen protein solution of hTF-his (SEQ ID NO: 5) and cTF-his (SEQ ID NO: 8) mixed in a mass ratio of 1:1 in normal saline was injected intraperitoneally (i.p.) at 50 µg/mouse.

### 3.3 Splenocyte fusion

Spleen lymphocytes and Sp2/0-Ag14 cells (myeloma cells, ATCC, CRL-8287^{™}) were electrofused to obtain hybridoma cells. The hybridoma cells were resuspended in complete culture medium (IMDM culture medium containing 20% FBS, 1 × HAT, and 1× OPI) at a density of 3-4 × 10⁵/mL and seeded in a 96-well cell culture plate at 150 µL/well. After 3-4 days of incubation at 37 °C with 5% CO₂, the supernatant was removed, and HT complete culture medium (IMDM culture medium containing 20% FBS, 1× HT, and 1× OPI) was added at 200 µL/well. After 3-5 days of culture at 37 °C with 5% CO₂, an assay was performed.

### 3.4 Screening of hybridoma cells

When the hybridoma cell density reached 90%, the cell supernatant was collected using a pipette, and primary screening was performed in a 96-well plate using a cell-based ELISA method. The positive wells selected by screening were then transferred to a 24-well plate. When the cell density reached 90% after 2-3 days, the cell supernatant was collected using a pipette, and rescreening was performed in a 24-well plate using experiments such as FACS and/or competition and/or blood coagulation. Single-well cells were selected and subcloned to obtain single-cell clones. The subclone cell supernatant was collected and re-assayed. Through the above screening experiment, hybridoma clones were obtained and used for subsequent experiments.

### Example 4: Determination of Amino Acid Sequences of Variable Regions of Mouse Monoclonal Antibody

The hybridoma monoclonal cell strain obtained in Example 3 was subjected to variable region amino acid sequence determination. The mouse hybridoma variable region sequences were combined with human constant region sequences to form a human-mouse chimeric antibody (CHAb) sequence, and the chimeric antibody was expressed and identified. The specific procedures were as follows:
Firstly, the total RNA of the hybridoma monoclonal cell strain was obtained by a conventional well-known method. Then, cDNA was prepared using a reverse transcription kit PrimeScript^{™} IV 1st strand cDNA Synthesis Mix (TAKARA, 6215A).

PCR amplification was performed with the cDNA as a template to obtain PCR fragments containing the light and heavy chain variable regions, and the corresponding variable region sequences were obtained by sequencing the PCR fragments. By conventional well-known methods, the obtained light and heavy chain sequences were cloned into a pTT5 vector, a recombinant monoclonal antibody was expressed, and its activity was verified. The amino acid residues of the VH/VL CDRs of the anti-human TF antibody were determined and annotated using the Kabat numbering scheme.
Sequences of mouse hybridoma cell monoclonal antibody 67#:
Heavy chain variable region of 67#: 
QVQLKQSGPGLVQPSQSLSITCIVSGFSLTNYGVNWVRQSPRKGLEWLGVIWSR
GNTDYNAAFISRLIISKDNSKSQVFLKMNRLQADDTAIYYCARKDAVVATDALD
YWGQGTSVTVSS
SEQ ID NO: 36
Light chain variable region of 67#:

DIVMTQSHKFMSTSLGDRVSITCKASQDVSTAVAWYQQKPGQSPKLLIYSTSYR
YTGVPDRFTGSGSGTDFTFTISSVQAEDLAVYYCQQHYSTPYTFGGGTKLEIK
SEQ ID NO: 37

Sequences of mouse hybridoma cell monoclonal antibody 310#:
Heavy chain variable region of 310#: 
EVQLQQSGPELVKPGASLKISCKASGYSFTGYYMNWVKQSPEKSLEWIGEINPS
TGGSTYNQKFKAKATLTVDQSSNTAYMQLKSLTSEDSAVYYCARRELGGPFGY
WGQGTPVTVSA
SEQ ID NO: 38
Light chain variable region of 310#:

SIVMTQTPKFLLVSSGDRVTITCKASQSVSNGIAWYQQKPGQSPKLLIYFASNRY
TGVPDRFTGSGYGTDFTFTISTVQAEDLAVYFCQQDYSSPWTFGGGTKLEIK
SEQ ID NO: 39

**Table 3. CDRs of murine antibody 67#**

| **CDR** | **Sequence** | **Sequence No.** |
|---|---|---|
| HCDR1 | NYGVN | SEQ ID NO: 40 |
| HCDR2 | VIWSRGNTDYNAAFIS | SEQ ID NO: 41 |
| HCDR3 | KDAVVATDALDY | SEQ ID NO: 42 |
| LCDR1 | KASQDVSTAVA | SEQ ID NO: 43 |
| LCDR2 | STSYRYT | SEQ ID NO: 44 |
| LCDR3 | QQHYSTPYT | SEQ ID NO: 45 |

**Table 4. CDRs of murine antibody 310#**

| **CDR** | **Sequence** | **Sequence No.** |
|---|---|---|
| HCDR1 | GYYMN | SEQ ID NO: 46 |
| HCDR2 | EINPSTGGSTYNQKFKA | SEQ ID NO: 47 |
| HCDR3 | RELGGPFGY | SEQ ID NO: 48 |
| LCDR1 | KASQSVSNGIA | SEQ ID NO: 49 |
| LCDR2 | FASNRYT | SEQ ID NO: 50 |
| LCDR3 | QQDYSSPWT | SEQ ID NO: 51 |

Heavy chain constant region of human IgG1: 







SEQ ID NO: 54
Human κ light chain constant region:



SEQ ID NO: 55

The heavy and light chain sequences of chimeric antibody CH67 are shown below:
Heavy chain of CH67: 
QVQLKQSGPGLVQPSQSLSITCIVSGFSLTNYGVNWVRQSPRKGLEWLGVIWSR
GNTDYNAAFISRLIISKDNSKSQVFLKMNRLQADDTAIYYCARKDAVVATDALD
YWGQGTSVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK
SEQ ID NO: 107
Light chain of CH67:

DIVMTQSHKFMSTSLGDRVSITCKASQDVSTAVAWYQQKPGQSPKLLIYSTSYR
YTGVPDRFTGSGSGTDFTFTISSVQAEDLAVYYCQQHYSTPYTFGGGTKLEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 108

The heavy and light chain sequences of chimeric antibody CH310 are shown below:
Heavy chain of CH310: 
EVQLQQSGPELVKPGASLKISCKASGYSFTGYYMNWVKQSPEKSLEWIGEINPS
TGGSTYNQKFKAKATLTVDQSSNTAYMQLKSLTSEDSAVYYCARRELGGPFGY
WGQGTPVTVSAASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
 
ALHNHYTQKSLSLSPGK
SEQ ID NO: 109
Light chain of CH310:

SIVMTQTPKFLLVSSGDRVTITCKASQSVSNGIAWYQQKPGQSPKLLIYFASNRY
TGVPDRFTGSGYGTDFTFTISTVQAEDLAVYFCQQDYSSPWTFGGGTKLEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 110

### Example 5: Humanization of Anti-Human TF Antibody 310#

By alignment with the IMGT human antibody heavy and light chain variable region germline gene database through the MOE software, heavy and light chain variable region germline genes with high homology to 310# were selected as templates, and CDRs of this murine antibody were grafted into corresponding human templates to form variable region sequences in the following order: FR1-CDR1-FR2-CDR2-FR3-CDR3-FR4. Illustratively, the amino acid residues of CDRs in the specific examples below were determined and annotated using the Kabat numbering scheme.

For the humanized antibody of the antibody 310#, the FR1, FR2, and FR3 of IGKV4-1*01 or IGKV1-33*01 and the FR4 of IGKJ4*01 were selected as a light chain framework region template, and the FR1, FR2, and FR3 of IGHV1-46*01 and the FR4 of IGHJ6*01 were selected as a heavy chain framework region template. Optionally, the amino acid residue(s) at position(s) 1, 9, 10, 31, 43, 67, and/or 73 in the light chain variable region of the humanized antibody were/was substituted; and/or the amino acid residue(s) at position(s) 1, 28, 69, 71, 73, 76, and/or 78 in the heavy chain variable region of the humanized antibody were/was substituted.

**Table 5. Humanization design for 310#**

| VL | | VH | |
|---|---|---|---|
| VL1 | Graft(IGKV4-1*01) | VH1 | Graft(IGHV1-46*01) + Q1E, T28S, R71V, T73Q |
| VL2 | Graft(IGKV4-1*01) + D1S, P43S, S67Y | VH2 | Graft(IGHV1-46*01) + Q1E, T28S, M69L, R71V, T73Q, S76N, V78A |
| VL3 | Graft(IGKV4-1*01) + D1S, D9K, S10F, P43S, S67Y, L73F | | |
| VL4 | Graft(IGKV1-33*01) | | |
| VL5 | Graft(IGKV1-33*01) + D1S, A43S, S67Y | | |
| VL6 | Graft(IGKV1-33*01) + D1S, A43S, S67Y + N31Q | | |
| VL7 | Graft(IGKV1-33*01) + D1S, A43S, S67Y + N31S | | |
| VL8 | Graft(IGKV1-33*01) + D1S, A43S, S67Y + N31T | | |
| FR4 | IGKJ4*01 | FR4 | IGHJ6*01 |

Note: Illustratively, D1S indicates that D at position 1 is mutated to S according to the Kabat numbering scheme, and so on for others.

**Table 6. CDRs of 310# humanized antibody**

| **Variable region** | **CDR** | **Sequence** | **Sequence No.** |
|---|---|---|---|
| All | HCDR1 | GYYMN | SEQ ID NO: 46 |
| All | HCDR2 | EINPSTGGSTYNQKFKA | SEQ ID NO: 47 |
| All | HCDR3 | RELGGPFGY | SEQ ID NO: 48 |
| VL1/VL2/VL3/VL4/VLS | LCDR1 | KASQSVSNGIA | SEQ ID NO: 49 |
| VL6 | LCDR1 | KASQSVSQGIA | SEQ ID NO:111 |
| VL7 | LCDR1 | KASQSVSSGIA | SEQ ID NO:112 |
| VL8 | LCDR1 | KASQSVSTGIA | SEQ ID NO:113 |
| All | LCDR2 | FASNRYT | SEQ ID NO: 50 |
| All | LCDR3 | QQDYSSPWT | SEQ ID NO: 51 |

The heavy and light chain variable region sequences of the 310# humanized antibody are shown below:
> hu310-VH1: 
EVQLVQSGAEVKKPGASVKVSCKASGYSFTGYYMNWVRQAPGQGLEWMGEI
NPSTGGSTYNQKFKARVTMTVDQSTSTVYMELSSLRSEDTAVYYCARRELGGP
FGYWGQGTTVTVSS
SEQ ID NO: 52
> hu310-VH2: 
EVQLVQSGAEVKKPGASVKVSCKASGYSFTGYYMNWVRQAPGQGLEWMGEI
NPSTGGSTYNQKFKARVTLTVDQSTNTAYMELSSLRSEDTAVYYCARRELGGP
FGYWGQGTTVTVSS
SEQ ID NO: 114
> hu310-VL1: 
DIVMTQSPDSLAVSLGERATINCKASQSVSNGIAWYQQKPGQPPKLLIYFASNRY
TGVPDRFSGSGSGTDFTLTISSLQAEDVAVYYCQQDYSSPWTFGGGTKVEIK
SEQ ID NO: 115
> hu310-VL2: 
SIVMTQSPDSLAVSLGERATINCKASQSVSNGIAWYQQKPGQSPKLLIYFASNRY
TGVPDRFSGSGYGTDFTLTISSLQAEDVAVYYCQQDYSSPWTFGGGTKVEIK
SEQ ID NO: 53
> hu310-VL3: 
SIVMTQSPKFLAVSLGERATINCKASQSVSNGIAWYQQKPGQSPKLLIYFASNRY
TGVPDRFSGSGYGTDFTFTISSLQAEDVAVYYCQQDYSSPWTFGGGTKVEIK
SEQ ID NO: 116
> hu310-VL4: 
DIQMTQSPSSLSASVGDRVTITCKASQSVSNGIAWYQQKPGKAPKLLIYFASNRY
TGVPSRFSGSGSGTDFTFTISSLQPEDIATYYCQQDYSSPWTFGGGTKVEIK
SEQ ID NO: 117
> hu310-VL5: 
SIQMTQSPSSLSASVGDRVTITCKASQSVSNGIAWYQQKPGKSPKLLIYFASNRY
TGVPSRFSGSGYGTDFTFTISSLQPEDIATYYCQQDYSSPWTFGGGTKVEIK
SEQ ID NO: 118
> hu310-VL6: 
SIQMTQSPSSLSASVGDRVTITCKASQSVSQGIAWYQQKPGKSPKLLIYFASNRY
TGVPSRFSGSGYGTDFTFTISSLQPEDIATYYCQQDYSSPWTFGGGTKVEIK
SEQ ID NO: 119
> hu310-VL7: 
SIQMTQSPSSLSASVGDRVTITCKASQSVSSGIAWYQQKPGKSPKLLIYFASNRY
TGVPSRFSGSGYGTDFTFTISSLQPEDIATYYCQQDYSSPWTFGGGTKVEIK
SEQ ID NO: 120
> hu310-VL8:

SIQMTQSPSSLSASVGDRVTITCKASQSVSTGIAWYQQKPGKSPKLLIYFASNRY
TGVPSRFSGSGYGTDFTFTISSLQPEDIATYYCQQDYSSPWTFGGGTKVEIK
SEQ ID NO: 121

Note: In the above sequences, the regions are arranged in the following order: FR1-CDR1-FR2-CDR2-FR3-CDR3-FR4; the CDR sequences determined according to the Kabat numbering scheme are underlined; the FR sequences are not underlined; the amino acid point mutations are double-underlined and shown in bold.

The heavy and light chain variable regions of the 310# humanized antibody were recombined with the human heavy chain IgG1 constant region (SEQ ID NO: 54) and the human light chain Kappa constant region (SEQ ID NO: 55), respectively, to obtain humanized antibodies as shown in Table 7 below.

**Table 7. Humanized antibodies of 310#**

| Variable region | VH1 | VH2 |
|---|---|---|
| VL1 | hu310-1 | hu310-9 |
| VL2 | hu310-2 | hu310-10 |
| VL3 | hu310-3 | hu310-11 |
| VL4 | hu310-4 | hu310-12 |
| VL5 | hu310-5 | hu310-13 |
| VL6 | hu310-6 | hu310-14 |
| VL7 | hu310-7 | hu310-15 |
| VL8 | hu310-8 | hu310-16 |

The heavy and light chain sequences of the 310# humanized antibodies are shown below:
Heavy chain of hu310-1/hu310-2/hu310-3/hu310-4/hu310-5/hu310-6/hu310-7/hu310-8: 
EVQLVQSGAEVKKPGASVKVSCKASGYSFTGYYMNWVRQAPGQGLEWMGEI
NPSTGGSTYNQKFKARVTMTVDQSTSTVYMELSSLRSEDTAVYYCARRELGGP
FGYWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
 
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 56
Heavy chain of hu310-9/hu310-10/hu310-11/hu310-12/hu310-13/hu310-14/hu310-15/hu310-16: 
EVQLVQSGAEVKKPGASVKVSCKASGYSFTGYYMNWVRQAPGQGLEWMGEI
NPSTGGSTYNQKFKARVTLTVDQSTNTAYMELSSLRSEDTAVYYCARRELGGP
FGYWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 122
Light chain of hu310-1/hu310-9: 
DIVMTQSPDSLAVSLGERATINCKASQSVSNGIAWYQQKPGQPPKLLIYFASNRY
TGVPDRFSGSGSGTDFTLTISSLQAEDVAVYYCQQDYSSPWTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 123
Light chain of hu310-2/hu310-10: 
SIVMTQSPDSLAVSLGERATINCKASQSVSNGIAWYQQKPGQSPKLLIYFASNRY
TGVPDRFSGSGYGTDFTLTISSLQAEDVAVYYCQQDYSSPWTFGGGTKVEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 57
Light chain of hu310-3/hu310-11: 
SIVMTQSPKFLAVSLGERATINCKASQSVSNGIAWYQQKPGQSPKLLIYFASNRY
TGVPDRFSGSGYGTDFTFTISSLQAEDVAVYYCQQDYSSPWTFGGGTKVEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 124
Light chain of hu310-4/hu310-12: 
DIQMTQSPSSLSASVGDRVTITCKASQSVSNGIAWYQQKPGKAPKLLIYFASNRY
TGVPSRFSGSGSGTDFTFTISSLQPEDIATYYCQQDYSSPWTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
 SEQ ID NO: 125
Light chain of hu310-5/hu310-13: 
SIQMTQSPSSLSASVGDRVTITCKASQSVSNGIAWYQQKPGKSPKLLIYFASNRY
TGVPSRFSGSGYGTDFTFTISSLQPEDIATYYCQQDYSSPWTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 126
Light chain of hu310-6/hu310-14: 
SIQMTQSPSSLSASVGDRVTITCKASQSVSQGIAWYQQKPGKSPKLLIYFASNRY
TGVPSRFSGSGYGTDFTFTISSLQPEDIATYYCQQDYSSPWTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 127
Light chain of hu310-7/hu310-15: 
SIQMTQSPSSLSASVGDRVTITCKASQSVSSGIAWYQQKPGKSPKLLIYFASNRY
TGVPSRFSGSGYGTDFTFTISSLQPEDIATYYCQQDYSSPWTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 128
Light chain of hu310-8/hu310-16:

SIQMTQSPSSLSASVGDRVTITCKASQSVSTGIAWYQQKPGKSPKLLIYFASNRY
TGVPSRFSGSGYGTDFTFTISSLQPEDIATYYCQQDYSSPWTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 129

### Example 6: Humanization of Anti-Human TF Antibody 67#

After the light and heavy chain sequences of the murine anti-TF monoclonal antibody 67# obtained in Example 4 were aligned with the IMGT human antibody heavy and light chain variable region germline gene database through the MOE software for homology, heavy and light chain variable region germline genes highly homologous with 67# were selected as templates, and the CDRs of this murine antibody were grafted into corresponding human templates to form variable region sequences in the following order: FR1-CDR1-FR2-CDR2-FR3-CDR3-FR4. Illustratively, the amino acid residues of CDRs in the specific examples below were determined and annotated using the Kabat numbering scheme.

For the human germline light chain template of antibody 67#, the FR1, FR2, and FR3 of IGKV4-1*01 or IGKV1-33*01 and the FR4 of IGKJ4*01 were selected. For the human germline heavy chain template, the FR1, FR2, and FR3 of IGHV2-26*01 or IGHV4-30-4*01 and the FR4 of IGHJ6*01 were selected. Optionally, the amino acid residue(s) at position(s) 8, 9, 10, 43, and/or 73 in the light chain variable region of the humanized antibody were/was substituted; and/or the amino acid residue(s) at position(s) 1, 6, 16, 27, 29, 30, 42, 44, 49, 71, 73, 76, 78, and/or 81 in the heavy chain variable region of the humanized antibody were/was substituted.

**Table 8. Humanization design for 67#**

| **VL** | | **VH** | |
|---|---|---|---|
| VL1 | Graft(IGKV4-1*01) + D9K | VH1 | Graft(IGHV2-26*01) + Q1E, E6Q, E16Q, S30T, G42R, T81K |
| VL2 | Graft(IGKV4-1*01) + D9K, P43S, L73F | VH2 | Graft(IGHV2-26*01) + Q1E, E6Q, E16Q, S30T, G42R, A44G, A49G, T73N, T81K |
| VL3 | Graft(IGKV1-33*01) | VH3 | Graft(IGHV4-30-4*01) + Q1E, G27F, I29L, S30T, V71K, F78V |
| VL4 | Graft(IGKV1-33*01) + P8H, S9K, S10F, A43S | VH4 | Graft(IGHV4-30-4*01) + Q1E, G27F, I29L, S30T, G42R, V71K, T73N, N76S, F78V |
| FR4 | IGKJ4*01 | FR4 | IGHJ6*01 |

Note: For example, G42R indicates that the G at position 42 is mutated back to R according to the Kabat numbering scheme.

The heavy and light chain variable region sequences of the 67# humanized antibody are shown below:
>hu67-VH1: 
EVTLKQSGPVLVKPTQTLTLTCTVSGFSLTNYGVNWIRQPPRKALEWLAVIWSR
GNTDYNAAFISRLTISKDTSKSQVVLKMTNMDPVDTATYYCARKDAVVATDAL
DYWGQGTTVTVSS
SEQ ID NO: 130
>hu67-VH2: 
EVTLKQSGPVLVKPTQTLTLTCTVSGFSLTNYGVNWIRQPPRKGLEWLGVIWS
RGNTDYNAAFISRLTISKDNSKSQVVLKMTNMDPVDTATYYCARKDAVVATDA
LDYWGQGTTVTVSS
SEQ ID NO: 131
>hu67-VH3: 
EVQLQESGPGLVKPSQTLSLTCTVSGFSLTNYGVNWIRQPPGKGLEWIGVIWSR
GNTDYNAAFISRVTISKDTSKNQVSLKLSSVTAADTAVYYCARKDAVVATDALD
YWGQGTTVTVSS
SEQ ID NO: 58
>hu67-VH4: 
EVQLQESGPGLVKPSQTLSLTCTVSGFSLTNYGVNWIRQPPRKGLEWIGVIWSR
GNTDYNAAFISRVTISKDNSKSQVSLKLSSVTAADTAVYYCARKDAVVATDALD
YWGQGTTVTVSS
 SEQ ID NO: 132
>67VL-VL1: 
DIVMTQSPKSLAVSLGERATINCKASQDVSTAVAWYQQKPGQPPKLLIYSTSYRY
TGVPDRFSGSGSGTDFTLTISSLQAEDVAVYYCQQHYSTPYTFGGGTKVEIK
SEQ ID NO: 133
>67VL-VL2: 
DIVMTQSPKSLAVSLGERATINCKASQDVSTAVAWYQQKPGQSPKLLIYSTSYRY
TGVPDRFSGSGSGTDFTFTISSLQAEDVAVYYCQQHYSTPYTFGGGTKVEIK
SEQ ID NO: 134
>67VL-VL3: 
DIQMTQSPSSLSASVGDRVTITCKASQDVSTAVAWYQQKPGKAPKLLIYSTSYRY
TGVPSRFSGSGSGTDFTFTISSLQPEDIATYYCQQHYSTPYTFGGGTKVEIK
SEQ ID NO: 59
>67VL-VL4:

DIQMTQSHKFLSASVGDRVTITCKASQDVSTAVAWYQQKPGKSPKLLIYSTSYR
YTGVPSRFSGSGSGTDFTFTISSLQPEDIATYYCQQHYSTPYTFGGGTKVEIK
SEQ ID NO: 135

Note: In the above sequences, the regions are arranged in the following order: FR1-CDR1-FR2-CDR2-FR3-CDR3-FR4; the CDR sequences determined according to the Kabat numbering scheme are underlined; the FR sequences are not underlined; the amino acid point mutations are double-underlined and shown in bold.

The heavy and light chain variable regions of the 67# humanized antibody were recombined with the human heavy chain IgG1 constant region (SEQ ID NO: 54) and the human light chain Kappa constant region (SEQ ID NO: 55), respectively, to obtain humanized antibodies as shown in Table 9 below.

**Table 9. Humanized antibodies of 67#**

| Variable region | VH1 | VH2 | VH3 | VH4 |
|---|---|---|---|---|
| VL1 | hu67-1 | hu67-2 | hu67-3 | hu67-4 |
| VL2 | hu67-5 | hu67-6 | hu67-7 | hu67-8 |
| VL3 | hu67-9 | hu67-10 | hu67-11 | hu67-12 |
| VL4 | hu67-13 | hu67-14 | hu67-15 | hu67-16 |

The heavy and light chain sequences of the humanized antibodies are shown below:
Heavy chain sequence of hu67-1/hu67-5/hu67-9/hu67-13: 
EVTLKQSGPVLVKPTQTLTLTCTVSGFSLTNYGVNWIRQPPRKALEWLAVIWSR
GNTDYNAAFISRLTISKDTSKSQVVLKMTNMDPVDTATYYCARKDAVVATDAL
DYWGQGTTVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
 
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 136
Heavy chain sequence of hu67-2/hu67-6/hu67-10/hu67-14: 
EVTLKQSGPVLVKPTQTLTLTCTVSGFSLTNYGVNWIRQPPRKGLEWLGVIWS
RGNTDYNAAFISRLTISKDNSKSQVVLKMTNMDPVDTATYYCARKDAVVATDA
LDYWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 137
Heavy chain sequence of hu67-3/hu67-7/hu67-11/hu67-15: 
EVQLQESGPGLVKPSQTLSLTCTVSGFSLTNYGVNWIRQPPGKGLEWIGVIWSR
GNTDYNAAFISRVTISKDTSKNQVSLKLSSVTAADTAVYYCARKDAVVATDALD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK
SEQ ID NO: 60
Heavy chain sequence of hu67-4/hu67-8/hu67-12/hu67-16: 
EVQLQESGPGLVKPSQTLSLTCTVSGFSLTNYGVNWIRQPPRKGLEWIGVIWSR
GNTDYNAAFISRVTISKDNSKSQVSLKLSSVTAADTAVYYCARKDAVVATDALD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK
SEQ ID NO: 138
Light chain sequence of hu67-1/hu67-2/hu67-3/hu67-4: 
DIVMTQSPKSLAVSLGERATINCKASQDVSTAVAWYQQKPGQPPKLLIYSTSYRY
TGVPDRFSGSGSGTDFTLTISSLQAEDVAVYYCQQHYSTPYTFGGGTKVEIKRTV
AAPSVFIFPPSDEQLKSGTASVVCLLNNFYPREAKVQWKVDNALQSGNSQESVT
 
EQDSKDSTYSLSSTLTLSKADYEKHKVYACEVTHQGLSSPVTKSFNRGEC
SEQ ID NO: 139
Light chain sequence of hu67-5/hu67-6/hu67-7/hu67-8: 
DIVMTQSPKSLAVSLGERATINCKASQDVSTAVAWYQQKPGQSPKLLIYSTSYRY
TGVPDRFSGSGSGTDFTFTISSLQAEDVAVYYCQQHYSTPYTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 140
Light chain sequence of hu67-9/hu67-10/hu67-11/hu67-12: 
DIQMTQSPSSLSASVGDRVTITCKASQDVSTAVAWYQQKPGKAPKLLIYSTSYRY
TGVPSRFSGSGSGTDFTFTISSLQPEDIATYYCQQHYSTPYTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 61
Light chain sequence of hu67-13/hu67-14/hu67-15/hu67-16:

DIQMTQSHKFLSASVGDRVTITCKASQDVSTAVAWYQQKPGKSPKLLIYSTSYR
YTGVPSRFSGSGSGTDFTFTISSLQPEDIATYYCQQHYSTPYTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 141

### Example 7: Affinity Maturation of hu67-11

The human anti-TF monoclonal antibody hu67-11 obtained in Example 6 was used to construct antibody yeast libraries using hTF-hFc (SEQ ID NO: 6) and cTF-hFc (SEQ ID NO: 7) as antigens for affinity maturation to improve the cell binding ability of the target antibody. By cloning, expressing, purifying, and detecting the above antibody, the optimal humanized antibodies were selected.

### 7.1 Construction of affinity maturation yeast libraries and library screening

To obtain better anti-TF hu67-11-related antibodies, hu67-11 was affinity-matured by yeast display platform technology. Based on hu67-11-scFv (SEQ ID NO: 171), affinity maturation yeast libraries for human TF and monkey TF binding CDRs were designed and prepared and were screened for new mutants.

Construction of yeast libraries: Degenerate primers were designed. The designed mutant amino acids were introduced into hu67-11-scFv mutant libraries by PCR, with the size of each library being about 10⁹. The diversity of the constructed yeast libraries was verified by second-generation sequencing.

In the first round of screening, yeast from the hu67-11-scFv mutant libraries was incubated with biotinylated hTF-hFc protein in 50 mL of phosphate-buffered saline containing 0.1% bovine serum albumin (BSA) (referred to as PBSA) at room temperature for 1 h. Then, the mixture was washed three times with PBSA to remove unbound antibody fragments. Streptavidin microbeads (Milenvi Biotec, Auburn, CA) were then added to the mutant libraries bound by biotinylated hTF-hFc protein and loaded onto the AutoMACS system for sorting. An antibody library with a high affinity for human TF was collected and subjected to amplification culture. The culture was then subjected to induced expression. The resulting enriched library was subjected to a second round of screening for binding to biotinylated cTF-hFc (SEQ ID NO: 7).

For the third and fourth rounds of screening, the library cells from the previous round were incubated with biotinylated hTF-hFc and Mouse Anti-cMyc (9E10, sigma) antibody in PBSA at room temperature for 1 h, and the mixture was washed three times with BSA to remove unbound antibody fragments. Goat anti-mouse-Alexa488 (Life technologies, A-11001) and Strepavidin-PE (Life technologies, S-866) were added. After 1 h of incubation at 4 °C, the mixture was washed three times with PBSA to remove unbound antibody fragments. Finally, antibodies with high affinity were selected by FACS screening (BD FACSAriaTM FUSION).

The mutant libraries were subjected to 2-3 rounds of MACS screening (streptomycin magnetic beads, Invitrogen) and 2-3 rounds of FACS screening (BD FACSAriaTM FUSION) using biotinylated TF-hFc antigen. About 600 yeast single clones were selected for culture and induced expression. The binding of the yeast single clones to the above hTF-hFc (SEQ ID NO: 6) and cTF-hFc (SEQ ID NO: 7) was assessed using FACS (BD FACSCanto II). The yeast single clones with better affinities than the wild type (hu67-11-scfv) were selected for sequencing verification. The sequenced clones were subjected to alignment analysis. After removal of redundant sequences, non-redundant sequences were converted into full-length human IgGs, followed by expression in mammalian cells. Sequence of hu67-11-scFv:

EVQLQESGPGLVKPSQTLSLTCTVSGFSLTNYGVNWIRQPPGKGLEWIGVIWSR
GNTDYNAAFISRVTISKDTSKNQVSLKLSSVTAADTAVYYCARKDAVVATDALD
YWGQGTTVTVSSGGGGSGGGGSGGGGSDIQMTQSPSSLSASVGDRVTITCKAS
QDVSTAVAWYQQKPGKAPKLLIYSTSYRYTGVPSRFSGSGSGTDFTFTISSLQPE
DIATYYCQQHYSTPYTFGGGTKVEIK
SEQ ID NO: 171

### Example 8: Engineering of hu67-11 Affinity-Matured Molecule

The humanized antibody hu67-11 obtained in Example 7 was subjected to CDR point mutation (single point and/or combination), and point mutation P40S was further introduced into the FRs to further improve the cell binding ability. By cloning, expressing, purifying, and detecting the above antibody, variable region sequences of antibodies hu67Y116 and hu67Y118 with further improved cell binding ability were obtained by screening.
Heavy chain variable region sequence of antibody hu67Y116/hu67Y118: 
EVQLQESGPGLVKPSQTLSLTCTVSGFSLTNYGVNWIRQSPGKGLEWIGVIWSR
GNTDYNAAFKSRVTISKDTSKNQVSLKLSSVTAADTAVYYCARKDAIVATDALD
YWGQGTTVTVSS
 SEQ ID NO: 62
Light chain variable region sequence of antibody hu67Y116: 
DIQMTQSPSSLSASVGDRVTITCKASQDVQTAVAWYQQKPGKAPKLLIYSTHYR
YTGVPSRFSGSGSGTDFTFTISSLQPEDIATYYCLQSYTLPFSFGGGTKVEIK
SEQ ID NO: 63
Light chain variable region sequence of antibody hu67Y118:

DIQMTQSPSSLSASVGDRVTITCKASQDVQTAVAWYQQKPGKAPKLLIYSTHYR
YTGVPSRFSGSGSGTDFTFTISSLQPEDIATYYCLQLIGVPHTFGGGTKVEIK
SEQ ID NO: 81

**Table 10. CDRs of antibody hu67Y116**

| hu67Y116 | **CDR sequences** | | **Sequence No.** |
|---|---|---|---|
| HCDR | HCDR1 | NYGVN | SEQ ID NO: 40 |
| | HCDR2 | VIWSRGNTDYNAAFKS | SEQ ID NO: 64 |
| | HCDR3 | KDAIVATDALDY | SEQ ID NO: 65 |
| LCDR | LCDR1 | KASQDVQTAVA | SEQ ID NO: 66 |
| | LCDR2 | STHYRYT | SEQ ID NO: 67 |
| | LCDR3 | LQSYTLPFS | SEQ ID NO: 68 |

**Table 11. CDRs of antibody hu67Y118**

| hu67Y118 | **CDR sequences** | | **Sequence No.** |
|---|---|---|---|
| HCDR | HCDR1 | NYGVN | SEQ ID NO: 40 |
| | HCDR2 | VIWSRGNTDYNAAFKS | SEQ ID NO: 64 |
| | HCDR3 | KDAIVATDALDY | SEQ ID NO: 65 |
| LCDR | LCDR1 | KASQDVQTAVA | SEQ ID NO: 66 |
| | LCDR2 | STHYRYT | SEQ ID NO: 67 |
| | LCDR3 | LQLIGVPHT | SEQ ID NO: 83 |

Heavy chain sequence of hu67Y116/hu67Y118: 
EVQLQESGPGLVKPSQTLSLTCTVSGFSLTNYGVNWIRQSPGKGLEWIGVIWSR
GNTDYNAAFKSRVTISKDTSKNQVSLKLSSVTAADTAVYYCARKDAIVATDALD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK
SEQ ID NO: 69
Light chain sequence of hu67Y116: DIQMTQSPSSLSASVGDRVTITCKASQDVQTAVAWYQQKPGKAPKLLIYSTHYR 
YTGVPSRFSGSGSGTDFTFTISSLQPEDIATYYCLQSYTLPFSFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 70
Light chain sequence of hu67Y118:

DIQMTQSPSSLSASVGDRVTITCKASQDVQTAVAWYQQKPGKAPKLLIYSTHYR
YTGVPSRFSGSGSGTDFTFTISSLQPEDIATYYCLQLIGVPHTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 82

### Example 9: Sequence Engineering of 10H10 Molecule

Antibody 10H10 does not affect blood coagulation, as has been confirmed by crystal structure analysis (Cellular Signalling 36 (2017) 139-144). After homology alignment in an antibody database, a humanized antibody model was established. The back mutation was selected according to the model, and the CDR regions were optimized. The above antibody was cloned, expressed, purified, and detected.

For the human germline light chain template of antibody 10H10, the FR1, FR2, and FR3 of IGKV1-39*01 and the FR4 of IGKJ2*01 were selected. For the human germline heavy chain template, the FR1, FR2, and FR3 of IGHV1-46*01 or IGHV7-4-1*02 and the FR4 of IGHJ1*01 were selected. Optionally, the amino acid residue(s) at position(s) 24, 25, 27b, 27d, 27f, 56, and/or 97 in the light chain variable region of the humanized antibody were/was substituted; and/or the amino acid residue(s) at position(s) 1, 26, 27, 30, 31, 38, 40, 53, 54, 56, 57, 67, 68, 69, 71, 81, and/or 101 in the heavy chain variable region of the humanized antibody were/was substituted.

**Table 12. Humanization design for antibody 10H10**

| VL | | VH | |
|---|---|---|---|
| VL1 | Graft(IGKV1-39*01) | VH1 | Graft(IGHV1-46*01)+Q1E, T30I, A40R, M69F, R71A, E81Q |
| VL2 | Graft(IGKV1-39*01) + K24R, S25A, L27b I | VH2 | Graft(IGHV7-4-1 *02)+Q1E, T30I, A40R, F67A, L71A |
| VL3 | Graft(IGKV1-39*01) + S56E | VH3 | Graft(IGHV1-46*01)+Q1E, G26I, T30I, A40R, M69F, R71A, E81Q |
| VL4 | Graft(IGKV1-39*01) + T97S | VH4 | Graft(IGHV7-4-1 *02)+Q1E, Y27F, T30I, A40R, F67A, L71A |
| VL5 | Graft(IGKV1-39*01) + S27d D, S56E | VHS | Graft(IGHV1-46*01)+Q1E, T30I, A40R, M69F, R71A, E81Q+T57S |
| VL6 | Graft(IGKV1-39*01) + G27f S, S56E | VH6 | Graft(IGHV7-4-1*02)+Q1E, T30I, A40R, F67A, L71A+A101V |
| VL9 | Graft(IGKV1-39*01) + K24Q | VH7 | Graft(IGHV7-4-1*02)+Q1E, T30I, R38K, A40R, F67A, V68T, L71A |
| | | VH8 | Graft(IGHV7-4-1*02)+Q1E, Y27R, T30I, A40R, F67A, V68T, L71A |
| | | VH9 | Graft(IGHV7-4-1*02)+Q1E, T30R, R38K, A40R, F67A, V68T, L71A |
| | | VH10 | Graft(IGHV7-4-1*02)+Q1E, T30I, R38K, A40R, F67A, V68T, L71A+ T31E |
| | | VH11 | Graft(IGHV7-4-1*02)+Q1E, T30I, R38K, A40R, F67A, V68T, L71A+ G53Y |
| | | VH12 | Graft(IGHV7-4-1*02)+Q1E, T30I, R38K, A40R, F67A, V68T, L71A+ S54H |
| | | VH13 | Graft(IGHV7-4-1*02)+Q1E, T30I, R38K, A40R, F67A, V68T, L71A+ S56W |
| | | VH14 | Graft(IGHV7-4-1*02)+Q1E, T30I, A40R, F67A, L71A+S56W |
| FR4 | IGKJ2*01 | FR4 | IGHJ1*01 |

Note: The amino acid positions in the table are numbered according to the Kabat numbering scheme. L27bI indicates that the L at position 27b is mutated to I according to the Kabat numbering scheme. The same applies hereinafter.

The sequences of the hu10H10 humanized antibody variable regions obtained are shown below:
hu10H10 VH1(SEQ ID NO: 84) 
EVQLVQSGAEVKKPGASVKVSCKASGYTFITYWIEWVRQRPGQGLEWMGDIL
PGSGSTNYNENFKGRVTFTADTSTSTVYMQLSSLRSEDTAVYYCARSGYYGNS
GFAYWGQGTLVTVSS
hu10H10 VH2(SEQ ID NO: 85) 
EVQLVQSGSELKKPGASVKVSCKASGYTFITYWIEWVRQRPGQGLEWMGDILP
GSGSTNYNENFKGRAVFSADTSVSTAYLQISSLKAEDTAVYYCARSGYYGNSGF
AYWGQGTLVTVSS
hu10H10 VH3(SEQ ID NO: 86) 
EVQLVQSGAEVKKPGASVKVSCKASIYTFITYWIEWVRQRPGQGLEWMGDILP
GSGSTNYNENFKGRVTFTADTSTSTVYMQLSSLRSEDTAVYYCARSGYYGNSG
FAYWGQGTLVTVSS
hu10H10 VH4(SEQ ID NO: 87) 
EVQLVQSGSELKKPGASVKVSCKASGFTFITYWIEWVRQRPGQGLEWMGDILP
GSGSTNYNENFKGRAVFSADTSVSTAYLQISSLKAEDTAVYYCARSGYYGNSGF
AYWGQGTLVTVSS
hu10H10 VH5(SEQ ID NO: 88) EVQLVQSGAEVKKPGASVKVSCKASGYTFITYWIEWVRQRPGQGLEWMGDIL 
PGSGSSNYNENFKGRVTFTADTSTSTVYMQLSSLRSEDTAVYYCARSGYYGNS
GFAYWGQGTLVTVSS
hu10H10 VH6(SEQ ID NO: 89) 
EVQLVQSGSELKKPGASVKVSCKASGYTFITYWIEWVRQRPGQGLEWMGDILP
GSGSTNYNENFKGRAVFSADTSVSTAYLQISSLKAEDTAVYYCARSGYYGNSGF
VYWGQGTLVTVSS
hu10H10 VH7(SEQ ID NO: 90) 
EVQLVQSGSELKKPGASVKVSCKASGYTFITYWIEWVKQRPGQGLEWMGDILP
GSGSTNYNENFKGRATFSADTSVSTAYLQISSLKAEDTAVYYCARSGYYGNSGF
AYWGQGTLVTVSS
hu10H10 VH8(SEQ ID NO: 91) 
EVQLVQSGSELKKPGASVKVSCKASGRTFITYWIEWVRQRPGQGLEWMGDILP
GSGSTNYNENFKGRATFSADTSVSTAYLQISSLKAEDTAVYYCARSGYYGNSGF
AYWGQGTLVTVSS
hu10H10 VH9(SEQ ID NO: 92) 
EVQLVQSGSELKKPGASVKVSCKASGYTFRTYWIEWVKQRPGQGLEWMGDIL
PGSGSTNYNENFKGRATFSADTSVSTAYLQISSLKAEDTAVYYCARSGYYGNSG
FAYWGQGTLVTVSS
hu10H10 VH10(SEQ ID NO: 93) 
EVQLVQSGSELKKPGASVKVSCKASGYTFIEYWIEWVKQRPGQGLEWMGDILP
GSGSTNYNENFKGRATFSADTSVSTAYLQISSLKAEDTAVYYCARSGYYGNSGF
AYWGQGTLVTVSS
hu10H10 VH11(SEQ ID NO: 71) 
EVQLVQSGSELKKPGASVKVSCKASGYTFITYWIEWVKQRPGQGLEWMGDILP
YSGSTNYNENFKGRATFSADTSVSTAYLQISSLKAEDTAVYYCARSGYYGNSGF
AYWGQGTLVTVSS
hu10H10 VH12(SEQ ID NO: 94) 
EVQLVQSGSELKKPGASVKVSCKASGYTFITYWIEWVKQRPGQGLEWMGDILP
GHGSTNYNENFKGRATFSADTSVSTAYLQISSLKAEDTAVYYCARSGYYGNSGF
AYWGQGTLVTVSS
hu10H10 VH13(SEQ ID NO: 95) 
EVQLVQSGSELKKPGASVKVSCKASGYTFITYWIEWVKQRPGQGLEWMGDILP
GSGWTNYNENFKGRATFSADTSVSTAYLQISSLKAEDTAVYYCARSGYYGNSG
FAYWGQGTLVTVSS
hu10H10 VH14(SEQ ID NO: 96) 
EVQLVQSGSELKKPGASVKVSCKASGYTFITYWIEWVRQRPGQGLEWMGDILP
GSGWTNYNENFKGRAVFSADTSVSTAYLQISSLKAEDTAVYYCARSGYYGNSG
FAYWGQGTLVTVSS
hu10H10 VL1(SEQ ID NO: 97) 
DIQMTQSPSSLSASVGDRVTITCKSSQSLLSSGNQKNYLTWYQQKPGKAPKLLIY
WASTRESGVPSRFSGSGSGTDFTLTISSLQPEDFATYYCQNDYTYPLTFGQGTKL
 EIK
hu10H10 VL2(SEQ ID NO: 98) 
DIQMTQSPSSLSASVGDRVTITCRASQSILSSGNQKNYLTWYQQKPGKAPKLLIY
WASTRESGVPSRFSGSGSGTDFTLTISSLQPEDFATYYCQNDYTYPLTFGQGTKL
EIK
hu10H10 VL3(SEQ ID NO: 99) 
DIQMTQSPSSLSASVGDRVTITCKSSQSLLSSGNQKNYLTWYQQKPGKAPKLLIY
WASTREEGVPSRFSGSGSGTDFTLTISSLQPEDFATYYCQNDYTYPLTFGQGTKL
EIK
hu10H10 VL4(SEQ ID NO: 100) 
DIQMTQSPSSLSASVGDRVTITCKSSQSLLSSGNQKNYLTWYQQKPGKAPKLLIY
WASTRESGVPSRFSGSGSGTDFTLTISSLQPEDFATYYCQNDYTYPLSFGQGTKL
EIK
hu10H10 VL5(SEQ ID NO: 101) 
DIQMTQSPSSLSASVGDRVTITCKSSQSLLDSGNQKNYLTWYQQKPGKAPKLLI
YWASTREEGVPSRFSGSGSGTDFTLTISSLQPEDFATYYCQNDYTYPLTFGQGTK
LEIK
hu10H10 VL6(SEQ ID NO: 102) 
DIQMTQSPSSLSASVGDRVTITCKSSQSLLSSSNQKNYLTWYQQKPGKAPKLLIY
WASTREEGVPSRFSGSGSGTDFTLTISSLQPEDFATYYCQNDYTYPLTFGQGTKL
EIK
hu10H10 VL9(SEQ ID NO: 72)

DIQMTQSPSSLSASVGDRVTITCQSSQSLLSSGNQKNYLTWYQQKPGKAPKLLI
YWASTRESGVPSRFSGSGSGTDFTLTISSLQPEDFATYYCQNDYTYPLTFGQGTK
LEIK

Note: The CDRs are single-underlined, the CDR amino acid substitution sites are single-underlined and shown in bold, the FR amino acid substitution sites are double-underlined and shown in bold, and the rest are FR regions. The same applies hereinafter.

**Table 13. CDRs of hu10H10 humanized antibodies**

| Variable region | CDR | Sequence | SEQ ID NO |
|---|---|---|---|
| hu10H10VH5 | HCDR2 | DILPGSGS**S**NYNENFKG | 142 |
| hu10H10VH6 | HCDR3 | SGYYGNSGF**V**Y | 143 |
| hu10H10VH10 | HCDR1 | **E**YWIE | 144 |
| hu10H10VH11 | HCDR2 | DILP**Y**SGSTNYNENFKG | 74 |
| hu10H10VH12 | HCDR2 | DILPG**H**GSTNYNENFKG | 145 |
| hu10H10VH13/VH14 | HCDR2 | DILPGSG**W**TNYNENFKG | 146 |
| hu10H10VL2 | LCDR1 | **RA**SQSILSSGNQKNYLT | 147 |
| hu10H10VL3/VL5/VL6 | LCDR2 | WASTRE**E** | 148 |
| hu10H10VL4 | LCDR3 | QNDYTYPL**S** | 149 |
| hu10H10VL5 | LCDR1 | KSSQSLL**D**SGNQKNYLT | 150 |
| hu10H10VL6 | LCDR1 | KSSQSLLSS**S**NQKNYLT | 151 |
| hu10H10VL9 | LCDR1 | **Q**SSQSLLSSGNQKNYLT | 76 |

**Table 14. Engineered humanized antibodies of 10H10**

| | L1 | L2 | L3 | L4 | L5 | L6 | L9 |
|---|---|---|---|---|---|---|---|
| H1 | hu10H10-1 | hu10H10-7 | hu10H10-13 | hu10H10-19 | | | |
| H2 | hu10H10-2 | hu10H10-8 | hu10H10-14 | hu10H10-20 | hu10H10-57 | hu10H10-58 | |
| H3 | hu10H10-3 | hu10H10-9 | hu10H10-15 | hu10H10-21 | | | |
| H4 | hu10H10-4 | hu10H10-10 | hu10H10-16 | hu10H10-22 | | | |
| H5 | hu10H10-5 | hu10H10-11 | hu10H10-17 | hu10H10-23 | | | |
| H6 | hu10H10-6 | hu10H10-12 | hu10H10-18 | hu10H10-24 | | | |
| H7 | hu10H10-25 | | hu10H10-33 | | hu10H10-41 | hu10H10-49 | |
| H8 | hu10H10-26 | | hu10H10-34 | | hu10H10-42 | hu10H10-50 | |
| H9 | hu10H10-27 | | hu10H10-35 | | hu10H10-43 | hu10H10-51 | |
| H10 | hu10H10-28 | | hu10H10-36 | | hu10H10-44 | hu10H10-52 | |
| H11 | hu10H10-29 | | hu10H10-37 | | hu10H10-45 | hu10H10-53 | hu10H10-77 |
| H12 | hu10H10-30 | | hu10H10-38 | | hu10H10-46 | hu10H10-54 | |
| H13 | hu10H10-31 | | bu10H10-39 | | hu10H10-47 | hu10H10-55 | |
| H14 | hul0H10-32 | | bu10H10-40 | | hu10H10-48 | hu10H10-56 | |

**Table 15. CDR sequences of humanized antibody hu10H10-77**

| **CDR** | **Sequence** | **Sequence No.** |
|---|---|---|
| HCDR1 | TYWIE | SEQ ID NO: 73 |
| HCDR2 | DILPYSGSTNYNENFKG | SEQ ID NO: 74 |
| HCDR3 | SGYYGNSGFAY | SEQ ID NO: 75 |
| LCDR1 | QSSQSLLSSGNQKNYLT | SEQ ID NO: 76 |
| LCDR2 | WASTRES | SEQ ID NO: 77 |
| LCDR3 | QNDYTYPLT | SEQ ID NO: 78 |

Note: CDRs are determined according to the Kabat numbering scheme.

The heavy chain sequence and the light chain sequence of the 10H10 humanized antibodies are shown below:
Heavy chain of hu10H10-1/hu10H10-7/hu10H10-13/hu10H10-19: 
EVQLVQSGAEVKKPGASVKVSCKASGYTFITYWIEWVRQRPGQGLEWMGDIL
PGSGSTNYNENFKGRVTFTADTSTSTVYMQLSSLRSEDTAVYYCARSGYYGNS
GFAYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 152
Heavy chain of hu10H10-2/hul0H10-8/hu10H10-14/hu10Hl0-20/hul0Hl0-57/hu10H10-58: 
EVQLVQSGSELKKPGASVKVSCKASGYTFITYWIEWVRQRPGQGLEWMGDILP
GSGSTNYNENFKGRAVFSADTSVSTAYLQISSLKAEDTAVYYCARSGYYGNSGF
AYWGQGTLVTVSSASTKGPSVFPLAPSSKSTSGGTAALGCLVKDYFPEPVTVSW
 
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 153
Heavy chain of hu10H10-3/hu10H10-9/hu10H10-15/hu10H10-21: 
EVQLVQSGAEVKKPGASVKVSCKASIYTFITYWIEWVRQRPGQGLEWMGDILP
GSGSTNYNENFKGRVTFTADTSTSTVYMQLSSLRSEDTAVYYCARSGYYGNSG
FAYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 154
Heavy chain of hu10H10-4/hu10H10-10/hu10H10-16/hu10H10-22: 
EVQLVQSGSELKKPGASVKVSCKASGFTFITYWIEWVRQRPGQGLEWMGDILP
GSGSTNYNENFKGRAVFSADTSVSTAYLQISSLKAEDTAVYYCARSGYYGNSGF
AYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 155
Heavy chain of hulOH10-5/hulOH10-11/hulOH10-17/hulOH10-23: 
EVQLVQSGAEVKKPGASVKVSCKASGYTFITYWIEWVRQRPGQGLEWMGDIL
PGSGSSNYNENFKGRVTFTADTSTSTVYMQLSSLRSEDTAVYYCARSGYYGNS
GFAYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 156
Heavy chain of hu10H10-6/hu10H10-12/hu10H10-18/hu10H10-24: 
EVQLVQSGSELKKPGASVKVSCKASGYTFITYWIEWVRQRPGQGLEWMGDILP
GSGSTNYNENFKGRAVFSADTSVSTAYLQISSLKAEDTAVYYCARSGYYGNSGF
VYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 157
Heavy chain of hu10H10-25/hu10H10-33/hu10H10-41/hu10H10-49: 
EVQLVQSGSELKKPGASVKVSCKASGYTFITYWIEWVKQRPGQGLEWMGDILP
GSGSTNYNENFKGRATFSADTSVSTAYLQISSLKAEDTAVYYCARSGYYGNSGF
AYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 158
Heavy chain of hu10H10-26/hu10H10-34/hu10H10-42/hu10H10-50: 
EVQLVQSGSELKKPGASVKVSCKASGRTFITYWIEWVRQRPGQGLEWMGDILP
GSGSTNYNENFKGRATFSADTSVSTAYLQISSLKAEDTAVYYCARSGYYGNSGF
AYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 159
Heavy chain of hu10H10-27/hu10H10-35/hu10H10-43/hu10H10-51: 
EVQLVQSGSELKKPGASVKVSCKASGYTFRTYWIEWVKQRPGQGLEWMGDIL
PGSGSTNYNENFKGRATFSADTSVSTAYLQISSLKAEDTAVYYCARSGYYGNSG
FAYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
 
MHEALHNHYTQKSLSLSPGK
SEQ ID NO: 160
Heavy chain of hu10H10-28/hu10H10-36/hu10H10-44/hu10H10-52: 
EVQLVQSGSELKKPGASVKVSCKASGYTFIEYWIEWVKQRPGQGLEWMGDILP
GSGSTNYNENFKGRATFSADTSVSTAYLQISSLKAEDTAVYYCARSGYYGNSGF
AYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 161
Heavy chain of hu10H10-29/hu10H10-37/hu10H10-45//hu10H10-53/hu10H10-77: 
EVQLVQSGSELKKPGASVKVSCKASGYTFITYWIEWVKQRPGQGLEWMGDILP
YSGSTNYNENFKGRATFSADTSVSTAYLQISSLKAEDTAVYYCARSGYYGNSGF
AYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 79
Heavy chain of hu10H10-30/hu10H10-38/hu10H10-46/hu10H10-54: 
EVQLVQSGSELKKPGASVKVSCKASGYTFITYWIEWVKQRPGQGLEWMGDILP
GHGSTNYNENFKGRATFSADTSVSTAYLQISSLKAEDTAVYYCARSGYYGNSGF
AYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 162
Heavy chain of hu10H10-31/hu10H10-39/hu10H10-47/hu10H10-55: 
EVQLVQSGSELKKPGASVKVSCKASGYTFITYWIEWVKQRPGQGLEWMGDILP
GSGWTNYNENFKGRATFSADTSVSTAYLQISSLKAEDTAVYYCARSGYYGNSG
FAYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
 
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 163
Heavy chain of hu10H10-32/hu10H10-40/hu10H10-48/hu10H10-56: 
EVQLVQSGSELKKPGASVKVSCKASGYTFITYWIEWVRQRPGQGLEWMGDILP
GSGWTNYNENFKGRAVFSADTSVSTAYLQISSLKAEDTAVYYCARSGYYGNSG
FAYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 164
Light chain of hu10H10-1/hu10H10-2/hu10H10-3/hu10H10-4/hu10H10-5/hu10H10-6/hu10H10-25/hu10H10-26/hu10H10-27/hu10H10-28/hu10H10-29/hu10H10-30/hu10H10-31/hu10H10-32: 
DIQMTQSPSSLSASVGDRVTITCKSSQSLLSSGNQKNYLTWYQQKPGKAPKLLIY
WASTRESGVPSRFSGSGSGTDFTLTISSLQPEDFATYYCQNDYTYPLTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC
SEQ ID NO: 165
Light chain of hu10H10-7/hu10H10-8/hu10H10-9/hu10H10-10/hu10H10-11/hu10H10-12: 
DIQMTQSPSSLSASVGDRVTITCRASQSILSSGNQKNYLTWYQQKPGKAPKLLIY
WASTRESGVPSRFSGSGSGTDFTLTISSLQPEDFATYYCQNDYTYPLTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC
SEQ ID NO: 166
Light chain of hu10H10-13/hu10H10-14/hu10H10-15/hu10H10-16/hu10H10-17/hu10H10-18/hul0Hl0-33/hu10H10-34/hu10H10-35/hu10H10-36/hu10Hl0-37/hu10H10-38/hu10H10-39/hu10H10-40: 
DIQMTQSPSSLSASVGDRVTITCKSSQSLLSSGNQKNYLTWYQQKPGKAPKLLIY
WASTREEGVPSRFSGSGSGTDFTLTISSLQPEDFATYYCQNDYTYPLTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC
 SEQ ID NO: 167
SEQ ID NO: 167 Light chain of hu10H10-19/hu10H10-20/hu10H10-21/hu10H10-22/hu10H10-23/hu10H10-24: 
DIQMTQSPSSLSASVGDRVTITCKSSQSLLSSGNQKNYLTWYQQKPGKAPKLLIY
WASTRESGVPSRFSGSGSGTDFTLTISSLQPEDFATYYCQNDYTYPLSFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC
SEQ ID NO: 168
Light chain of hu10H10-41/hu10H10-42/hu10H10-43/hu10H10-44/hu10H10-45/hu10H10-46/hu10H10-47/hu10H10-48/hu10H10-57: 
DIQMTQSPSSLSASVGDRVTITCKSSQSLLDSGNQKNYLTWYQQKPGKAPKLLI
YWASTREEGVPSRFSGSGSGTDFTLTISSLQPEDFATYYCQNDYTYPLTFGQGTK
LEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC
SEQ ID NO: 169
Light chain of hu10H10-49/hu10H10-50/hu10H10-51/hu10H10-52/hu10H10-53/hu10H10-54/hu10H10-55/hu10H10-56/hu10H10-58: 
DIQMTQSPSSLSASVGDRVTITCKSSQSLLSSSNQKNYLTWYQQKPGKAPKLLIY
WASTREEGVPSRFSGSGSGTDFTLTISSLQPEDFATYYCQNDYTYPLTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC
SEQ ID NO: 170
Light chain of hu10H10-77:

DIQMTQSPSSLSASVGDRVTITCQSSQSLLSSGNQKNYLTWYQQKPGKAPKLLIY
WASTRESGVPSRFSGSGSGTDFTLTISSLQPEDFATYYCQNDYTYPLTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC
SEQ ID NO: 80

### Example 10: Preparation of ADCs

### Determination of DAR values of ADCs

The DAR values of the ADCs of the present disclosure were calculated by RP-HPLC (reversed-phase high performance liquid chromatography).

The drug moiety of the conjugates of the present disclosure may be any suitable drug. Particularly suitable drugs are described in, for example, WO2020063676A1 (which is incorporated herein by reference in its entirety). The compound 9A of the present disclosure (i.e., the compound 9-A of Example 9 in WO2020063676A1) has the following structure:

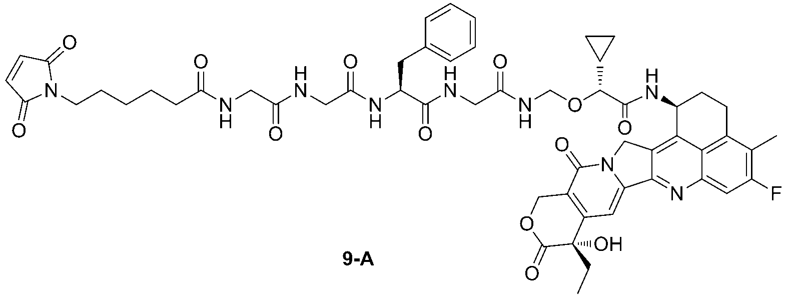

The present disclosure uses the following methods and adjusts reaction parameters to prepare antibody-drug conjugates represented by the ADC general formula (TF-9A).

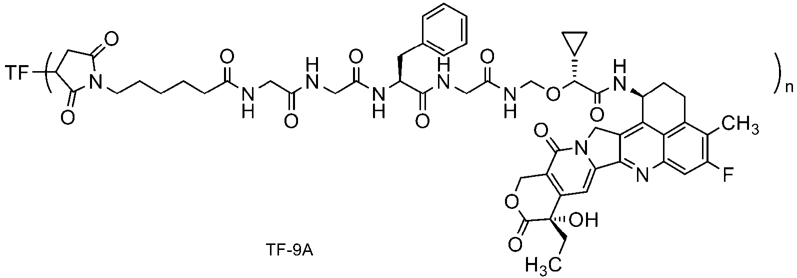

### Example 10-1. ADC-1

A prepared aqueous solution of tris(2-carboxyethyl)phosphine hydrochloride (TCEP.HCl) (10 mM, 131.8 µL, 1.318 µmol) was added to antibody hu67Y116 in an aqueous PBS buffer solution (pH = 6.5, 0.05 M) (10.0 mg/mL, 7.4 mL, 511 nmol) at 37 °C. The mixture was shaken on a thermostatic shaker at 37 °C for 3 h, and the reaction was stopped. The reaction mixture was cooled to 25 °C in a water bath.

Compound 9A (5.49 mg, 5.11 µmol) was dissolved in 370 mL of DMSO, and the resulting solution was added dropwise to the above reaction mixture. The mixture was shaken on a thermostatic shaker at 25 °C for 3 h, and the reaction was stopped. The reaction mixture was subjected to desalting purification using a Sephadex G25 gel column (elution phase: an aqueous His buffer solution with a pH of 5.0) to give an exemplary product of conjugate TF-9A, **ADC-1** (hu67Y116-9A), in a His buffer (3.8 mg/mL, 17.6 mL), which was then stored at 4 °C. RP-HPLC calculated average: DAR = 4.14.

### Example 10-2. ADC-2

A prepared aqueous solution of tris(2-carboxyethyl)phosphine hydrochloride (TCEP.HCl) (10 mM, 178.9 µL, 1.789 µmol) was added to antibody hu67Y116 in an aqueous PBS buffer solution (pH = 6.5, 0.05 M) (10.0 mg/mL, 7.4 mL, 511 nmol) at 37 °C. The mixture was shaken on a water bath shaker at 37 °C for 3 h, and the reaction was stopped. The reaction mixture was cooled to 25 °C in a water bath.

Compound **9A** (6.586 mg, 6.132 µmol) was dissolved in 370 µL of DMSO, and the resulting solution was added to the above reaction mixture. The mixture was shaken on a water bath shaker at 25 °C for 3 h, and the reaction was stopped. The reaction mixture was subjected to desalting purification using a Sephadex G25 gel column (elution phase: an aqueous His buffer solution with a pH of 5.0) to give an exemplary product of conjugate TF-9A, **ADC-2** (hu67Y116-9A), in PBS buffer (3.52 mg/mL, 17.9 mL), which was then stored at 4 °C. RP-HPLC calculated average: DAR = 5.91.

### Example 10-3. ADC-3

A prepared aqueous solution of tris(2-carboxyethyl)phosphine hydrochloride (TCEP.HCI) (10 mM, 48.3 µL, 483 nmol) was added to antibody hu67Y116 in an aqueous PBS buffer solution (pH = 6.5, 0.05 M) (10.0 mg/mL, 1 mL, 69 nmol) at 37 °C. The mixture was shaken on a water bath shaker at 37 °C for 3 h, and the reaction was stopped. The reaction mixture was cooled to 25 °C in a water bath.

Compound **9A** (1.1 mg, 1.035 µmol) was dissolved in 50 µL of DMSO, and the resulting solution was added to the above reaction mixture. The mixture was shaken on a water bath shaker at 25 °C for 3 h, and the reaction was stopped. The reaction mixture was subjected to desalting purification using a Sephadex G25 gel column (elution phase: an aqueous His buffer solution with a pH of 5.0) to give an exemplary product of conjugate TF-9A, **ADC-3** (hu67Y116-9A), in PBS buffer (0.8 mg/mL, 11.5 mL), which was then stored at 4 °C. RP-HPLC calculated average: DAR = 7.23.

### Example 10-4. ADC-4

A prepared aqueous solution of tris(2-carboxyethyl)phosphine hydrochloride (TCEP.HCI) (10 mM, 145.5 µL, 1.455 µmol) was added to antibody hu10H10-77 in an aqueous PBS buffer solution (pH = 6.5, 0.05 M) (10.0 mg/mL, 8 mL, 549 nmol) at 37 °C. The mixture was shaken on a water bath shaker at 37 °C for 3 h, and the reaction was stopped. The reaction mixture was cooled to 25 °C in a water bath.

Compound **9A** (5.9 mg, 5.49 µmol) was dissolved in 400 µL of DMSO, and the resulting solution was added to the above reaction mixture. The mixture was shaken on a water bath shaker at 25 °C for 3 h, and the reaction was stopped. The reaction mixture was subjected to desalting purification using a Sephadex G25 gel column (elution phase: an aqueous His buffer solution with a pH of 5.0) to give an exemplary product of conjugate TF-9A, **ADC-4** (hu10H10-77-9A), in PBS buffer (3.85 mg/mL, 19 mL), which was then stored at 4 °C. RP-HPLC calculated average: DAR = 4.2.

### Example 10-5. ADC-5

A prepared aqueous solution of tris(2-carboxyethyl)phosphine hydrochloride (TCEP.HCI) (10 mM, 175.7 µL, 1.757 µmol) was added to antibody hu10H10-77 in an aqueous PBS buffer solution (pH = 6.5, 0.05 M) (10.0 mg/mL, 8 mL, 549 nmol) at 37 °C.

The mixture was shaken on a water bath shaker at 37 °C for 3 h, and the reaction was stopped. The reaction mixture was cooled to 25 °C in a water bath.

Compound **9A** (7.08 mg, 6.59 µmol) was dissolved in 400 µL of DMSO, and the resulting solution was added to the above reaction mixture. The mixture was shaken on a water bath shaker at 25 °C for 3 h, and the reaction was stopped. The reaction mixture was subjected to desalting purification using a Sephadex G25 gel column (elution phase: an aqueous His buffer solution with a pH of 5.0) to give an exemplary product of conjugate TF-9A, ADC-5 (hu10H10-77-9A), in PBS buffer (3.57 mg/mL, 19.5 mL), which was then stored at 4 °C. RP-HPLC calculated average: DAR = 5.87.

### Example 10-6. ADC-6

A prepared aqueous solution of tris(2-carboxyethyl)phosphine hydrochloride (TCEP.HCl) (10 mM, 124.4 µL, 1.244 µmol) was added to antibody hu310-2 in an aqueous PBS buffer solution (pH = 6.5, 0.05 M) (10.0 mg/mL, 8 mL, 553 nmol) at 37 °C. The mixture was shaken on a water bath shaker at 37 °C for 3 h, and the reaction was stopped. The reaction mixture was cooled to 25 °C in a water bath.

Compound **9A** (5.94 mg, 5.53 µmol) was dissolved in 400 µL of DMSO, and the resulting solution was added to the above reaction mixture. The mixture was shaken on a water bath shaker at 25 °C for 3 h, and the reaction was stopped. The reaction mixture was subjected to desalting purification using a Sephadex G25 gel column (elution phase: an aqueous His buffer solution with a pH of 5.0) to give an exemplary product of conjugate TF-9A, ADC-6 (hu310-2-9A), in PBS buffer (3.77 mg/mL, 19.2 mL), which was then stored at 4 °C. RP-HPLC calculated average: DAR = 4.05.

### Example 10-7. ADC-7

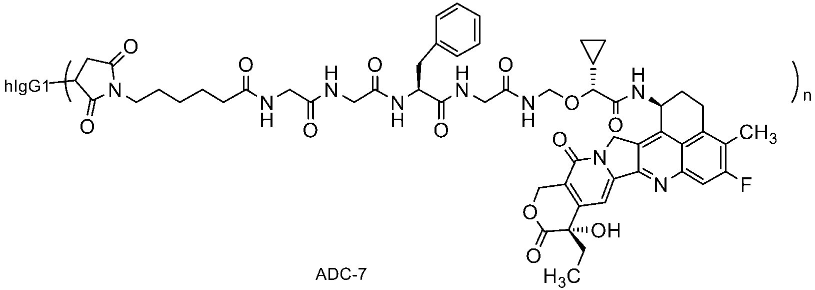

A prepared aqueous solution of tris(2-carboxyethyl)phosphine hydrochloride (TCEP.HCl) (10 mM, 16.6 µL, 166 nmol) was added to antibody hIgG1 in an aqueous PBS buffer solution (a 0.05 M aqueous PBS buffer solution (pH 6.5); 10.0 mg/mL, 1 mL, 67.6 nmol) at 37 °C. The mixture was shaken on a water bath shaker at 37 °C for 3 h, and the reaction was stopped. The reaction mixture was cooled to 25 °C in a water bath. Compound 9A (0.73 mg, 676 nmol) was dissolved in 60 µL of dimethyl sulfoxide, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 0.05 M aqueous PBS buffer solution with a pH of 6.5, containing 0.001 M EDTA) to give the title product **ADC-7** in PBS buffer (0.91 mg/mL, 9.8 mL), which was then stored at 4 °C.

RP-HPLC calculated average: n = 4.73.

### Test Examples:

### Test Example 1: Binding Assay of Antibodies to Free Human and Monkey TF Proteins

AffiniPure Goat Anti-Human IgG (Jackon, 109-005-008) protein was diluted to 2 µg/mL in a PBS buffer with a pH of 7.4 (Shanghai BasalMedia Technologies Co., Ltd., B320KJ) and added to a 96-well microplate at 100 µL/well, and the plate was incubated overnight at 4 °C. After the liquid was discarded, 5% skim milk (BD, 232100) diluted in PBS was added at 300 µL/well for blocking, and the plate was incubated at 37 °C for 2 h. After the blocking, the blocking solution was discarded, and after the plate was washed 3 times with a PBST buffer (pH 7.4 PBS containing 0.1% tween-20), a 2 µg/mL diluted test antibody solution was added at 50 µL/well, and the plate was incubated at 37 °C for 1 h. After the incubation, the plate was washed 3 times with PBST, and a serially diluted (10 µg/mL, 5-fold serially diluted) biotinylated human or monkey TF-his protein (SEQ ID No. 5/8) (biotin labeling kit: DO JINDO, LK03-10) was added at 50 µL/well. The plate was then incubated at 37 °C for 1 h. After the incubation, the plate was washed 3 times with PBST, and Streptavidin-Peroxidase Polymer (sigma, S2438-250UG), diluted in a 1:8000 ratio, was added. The plate was then incubated at 37 °C for 1 h. After the plate was washed 3 times with PBST, TMB chromogenic substrate (KPL, 5120-0077) was added at 50 µL/well, and the plate was incubated at room temperature for 5 min. Then, 50 µL of 1 M H₂SO₄ was added to each well to stop the reaction. The absorbance at 450 nm was measured using a microplate reader. Binding curves of the antibodies to the antigens were fitted using GraphPad Prism, and the EC₅₀ values were calculated.

**Table 16. Binding of antibodies to free TF**

| Antibody | bio-hTF-his | | bio-cTF-his | |
|---|---|---|---|---|
| | Emax | EC₅₀ (µg/mL) | Emax | EC₅₀ (µg/mL) |
| hu10H10-77 | 1.00 | 0.0035 | 1.13 | 0.0027 |
| hu310-2 | 0.92 | 0.0529 | 0.92 | 0.0410 |
| hu67Y116 | 0.89 | 0.1035 | 1.17 | 0.0691 |
| hIgG1 | 0.10 | No binding | 0.05 | No binding |

The antibody of the present disclosure exhibits relatively good binding activity for TF antigens.

### Test Example 2: Binding Assay of Antibodies to Cells

A 1 × 10⁶ cells/mL suspension of MDA-MB-231 cells, NCI-H358 cells (H358 cells for short), or HCT116 cells was prepared in FACS buffer (1% BSA, pH 7.4 PBS) and added to a 96-well round-bottom plate at 100 µL/well. After centrifugation and removal of the supernatant, the test antibodies, diluted in FACS buffer to different concentrations (60 µg/mL, 3-fold serially diluted), were added at 100 µL/well, and the plate was incubated at 4 °C for 1 h in a dark place. After 3 centrifugal washes at 300 g with FACS buffer, APC Rat Anti-human IgG Fc (BioLegend, 410712), at a working concentration, was added, and the plate was incubated at 4 °C for 40 min in a dark place. After 3 centrifugal washes at 300 g with FACS buffer, the geometric mean fluorescence intensity was measured using a Thermo Attune Nxt flow cytometer, and the EC ₅₀ values were calculated.

**Table 17. Binding of antibodies to cells**

| Antibody | MDA-MB-231 | H358 | HCT116 |
|---|---|---|---|
| | EC₅₀ (nM) | EC₅₀ (nM) | EC₅₀ (nM) |
| Assay 1 | | | |
| hu10H10-1 | 2.05 | No detection | No detection |

| Assay 2 | | | |
|---|---|---|---|
| hu10H10-19 | 2.35 | No detection | No detection |

| Assay 3 | | | |
|---|---|---|---|
| hu10H10-33 | 1.09 | 0.96 | No detection |
| hu10H10-34 | 1.12 | 0.99 | No detection |
| hu10H10-35 | 1.09 | 0.79 | No detection |
| hu10H10-36 | 2.14 | 0.96 | No detection |
| hu10H10-37 | 1.07 | 0.35 | No detection |
| hu10H10-38 | 1.14 | 0.81 | No detection |
| hu10H10-39 | 1.10 | 0.42 | No detection |
| hu10H10-40 | 1.07 | 0.55 | No detection |
| hu10H10-57 | 1.12 | 1.02 | No detection |
| hu10H10-58 | 1.43 | 1.09 | No detection |

| Assay 4 | | | |
|---|---|---|---|
| hu10H10-29 | 1.09 | 0.45 | No detection |
| hu10H10-53 | 1.09 | 0.41 | No detection |

| Assay 5 | | | |
|---|---|---|---|
| hu10H10-77 | 1.64 | 0.68 | 0.47 |

| Assay 6 | | | |
|---|---|---|---|
| hu310-2 | 1.32 | 0.67 | 0.34 |

| Assay 7 | | | |
|---|---|---|---|
| hu310-3 | 1.83 | No detection | No detection |
| hu310-4 | 2.11 | No detection | No detection |
| hu310-5 | 1.55 | No detection | No detection |
| hu310-6 | 2.56 | No detection | No detection |
| hu310-7 | 2.08 | No detection | No detection |
| hu310-8 | 1.73 | No detection | No detection |

| Assay 8 | | | |
|---|---|---|---|
| hu310-9 | 1.97 | No detection | No detection |
| hu310-10 | 1.33 | No detection | No detection |
| hu310-11 | 1.76 | No detection | No detection |
| hu310-12 | 1.95 | No detection | No detection |
| hu310-13 | 1.39 | No detection | No detection |
| hu310-14 | 1.76 | No detection | No detection |
| hu310-15 | 1.98 | No detection | No detection |
| hu310-16 | 2.26 | No detection | No detection |
| CH310 | 1.84 | No detection | No detection |

| Assay 9 | | | |
|---|---|---|---|
| hu67Y116 | 4.91 | 5.42 | 1.66 |
| hu67Y118 | 7.049 | No detection | No detection |
| hu67-11 | 26.27 | No detection | No detection |

| Assay 10 | | | |
|---|---|---|---|
| CH67 | 16.48 | 55.88 | No detection |
| hu67-4 | 18.02 | 90.53 | No detection |
| hu67-14 | 18.89 | 106.7 | No detection |

The antibodies of the present disclosure exhibit relatively good binding activity for cells expressing the TF antigen.

### Test Example 3: Affinity Binding Assay

4 µg/mL solutions of the antibody samples were prepared in 1× HBS-EP buffer solution (pH 7.4) (Cytiva, BR-1006-69), and the antibodies were affinity-captured by a Protein A biosensor chip (Cytiva, 29127556). Then the antigen molecule human TF-His protein (SEQ ID NO: 5) or monkey TF-His protein (SEQ ID NO: 8), at a certain concentration, was allowed to flow through the surface of the chip, and reaction signals were detected in real time using a Biacore T200 (Cytiva) instrument to obtain association and dissociation curves. After the dissociation in each experimental cycle was complete, the biosensor chip was washed with 10 mM glycine-HCl (pH 1.5) (Cytiva, BR-1003-54) for regeneration. Data fitting was performed using a 1:1 binding model. The results are shown in Table 18.

**Table 18. Antibody affinities**

| Antibody | Human TF | | | Monkey TF | | |
|---|---|---|---|---|---|---|
| | ka (1/Ms) | kd (1/s) | KD (M) | ka (1/Ms) | kd (1/s) | KD (M) |
| hu10H10-29 | 1.37E+05 | 7.45E-05 | 5.45E-10 | 1.53E+05 | 9.83E-05 | 6.42E-10 |
| hu10H10-53 | 1.56E+05 | 7.34E-05 | 4.70E-10 | 1.81E+05 | 6.77E-05 | 3.75E-10 |
| hu10H10-77 | 1.46E+05 | 1.10E-04 | 7.49E-10 | 1.69E+05 | 1.14E-04 | 6.77E-10 |
| CH67 | 2.64E+04 | 9.69E-04 | 3.67E-08 | 2.95E+04 | 4.73E-04 | 1.60E-08 |
| hu67-1 | 4.29E+04 | 5.85E-04 | 1.36E-08 | 2.41E+04 | 3.58E-04 | 1.48E-08 |
| hu67-2 | 4.26E+04 | 6.73E-04 | 1.58E-08 | 3.37E+04 | 4.29E-04 | 1.27E-08 |
| hu67-3 | 4.66E+04 | 6.63E-04 | 1.42E-08 | 3.19E+04 | 4.31E-04 | 1.35E-08 |
| hu67-4 | 4.05E+04 | 7.66E-04 | 1.89E-08 | 3.37E+04 | 5.23E-04 | 1.55E-08 |
| hu67-5 | 3.28E+04 | 5.82E-04 | 1.77E-08 | 2.58E+04 | 3.90E-04 | 1.51E-08 |
| hu67-6 | 3.00E+04 | 6.52E-04 | 2.17E-08 | 2.99E+04 | 4.28E-04 | 1.43E-08 |
| hu67-7 | 4.08E+04 | 5.84E-04 | 1.43E-08 | 3.42E+04 | 3.60E-04 | 1.05E-08 |
| hu67-8 | 3.90E+04 | 6.68E-04 | 1.71E-08 | 3.25E+04 | 4.46E-04 | 1.37E-08 |
| hu67-9 | 3.45E+04 | 5.64E-04 | 1.64E-08 | 3.24E+04 | 3.51E-04 | 1.08E-08 |
| hu67-10 | 3.92E+04 | 5.94E-04 | 1.52E-08 | 4.00E+04 | 3.56E-04 | 8.90E-09 |
| hu67-11 | 4.09E+04 | 6.75E-04 | 1.65E-08 | 2.71E+04 | 2.43E-04 | 8.95E-09 |
| hu67-12 | 3.92E+04 | 7.31E-04 | 1.87E-08 | 3.34E+04 | 4.68E-04 | 1.40E-08 |
| hu67-13 | 3.04E+04 | 5.77E-04 | 1.90E-08 | 3.15E+04 | 3.44E-04 | 1.09E-08 |
| hu67-14 | 3.10E+04 | 6.24E-04 | 2.01E-08 | 3.10E+04 | 3.99E-04 | 1.29E-08 |
| hu67-15 | 3.34E+04 | 6.38E-04 | 1.91E-08 | 3.12E+04 | 4.19E-04 | 1.34E-08 |
| hu67-16 | 3.65E+04 | 7.02E-04 | 1.92E-08 | 3.22E+04 | 4.78E-04 | 1.48E-08 |
| hu67Y116 | 6.67E+04 | 2.63E-03 | 3.94E-08 | 3.50E+04 | 9.47E-04 | 2.71E-08 |
| CH310 | 4.09E+05 | 9.49E-03 | 2.32E-08 | 3.27E+05 | 7.67E-03 | 2.35E-08 |
| hu310-2 | 3.78E+05 | 6.99E-03 | 1.85E-08 | 3.32E+05 | 6.37E-03 | 1.92E-08 |

The antibodies of the present disclosure exhibit relatively high affinities for the TF antigens.

### Test Example 4: Binding Assay of Antibodies to Mouse and Rat TF Proteins

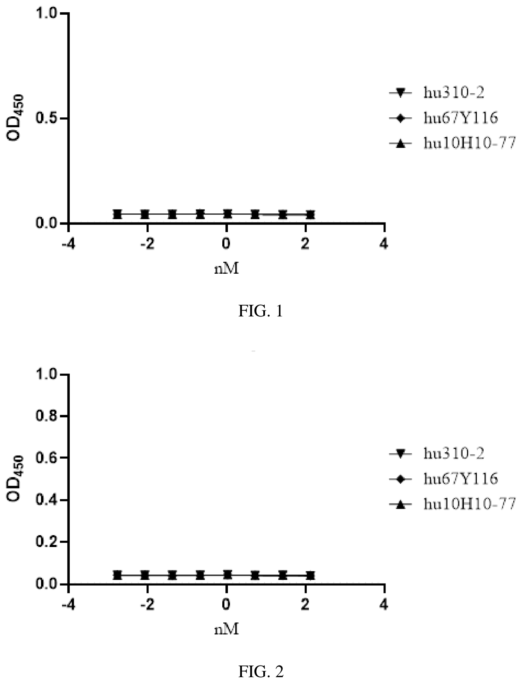

Mouse TF-His protein (SEQ ID NO: 10) or rat TF-His protein (SEQ ID NO: 9) was diluted to 4 µg/mL in a PBS buffer with a pH of 7.4 and added to a 96-well microplate at 100 µL/well, and the plate was incubated overnight at 4 °C. After the supernatant was discarded, 5% skim milk (BD, 232100) diluted in PBS was added at 300 µL/well for blocking, and the plate was incubated at 37 °C for 2 h. After the blocking, the blocking solution was discarded, and after the plate was washed 3 times with a PBST buffer (pH 7.4 PBS containing 0.1% tween-20), a serially diluted antibody solution was added at 100 µL/each well, and the plate was incubated at 37 °C for 1 h. After the incubation, the plate was washed 3 times with PBST, and Peroxidase AffiniPure Goat Anti-Human IgG (H+L) (Jackson, 109-035-003), diluted in a 1:4000 ratio, was added at 100 µL/well. The plate was then incubated at 37 °C for 1 h. After the plate was washed 3 times with PBST, TMB chromogenic substrate (KPL, 5120-0077) was added at 50 µL/well, and the plate was incubated at room temperature for 5 min. Then, 1 M H₂SO₄ was added at 50 µL/well to stop the reaction. The absorbance at 450 nm was measured using a microplate reader. Binding curves of the antibodies to the antigens were fitted using GraphPad Prism (FIG. 1 and FIG. 2).

The results show that none of hu10H10-77, hu310-2, and hu67Y116 binds to mouse TF-His protein (FIG. 1) or rat TF-His protein (FIG. 2).

### Test Example 5: Factor-VII-Human Chromogenic-Activity Blood Coagulation Pathway Assay

In the TF-FVII-FX pathway, TF ultimately affects blood coagulation by affecting FX substrate production. This test was used to assess the effects of different antibodies on the blood coagulation pathway.

The 96-well plate in the Factor-VII-Human Chromogenic-Activity kit (Abcam, ab 108830) was left to stand at room temperature for about 10 min, and 0.5 µg/mL FVIIa (recombinant human coagulation factor VIIa for injection, i.e., NovoSeven) was then added at 100 µL/well. The plate was then incubated at 37 °C for 2 h. After the incubation, the plate was washed 5 times with the wash buffer (provided in the kit). The diluted antibodies were added at 40 µL/well, and the mixture of MDA-MB-231 cells and FX (provided in the kit) was then added at 40 µL/well. The plate was then incubated at 37 °C for half an hour. The substrate (provided in the kit) was added at 20 µL/well to start reactions. The absorbance at 405 nm was measured using a microplate reader over 30 min at intervals of 3 min. The value of the well without antibody addition was taken as the maximum (MAX), and the value of the well without FVIIa addition was taken as the minimum (Min). Data within a linear range were selected to calculate ratios for the effects on the blood coagulation pathway. The blood coagulation ratios are shown in Table 19.

**Table 19. Blood coagulation assay**

| Antibody (20 µg/mL) | Inhibition rate (%) |
|---|---|
| TF011 | 92.8% |
| H39 | 65.5% |
| hu10H10-77 | -1.8% |
| hu310-2 | -2.8% |
| hu67Y116 | 4.0% |
| Negative control hIgG1 | -5.0% |
| Max | 0.0% |
| Min | 100.0% |

The results show that none of the 3 antibodies of the present disclosure affects the blood coagulation pathway, and the positive controls TF011 and H39 affect the blood coagulation pathway.

### Test Example 6: DT3C Endocytosis Assay

After DT3C protein enters cells, activated diphtheria toxin (DT) kills the cells, which indirectly reflects antibody endocytosis. The *in vitro* endocytic activity of an antibody was evaluated based on IC₅₀ and Imax.

DT3C is a recombinantly expressed fusion protein formed by fusion of Fragment A (toxin-only portion) of diphtheria toxin to fragment 3C (IgG binding portion) of group G streptococcus. The protein has a high affinity for the Fc structure of an antibody and enters cells along with the antibody when the antibody is endocytosed. Under the action of intracellular furin, toxic DT is released. DT can inhibit the EF2-ADP ribosylation activity, block the protein translation process, and finally cause cell death. DT3C molecules that have not entered cells have no cell killing activity. The endocytic activity of the antibody was evaluated based on cell killing.

A suspension of MDA-MB-231 cells with a cell density of 4 × 10⁴ cells/mL was prepared in a fresh cell culture medium containing 20% low IgG FBS (Gibco, 1921005PJ) and added to a cell culture plate at 50 µL/well, i.e., 2000 cells/well, and the plate was incubated with 5% carbon dioxide at 37 °C for 16 h.

A DT3C solution with 4× concentration (120 µg/mL) was prepared in serum-free RPMI 1640 culture medium and sterilized by filtration through a 0.22 µm filter. An antibody solution with 4× concentration (40 µg/mL) was prepared in serum-free RPMI 1640 culture medium. 80 µL of DT3C (about 70 KD) and 80 µL of antibody (about 145 KD) were mixed well in a volume ratio of 1:1, and the mixture was left to stand at room temperature for 30 min. Then, 40 µL of the mixture was added to 120 µL of serum-free RPMI 1640 culture medium, and a cell well was set, to which only 160 µL of serum-free RPMI 1640 culture medium was added. The final antibody concentration was 10 µg/mL. The well to which only the culture medium was added was used as a zero point for calculation of the endocytosis ratio. 50 µL of the diluted antibody was added to 50 µL of cells, and the mixture was incubated at 37 °C with 5% CO ₂ for three days. CellTiter-Glo^{™} (CTG) (Promega, G7573) was added at 50 µL/well, and the plate was incubated at room temperature for 10 min in a dark place. The chemiluminescence was measured using ENVISION. The results are shown in Table 20.

**Table 20. Endocytic activities of antibodies**

| Antibody | Final antibody concentration (µg/mL) | Endocytosis ratio (%) |
|---|---|---|
| hu67Y116 | 10 | 69 |
| hu10H10-77 | 10 | 39 |
| hu310-2 | 10 | 70 |

The results show that the antibodies of the present disclosure exhibit endocytic activity.

### Test Example 7: Prothrombin Time (PT) Assay

The effects of antibodies on *in vitro* PT in human plasma were evaluated by determining the PT values of human plasma samples to which anti-TF antibodies were added. Venous blood samples were collected from the upper limb veins of volunteers and poured into 15 mL centrifuge tubes to which 3.8% trisodium citrate anticoagulant (Sinopharm Chemical Reagent Co., Ltd., 6132-04-3) was added in advance (anticoagulant:whole blood = 1:9). The blood samples were centrifuged at room temperature at 2500 rpm for 10 min, and the plasma was collected. The test antibodies were dissolved in PBS (MeilunBio, PWL050) to prepare working solutions with concentrations of 16,000 nM, 8000 nM, 4000 nM, 2000 nM, 1000 nM, and 500 nM (final concentrations of 1600 nM, 800 nM, 400 nM, 200 nM, 100 nM, and 50 nM). A PT reagent (Shanghai Dacheng Medical Equipment Co., Ltd., OUHP29) was added to 4 mL of purified water, and the mixture was incubated at 37 °C for 30 min. According to the operating manual of a CA600 blood coagulation analyzer (sysmex), the human plasma, the PT reagent, the test antibodies, the blank control PBS, and the cleaning agent Clean I (Shanghai Dacheng Medical Equipment Co., Ltd., GSA-500A) were separately placed into their designated positions in the instrument, a program was started, and the PT values were determined. The obtained results were fitted to curves using GraphPad Prism, and numerical values (Table 21) were analyzed and calculated. The effects of the antibodies on PT were determined mainly based on the readings for the maximum concentration of the antibodies.

**Table 21. Results of PT assay**

| Antibody concentration (nM) | TF011 | | H39 | | hIgG | | hu10H10-77 | | hu67Y116 | | hu310-2 | |
|---|---|---|---|---|---|---|---|---|---|---|---|---|
| | Delta PT(s) | Emax | Delta PT(s) | Emax | Delta PT(s) | Emax | Delta PT(s) | Emax | Delta PT(s) | Emax | Delta PT(s) | Emax |
| 1600 | 5.4 | 1.000 | 2.0 | 0.370 | 0.10 | 0.019 | 0.9 | 0.167 | -0.1 | -0.019 | 0.2 | 0.037 |
| 800 | 3.1 | 0.574 | 1.0 | 0.185 | 0.00 | 0.000 | 0.7 | 0.130 | 0.0 | 0.000 | 0.2 | 0.037 |
| 400 | 1.7 | 0.315 | 0.6 | 0.111 | 0.00 | 0.000 | 0.4 | 0.074 | -0.1 | -0.019 | 0.2 | 0.037 |
| 200 | 1.0 | 0.185 | 0.3 | 0.056 | 0.00 | 0.000 | 0.1 | 0.019 | -0.1 | -0.019 | 0.1 | 0.019 |
| 100 | 0.6 | 0.111 | 0.3 | 0.056 | 0.00 | 0.000 | 0.0 | 0.000 | -0.1 | -0.019 | 0.2 | 0.037 |
| 50 | 0.3 | 0.056 | 0.1 | 0.019 | 0.00 | 0.000 | -0.1 | -0.019 | 0.0 | 0.000 | 0.0 | 0.000 |

The results show that none of the 3 antibodies of the present disclosure affects blood coagulation, and their effects on PT are smaller than those of TF011 and H39. TF011 exhibits a relatively significant inhibitory effect on blood coagulation in the PT assay.

### Test Example 8: Binding Assay of Antibodies to Human-Rat Hybrid TF Protein HuRatTF

In order to screen for antibodies that bind to human TF without affecting specific epitopes of the human-related blood coagulation function, the human-rat hybrid antigen HuRatTF protein (SEQ ID No: 11) in Example 1 was constructed and used to perform an antibody binding assay. In the HuRatTF protein, some sites that affect blood coagulation had been mutated into alanine (Ala), and it contained part of the human and rat sequences.

The HuRatTF protein was diluted to 4 µg/mL in a PBS buffer with a pH of 7.4 and added to a 96-well microplate at 100 µL/well, and the plate was incubated overnight at 4 °C. After the supernatant was discarded, 5% skim milk (BD, 232100) diluted in PBS was added at 300 µL/well for blocking, and the plate was incubated at 37 °C for 2 h. After the blocking, the blocking solution was discarded, and after the plate was washed 3 times with a PBST buffer (pH 7.4 PBS containing 0.1% tween-20), a serially diluted (starting from 20 µg/mL, 5-fold serially diluted) antibody solution was added at 100 µL/well, and the plate was incubated at 37 °C for 1 h. After the incubation, the plate was washed 3 times with PBST, and Peroxidase AffiniPure Goat Anti-Human IgG (H+L) (Jackson, 109-035-003), diluted in a 1:4000 ratio, was added at 100 µL/well. The plate was then incubated at 37 °C for 1 h. After the plate was washed 3 times with PBST, TMB chromogenic substrate (KPL, 5120-0077) was added at 50 µL/well, and the plate was incubated at room temperature for 5 min. Then, 1 M H₂SO₄ was added at 50 µL/well to stop the reaction. The absorbance at 450 nm was measured using a microplate reader. Binding curves of the antibodies to the antigens were fitted using GraphPad Prism. The data are shown in Table 22.

**Table 22. Results of binding assay of antibodies to HuRatTF protein**

| Antibody | EC₅₀ (nM) | Maximum (OD450 nm) |
|---|---|---|
| hu310-2 | 2.134 | 0.4282 |
| hu10H10-77 | 2.004 | 1.3875 |
| hu67Y116 | 53.39 | 0.2916 |
| TF011 | No binding | 0.0585 |

The results show that antibody TF011, which affects the blood coagulation function, does not bind to the HuRatTF protein, and the antibody of the present disclosure, which does not affect the blood coagulation function, binds to the HuRatTF protein.

### Test Example 9: IL-8 Cytokine Assay

A 3 × 10⁵ cells/mL suspension of MDA-MB-231 cells was prepared in a serum-free culture medium and added to a 96-well plate at 100 µL/well, and the cells were starved at 37 °C for 2 h. The supernatant was discarded, and serially diluted (starting from 10 µg/mL, 6-fold serially diluted) antibodies prepared in a serum-free culture medium were added at 100 µL/well. The plate was then incubated at 37 °C for 0.5 h. The supernatant was discarded, and FVIIa (recombinant human coagulation factor VIIa for injection, NovoSeven), prepared in a serum-free culture medium, was added at 120 µL/well. The plate was then incubated at 37 °C for 5 h. After the incubation, the plate was centrifuged at 300 g for 5 min, and 100 µL of the supernatant was transferred to a 96-well plate from an IL-8 kit (NeoBioscience, EHC008). An IL-8 assay was performed according to the instructions. The absorbance at 450 nm was measured using a microplate reader, and binding curves of the antibodies to the antigen were fitted using GraphPad Prism. The EC₅₀ values were calculated, and the results are shown in Table 23.

**Table 23. IL8 assay**

| Antibody | EC₅₀ (nM) |
|---|---|
| hu10H10-77 | 0.412 |
| hu310-2 | 0.407 |
| hu67Y116 | 1.569 |
| hIgG1 | No binding |

The results show that the antibodies of the present disclosure exhibit a relatively strong inhibitory effect on IL8.

### Test Example 10: Cell Activity Assay of ADC Molecules

The purpose of this assay is to assess the killing effects of ADC samples on cells and to evaluate the *in vitro* activity of the ADCs based on IC₅₀ and Imax.

HPAFII cells (human pancreatic cancer cells), MDA-MB-231 cells (human breast cancer cells), H358 cells (human non-small cell lung cancer cells), and CHO-K1 cells (hamster ovary cells) were trypsinized, neutralized with a fresh culture medium, centrifuged at 1000 rpm, resuspended in a culture medium, and counted, and the density of the cell suspensions was then adjusted to: 3700 HPAFII cells/mL, 7400 MDA-MB-231 cells/mL, 7400 H358 cells/mL, and 3700 CHO-K1 cells/mL. 135 µL of each of the cell suspensions was added to a 96-well cell culture plate, i.e., 500 HPAFII cells per well, 1000 MDA-MB-231 cells per well, 1000 H358 cells per well, and 500 CHO-K1 cells per well. Only 135 µL of culture medium was added to column 11 (no cells). The plate was then incubated at 37 °C with 5% carbon dioxide for 16 h.

The ADC samples were diluted to 3.333 µM (10× concentration) in PBS, and that concentration was used as the initial concentration and five-fold serially diluted with PBS to obtain a total of 9 concentrations. 15 µL of 10× serially diluted solution was added to each well. The 10th and 11th wells contained the culture medium. The solution of each concentration point was added in duplicate. The plate was incubated at 37 °C with 5% carbon dioxide for 6 days.

After the incubation, CTG was added at 50 µL/well, and the plate was incubated at room temperature for 10 min in a dark place. A white membrane was attached to the bottom of the cell culture plate, and the chemiluminescence was measured using ENVISION. The *in vitro* cell killing activity results are shown in Table 24.

**Table 24. In vitro cell killing activity of ADCs**

| ADC | HPAF II | | MDA-MB-231 | | H358 | | CHO-K1 | |
|---|---|---|---|---|---|---|---|---|
| | IC₅₀ (nM) | Imax (%) | IC₅₀ (nM) | Imax (%) | IC₅₀ (nM) | Imax (%) | IC₅₀ (nM) | Imax (%) |
| ADC-1 | 1.06 | 61.78 | 19.31 | 79.43 | 11.07 | 73.32 | >333 | 7.01 |
| ADC-6 | 0.44 | 65.52 | 11.98 | 79.97 | 1.13 | 69.9 | >333 | 4.37 |
| ADC-4 | 0.15 | 65.75 | 3.31 | 79.26 | 0.53 | 73.39 | >333 | 4.34 |
| ADC-2 | 0.30 | 77.79 | 3.38 | 86.48 | 3.40 | 85.72 | >333 | 1.80 |

The results show that the above ADCs exhibit relatively strong killing effects on the cell strains HPAFII, MDA-MB-231, and H358.

### Test Example 11: In Vivo Efficacy Assay in CDX Model of HPAFII

In this test example, the efficacy of ADC drugs against NUNU mouse subcutaneous xenograft tumors was evaluated and compared.

Each NUNU mouse (4-6 weeks, female, SPF, Beijing Vital River Laboratory Animal Technology Co., Ltd.) was subcutaneously inoculated with 4 × 10⁶ HPAF-II cells (human pancreatic cancer)/200 µL (containing 50% matrigel). After the tumors grew to about 250-300 mm³, the animals were grouped according to tumor volume (D0). The mice were dosed by intraperitoneal injection (IP) once weekly, and a total of 3 injections were administered. The volume of the once-weekly administration was 10 mL/kg. Specific doses and administration regimens are shown in Table 25. The tumor volume was measured twice weekly, the body weight of the mice was measured, and the measurements were recorded.

The experimental index was to study the effects of the drugs on tumor growth, and the specific index was T/C% or tumor growth inhibition rate TGI%. The tumor diameter was measured twice weekly with a vernier caliper, and the tumor volume (V) was calculated using the formula: V = 1/2 × a × b², where a and b represent length and width, respectively. T/C% = (T - T0)/(C - C0) × 100, where T and C represent tumor volumes at the end of the experiment; T0 and C0 represent tumor volumes at the beginning of the experiment; T represents the tumor volume of the ADC treatment groups, and C represents the tumor volume of the control group. Tumor growth inhibition rate% (TGI%) = 100 - T/C%; when a tumor regressed, the tumor growth inhibition rate% (TGI%) = 100 - (T - T0)/T0 × 100; if the volume of a tumor was smaller than its initial volume, i.e., T < T0 or C < C0, it was defined as partial regression (PR) of the tumor; if a tumor completely disappeared, it was defined as complete regression (CR) of the tumor. Unless otherwise specified, a two-way ANOVA test was used to compare the tumor volumes of two groups, with P < 0.05 defined as indicating a statistically significant difference. The results are shown in Table 25.

**Table 25. Efficacy of ADCs against HPAF-II nude mouse subcutaneous xenograft tumors**

| Group | Mean tumor volume (mm³ ± SEM) | | T/C (%) D25 | Tumor growth inhibition rate (%) D25 | P value D25 | Partial regression |
|---|---|---|---|---|---|---|
| | DO | D25 | | | | |
| Blank control (PBS) | 278.2±16.0 | 2127.1±96.0 | - | - | - | 0/8 |
| ADC-1 (1 mg/kg) | 277.7±14.7 | 1869.2±244.6 | 86 | 13.93 | 0.343 | 0/8 |
| ADC-1 (2 mg/kg) | 277.5±15.5 | 815.2±105.6 | 29 | 70.92 | <0.001 | 0/8 |
| ADC-4 (1 mg/kg) | 280.3±14.1 | 1926.5±218.2 | 89 | 10.96 | 0.414 | 0/8 |
| ADC-4 (2 mg/kg) | 279.9±12.3 | 690.4:1:112.9 | 22 | 77.79 | <0.001 | 0/8 |
| hIgG-9A (2 mg/kg) | 277.5±13.6 | 2012.5±207.5 | 94 | 6.17 | 0.624 | 0/8 |

| | | | | | | |
|---|---|---|---|---|---|---|
| P value: compared to the blank control group; IP: intraperitoneal injection; partial regression: the D25 tumor volume was less than the D0 tumor volume. | | | | | | |

After three administrations, the antibodies of the ADC-4 (2mg/kg) and ADC-1 (2 mg/kg) groups exhibit significant efficacy against HPAF-II nude mouse subcutaneous xenograft tumors, showing significant difference compared to the blank control group; the drugs exhibit a significant dose dependence. The tumor-bearing mice well tolerate all of the above drugs.

### Test Example 12: In Vivo Efficacy Assay in CDX Model of H358

In this test example, the efficacy of ADC drugs against nude mouse subcutaneous xenograft tumors was evaluated and compared.

Each nude mouse (4-6 weeks, female, SPF, Shanghai SLAC Laboratory Animal Co., Ltd.) was subcutaneously inoculated with 5 × 10⁶ H358 cells (human non-small cell lung cancer)/200 µL (containing 50% matrigel). After the tumors grew to about 300 mm³, the animals were grouped according to tumor volume (D0). The mice were dosed by intraperitoneal injection (IP) once weekly, and a total of 3 injections were administered. The volume of the once-weekly administration was 10 mL/kg. Specific doses and administration regimens are shown in Table 26. The tumor volume was measured twice weekly, the body weight of the mice was measured, and the measurements were recorded. The experimental index was to study the effects of the drugs on tumor growth, and the specific index was T/C% or tumor growth inhibition rate TGI%. The tumor diameter was measured twice weekly with a vernier caliper, and the tumor volume (V) was calculated using the formula: V = 1/2 × a × b², where a and b represent length and width, respectively. T/C% = (T - T0)/(C - C0) × 100, where T and C represent tumor volumes at the end of the experiment; T0 and C0 represent tumor volumes at the beginning of the experiment; T represents the tumor volume of the ADC treatment groups, and C represents the tumor volume of the control group. Tumor growth inhibition rate% (TGI%) = 100 - T/C% ; when a tumor regressed, the tumor growth inhibition rate% (TGI%) = 100 - (T - T0)/T0 × 100; if the volume of a tumor was smaller than its initial volume, i.e., T < T0 or C < C0, it was defined as partial regression (PR) of the tumor; if a tumor completely disappeared, it was defined as complete regression (CR) of the tumor. Unless otherwise specified, a two-way ANOVA test was used to compare the tumor volumes of two groups, with P < 0.05 defined as indicating a statistically significant difference. The results are shown in Table 26.

**Table 26. Efficacy of ADCs against H358 nude mouse subcutaneous xenograft tumors**

| Group | Mean tumor volume (mm³ ± SEM) | | T/C (%) D32 | Tumor growth inhibition rate (%) D32 | P value D32 | Partial regression | Number of animals at beginning/end of experiment |
|---|---|---|---|---|---|---|---|
| | DO | D32 | | | | | |
| hIgG-9A (3mg/kg) | 310.4±17.6 | 813.9±61.8 | - | - | - | - | 8/7 |
| ADC-1 (3 mg/kg) | 309.7±20.3 | 182.2±24.5 | -25 | 141.2 | <0.001 | 8/8 | 8/8 |
| ADC-1 (1 mg/kg) | 308.5±18.4 | 323.8±43.0 | 3 | 96.97 | <0.001 | 3/8 | 8/8 |
| ADC-4 (3 mg/kg) | 307.8±18.0 | 201.8:1:15.7 | -21 | 134.4 | <0.001 | 7/8 | 8/8 |
| ADC-4 (1 mg/kg) | 309.6±19.5 | 252.2±21.5 | -11 | 118.5 | <0.001 | 8/8 | 8/7 |
| ADC-6 (3 mg/kg) | 310.6±19.1 | 178.9±11.6 | -26 | 142.4 | <0.001 | 8/8 | 8/6 |
| ADC-6 (1 mg/kg) | 309.9±19.8 | 179.8±24.7 | -26 | 142.0 | <0.001 | 8/8 | 8/8 |

| | | | | | | | |
|---|---|---|---|---|---|---|---|
| P value: compared to the negative antibody group; IP: intraperitoneal injection; partial regression: the D32 tumor volume was less than the DO tumor volume. ADC-1 (3 mg/kg and 1 mg/kg), ADC-4 (3 mg/kg and 1 mg/kg) and ADC-6 (3 mg/kg and 1 mg/kg) exhibit very good anti-tumor effects after three administrations, inducing partial regression of tumors; the drugs exhibit a certain dose dependence. The tumor-bearing mice well tolerate all of the above drugs. | | | | | | | |

### Test Example 13: In Vivo Efficacy Assay in CDX Model of MDA-MB-231

In this test example, the efficacy of ADC drugs against NOD scid mouse subcutaneous xenograft tumors was evaluated and compared.

Each NOD scid mouse (4-6 weeks, female, SPF, Shanghai SLAC Laboratory Animal Co., Ltd.) was subcutaneously inoculated with 3 × 10⁶ MDA-MB-231 cells (human breast cancer)/100 µL (containing 50% matrigel). After the tumors grew to about 250-300 mm³, the animals were grouped according to tumor volume (D0). The mice were dosed by intraperitoneal injection (IP) once weekly, and a total of 3 injections were administered. The volume of the once-weekly administration was 10 mL/kg. The tumor volume was measured twice weekly, the body weight of the mice was measured, and the measurements were recorded.

The experimental index was to study the effects of the drugs on tumor growth, and the specific index was T/C% or tumor growth inhibition rate TGI%. The tumor diameter was measured twice weekly with a vernier caliper, and the tumor volume (V) was calculated using the formula: V = 1/2 × a × b², where a and b represent length and width, respectively. T/C% = (T - T0)/(C - C0) × 100, where T and C represent tumor volumes at the end of the experiment; T0 and C0 represent tumor volumes at the beginning of the experiment; T represents the tumor volume of the ADC treatment groups, and C represents the tumor volume of the control group. Tumor growth inhibition rate% (TGI%) = 100 - T/C% ; when a tumor regressed, the tumor growth inhibition rate% (TGI%) = 100 - (T - T0)/T0 × 100; if the volume of a tumor was smaller than its initial volume, i.e., T < T0 or C < C0, it was defined as partial regression (PR) of the tumor; if a tumor completely disappeared, it was defined as complete regression (CR) of the tumor. Unless otherwise specified, a two-way ANOVA test was used to compare the tumor volumes of two groups, with P < 0.05 defined as indicating a statistically significant difference. The tumor-bearing mice well tolerate all of the above drugs, and no symptoms such as significant weight loss are observed. PBS was used as a blank control. The results are shown in Table 27.

**Table 27. Efficacy of ADCs against MDA-MB-231 mouse subcutaneous xenograft tumors**

| Group | Mean tumor volume (mm³ ± SEM) | | T/C (%) D21 | % Tumor growth inhibition rate D21 | P value D21 | Partial regression |
|---|---|---|---|---|---|---|
| | D0 | D21 | | | | |
| Blank control (PBS) | 273.4±15.5 | 1168.1±66.0 | - | - | - | 0/8 |
| hIgG-9A (3mg/kg) | 270.2±17.2 | 586.5±96.5 | 35 | 64.64 | <0.001 | 1/8 |
| ADC-1 (1mg/kg) | 271.8±15.9 | 975.0±88.5 | 79 | 21.40 | 0.102 | 0/8 |
| ADC-1 (3 mg/kg) | 271.3±16.6 | 235.0±57.4 | -4 | 113.39 | <0.001 | 6/8 |
| ADC-2 (1 mg/kg) | 274.2±17.3 | 747.8±67.2 | 53 | 47.07 | <0.001 | 0/8 |
| ADC-3 (1 mg/kg) | 271.1±19.5 | 730.7±78.5 | 51 | 48.62 | <0.001 | 0/8 |
| ADC-6 (1 mg/kg) | 272.6±19.7 | 860.4±76.7 | 66 | 34.30 | 0.009 | 0/8 |
| ADC-6 (3 mg/kg) | 271.9±17.1 | 244.1±47.6 | -3 | 110.23 | <0.001 | *7*/*8* |

| | | | | | | |
|---|---|---|---|---|---|---|
| P value: compared to the blank control group; IP: intraperitoneal injection; partial regression: the D21 tumor volume was less than the D0 tumor volume. | | | | | | |

After three administrations, the antibodies of the ADC-1 (3 mg/kg and 1 mg/kg), ADC-2 (1 mg/kg), ADC-3 (1 mg/kg), ADC-6 (3 mg/kg and 1 mg/kg), and hIgG-9A (3 mg/kg) groups all exhibit significant efficacy against MDA-MB-231 mouse subcutaneous xenograft tumors, showing significant difference compared to the blank control group; the drugs exhibit a significant dose dependence. The tumor-bearing mice well tolerate all of the above drugs.

### Test Example 14: PK and Toxicity Observation Assays in Cynomolgus Monkeys

In this test example, the PK of ADC-1 and ADC-2 in cynomolgus monkeys was evaluated, and meanwhile, toxicity observations were performed.

Each cynomolgus monkey (Suzhou Xishan Zhongke Laboratory Animal Co., Ltd.; SCXK (Jiangsu) 2018-0001) was dosed by intravenous infusion at a dose of 12 mg/kg. Blood was collected at 0.083 h (5 min ± 1 min), 1 h (± 5 min), 6 h (± 15 min), 12 h (± 15 min), 24 h (± 30 min), 48 h (± 30 min), 96 h (± 30 min), 168 h (± 30 min), 240 h (± 60 min), 336 h (± 60 min), and 504 h (± 60 min) post-dose, and plasma concentration determination and other toxicological observations were performed.

In the PK assay, the total antibody and intact ADC concentrations of ADC-1 in cynomolgus monkey serum were quantitatively determined using the LBA method (Delfia).

**Table 28. Pharmacokinetic total antibody and intact ADC plasma concentrations of ADC-1 in cynomolgus monkeys**

| 12mpk | Metabolic data |
|---|---|
| Total antibody half-life (days) | 4.0 ± 0.2 |
| Intact ADC half-life (days) | 3.7 ± 0.1 |
| AUC 0-t-total antibody (µg/mL*h) | 22609 |
| AUC 0-t-intact ADC (µg/mL*h) | 18923 |
| AUC 0-t-free toxin (ng/mL*h) | 18.4 |
| AUC ratio (intact ADC/total antibody) | 84% |
| CL-total antibody (mL/day/kg) | 12.4 |
| CL-intact ADC (mL/day/kg) | 15.0 |

**Table 29. Pharmacokinetic total antibody and intact ADC plasma concentrations of ADC-2 in cynomolgus monkeys**

| 12mpk | Metabolic data |
|---|---|
| Total antibody half-life (days) | 2.8 ± 0.2 |
| Intact ADC half-life (days) | 2.6 ± 0.1 |
| AUC 0-t-total antibody (µg/mL*h) | 17693 |
| AUC 0-t-intact ADC (µg/mL*h) | 15786 |
| AUC ratio (intact ADC/total antibody) | 89% |
| CL-total antibody (mL/day/kg) | 16.1 |
| CL-intact ADC (mL/day/kg) | 18.2 |

The half-lives and AUCs of the total antibodies of the ADC-1 and ADC-2 molecules were substantially consistent with those of the intact ADCs in cynomolgus monkeys, indicating that the ADC-1 and ADC-2 molecules have relatively good stability in cynomolgus monkeys; both their half-lives and AUCs after the second administration were lower than those after the first administration due to the presence of immunogenicity.

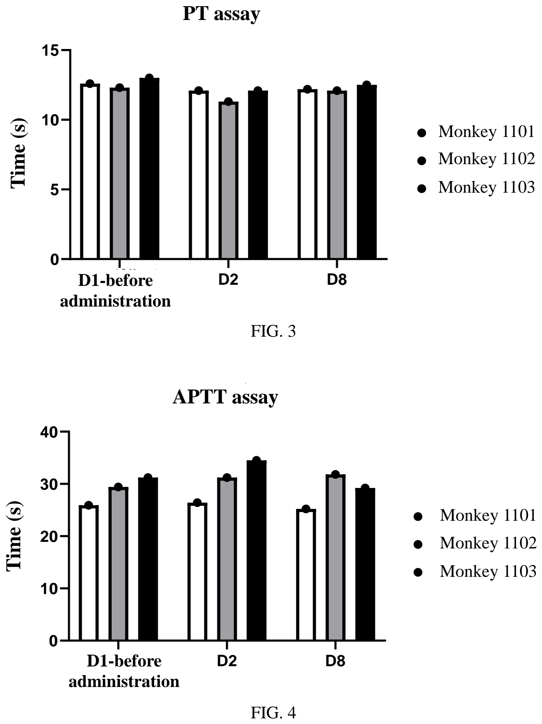

During the pharmacokinetic assay of ADC-1, blood was collected from all animals before the first administration, on day 2, and on day 8, and the blood coagulation function was tested. The animals were fed overnight before blood collection. Blood samples were collected, and PT and APTT assays were performed using a Sysmex CS-2000i fully automatic blood coagulation analyzer or a Sysmex CA-1500 automatic blood coagulation analyzer. The results of the PT and APTT assay are shown in FIGs. 3 and 4. The results show that there are no significant increases in blood coagulation time and risk of bleeding compared to the situation before administration.

### Test Example 15: TGA Assay

In this test example, it is determined whether antibody hu67Y116 affects the blood coagulation activity of TF.

The human plasma contains the coagulation factors of the extrinsic blood coagulation pathway other than TF, such as FVII, FX, FV, FII, and FI. If an antibody does not affect the blood coagulation activity of TF, it will not affect the blood coagulation time and peak value of the human plasma. Human plasma was prepared according to the instructions of a Technothrombin TGA Kit. Then a 20× antibody was prepared, and the antibody and plasma were mixed in a ratio of 1:19. The mixture was added to a black 96-well plate at 40 µL/well, and RCH (TGA reagent C high, provided in the kit) was then added at 10 µL/well. Finally, a substrate was added at 50 µL/well to start the reaction. The fluorescence was measured (~360 nm/~460 nm, excitation/emission) with a microplate reader. The results are shown in Table 30. TF011 exhibits a significant inhibitory effect on blood coagulation compared to the negative control antibody, while hu67Y116 has little effect on blood coagulation.

**Table 30. Results of TGA assay**

| **Antibody** | **Adjustment period** | **Peak value** | |
|---|---|---|---|
| | **Time [min]** | **Thrombin [nM]** | **Time [min]** |
| TF011 | 37 | 93.9 | 82 |
| hu67Y116 | 27 | 111.9 | 47 |
| hIgG1 | 24 | 128.6 | 47 |

## Claims

1. An anti-TF antibody, comprising a heavy chain variable region and a light chain variable region, wherein:
a. the heavy chain variable region comprises a HCDR1 comprising the amino acid sequence of SEQ ID NO: 40, a HCDR2 comprising the amino acid sequence of SEQ ID NO: 64 or 41, and a HCDR3 comprising the amino acid sequence of SEQ ID NO: 65 or 42, and the light chain variable region comprises a LCDR1 comprising the amino acid sequence of SEQ ID NO: 66 or 43, a LCDR2 comprising the amino acid sequence of SEQ ID NO: 67 or 44, and a LCDR3 comprising the amino acid sequence of SEQ ID NO: 68, 83, or 45;
b. the heavy chain variable region comprises a HCDR1 comprising the amino acid sequence of SEQ ID NO: 46, a HCDR2 comprising the amino acid sequence of SEQ ID NO: 47, and a HCDR3 comprising the amino acid sequence of SEQ ID NO: 48, and the light chain variable region comprises a LCDR1 comprising the amino acid sequence of SEQ ID NO: 49, 111, 112, or 113, a LCDR2 comprising the amino acid sequence of SEQ ID NO: 50, and a LCDR3 comprising the amino acid sequence of SEQ ID NO: 51;
c. the heavy chain variable region comprises a HCDR1 comprising the amino acid sequence of SEQ ID NO: 73 or 144, a HCDR2 comprising the amino acid sequence of SEQ ID NO: 74, 142, 145, or 146, and a HCDR3 comprising the amino acid sequence of SEQ ID NO: 75 or 143, and the light chain variable region comprises a LCDR1 comprising the amino acid sequence of SEQ ID NO: 76, 151, 147, or 150, a LCDR2 comprising the amino acid sequence of SEQ ID NO: 77 or 148, and a LCDR3 comprising the amino acid sequence of SEQ ID NO: 78 or 149;
preferably,
a. the heavy chain variable region comprises a HCDR1 comprising the amino acid sequence of SEQ ID NO: 40, a HCDR2 comprising the amino acid sequence of SEQ ID NO: 64, and a HCDR3 comprising the amino acid sequence of SEQ ID NO: 65, and the light chain variable region comprises a LCDR1 comprising the amino acid sequence of SEQ ID NO: 66, a LCDR2 comprising the amino acid sequence of SEQ ID NO: 67, and a LCDR3 comprising the amino acid sequence of SEQ ID NO: 68 or 83; or
the heavy chain variable region comprises a HCDR1 comprising the amino acid sequence of SEQ ID NO: 40, a HCDR2 comprising the amino acid sequence of SEQ ID NO: 41, and a HCDR3 comprising the amino acid sequence of SEQ ID NO: 42, and the light chain variable region comprises a LCDR1 comprising the amino acid sequence of SEQ ID NO: 43, a LCDR2 comprising the amino acid sequence of SEQ ID NO: 44, and a LCDR3 comprising the amino acid sequence of SEQ ID NO: 45;
b. the heavy chain variable region comprises a HCDR1 comprising the amino acid sequence of SEQ ID NO: 46, a HCDR2 comprising the amino acid sequence of SEQ ID NO: 47, and a HCDR3 comprising the amino acid sequence of SEQ ID NO: 48, and the light chain variable region comprises a LCDR1 comprising the amino acid sequence of SEQ ID NO: 49, a LCDR2 comprising the amino acid sequence of SEQ ID NO: 50, and a LCDR3 comprising the amino acid sequence of SEQ ID NO: 51;
c. the heavy chain variable region comprises a HCDR1 comprising the amino acid sequence of SEQ ID NO: 73, a HCDR2 comprising the amino acid sequence of SEQ ID NO: 74, and a HCDR3 comprising the amino acid sequence of SEQ ID NO: 75, and the light chain variable region comprises a LCDR1 comprising the amino acid sequence of SEQ ID NO: 76, or 151, a LCDR2 comprising the amino acid sequence of SEQ ID NO: 77 or 148, and a LCDR3 comprising the amino acid sequence of SEQ ID NO: 78.

2. The anti-TF antibody according to claim 1, wherein the anti-TF antibody is a murine antibody, a chimeric antibody, or a humanized antibody, preferably a chimeric antibody or a humanized antibody, and more preferably a humanized antibody.

3. The anti-TF antibody according to claim 1 or 2, comprising a heavy chain variable region and a light chain variable region, wherein:
a. the heavy chain variable region comprises an amino acid sequence having at least 80% sequence identity to SEQ ID NO: 62, 58, 130, 131, or 132, and the light chain variable region comprises an amino acid sequence having at least 80% sequence identity to SEQ ID NO: 63, 81, 59, 133, 134, or 135; or the heavy chain variable region comprises an amino acid sequence having at least 80% sequence identity to SEQ ID NO: 36, and the light chain variable region comprises an amino acid sequence having at least 80% sequence identity to SEQ ID NO: 37;
b. the heavy chain variable region comprises an amino acid sequence having at least 80% sequence identity to SEQ ID NO: 52 or 114, and the light chain variable region comprises an amino acid sequence having at least 80% sequence identity to SEQ ID NO: 53, 115, 116, 117, 118, 119, 120, or 121; or the heavy chain variable region comprises an amino acid sequence having at least 80% sequence identity to SEQ ID NO: 38, and the light chain variable region comprises an amino acid sequence having at least 80% sequence identity to SEQ ID NO: 39;
c. the heavy chain variable region comprises an amino acid sequence having at least 80% sequence identity to SEQ ID NO: 71, 84, 85, 86, 87, 88, 89, 90, 91, 92, 93, 94, 95, or 96, and the light chain variable region comprises an amino acid sequence having at least 80% sequence identity to SEQ ID NO: 72, 97, 102, 98, 99, 100, or 101;
preferably,
a. the heavy chain variable region comprises the amino acid sequence of SEQ ID NO: 62, 58, 130, 131, or 132, and the light chain variable region comprises the amino acid sequence of SEQ ID NO: 63, 81, 59, 133, 134, or 135; or the heavy chain variable region comprises the amino acid sequence of SEQ ID NO: 36, and the light chain variable region comprises the amino acid sequence of SEQ ID NO: 37;
b. the heavy chain variable region comprises the amino acid sequence of SEQ ID NO: 52 or 114, and the light chain variable region comprises the amino acid sequence of SEQ ID NO: 53, 115, 116, 117, 118, 119, 120, or 121; or the heavy chain variable region comprises the amino acid sequence of SEQ ID NO: 38, and the light chain variable region comprises the amino acid sequence of SEQ ID NO: 39;
c. the heavy chain variable region comprises the amino acid sequence of SEQ ID NO: 71, 84, 85, 86, 87, 88, 89, 90, 91, 92, 93, 94, 95, or 96, and the light chain variable region comprises the amino acid sequence of SEQ ID NO: 72, 97, 102, 98, 99, 100, or 101;
more preferably,
a. the heavy chain variable region comprises the amino acid sequence of SEQ ID NO: 62, and the light chain variable region comprises the amino acid sequence of SEQ ID NO: 63 or 81; or
the heavy chain variable region comprises the amino acid sequence of SEQ ID NO: 58, and the light chain variable region comprises the amino acid sequence of SEQ ID NO: 59; or
the heavy chain variable region comprises the amino acid sequence of SEQ ID NO: 36, and the light chain variable region comprises the amino acid sequence of SEQ ID NO: 37; or
the heavy chain variable region comprises the amino acid sequence of SEQ ID NO: 130, and the light chain variable region comprises the amino acid sequence of SEQ ID NO: 133; or
the heavy chain variable region comprises the amino acid sequence of SEQ ID NO: 131, and the light chain variable region comprises the amino acid sequence of SEQ ID NO: 133; or
the heavy chain variable region comprises the amino acid sequence of SEQ ID NO: 58, and the light chain variable region comprises the amino acid sequence of SEQ ID NO: 133; or
the heavy chain variable region comprises the amino acid sequence of SEQ ID NO: 132, and the light chain variable region comprises the amino acid sequence of SEQ ID NO: 133; or
the heavy chain variable region comprises the amino acid sequence of SEQ ID NO: 130, and the light chain variable region comprises the amino acid sequence of SEQ ID NO: 134; or
the heavy chain variable region comprises the amino acid sequence of SEQ ID NO: 131, and the light chain variable region comprises the amino acid sequence of SEQ ID NO: 134; or
the heavy chain variable region comprises the amino acid sequence of SEQ ID NO: 58, and the light chain variable region comprises the amino acid sequence of SEQ ID NO: 134; or
the heavy chain variable region comprises the amino acid sequence of SEQ ID NO: 132, and the light chain variable region comprises the amino acid sequence of SEQ ID NO: 134; or
the heavy chain variable region comprises the amino acid sequence of SEQ ID NO: 130,
and the light chain variable region comprises the amino acid sequence of SEQ ID NO: 59; or
the heavy chain variable region comprises the amino acid sequence of SEQ ID NO: 131, and the light chain variable region comprises the amino acid sequence of SEQ ID NO: 59; or
the heavy chain variable region comprises the amino acid sequence of SEQ ID NO: 132, and the light chain variable region comprises the amino acid sequence of SEQ ID NO: 59; or
the heavy chain variable region comprises the amino acid sequence of SEQ ID NO: 130, and the light chain variable region comprises the amino acid sequence of SEQ ID NO: 135; or
the heavy chain variable region comprises the amino acid sequence of SEQ ID NO: 131, and the light chain variable region comprises the amino acid sequence of SEQ ID NO: 135; or
the heavy chain variable region comprises the amino acid sequence of SEQ ID NO: 58, and the light chain variable region comprises the amino acid sequence of SEQ ID NO: 135; or
the heavy chain variable region comprises the amino acid sequence of SEQ ID NO: 132, and the light chain variable region comprises the amino acid sequence of SEQ ID NO: 135;
b. the heavy chain variable region comprises the amino acid sequence of SEQ ID NO: 52, and the light chain variable region comprises the amino acid sequence of SEQ ID NO: 53; or
the heavy chain variable region comprises the amino acid sequence of SEQ ID NO: 38, and the light chain variable region comprises the amino acid sequence of SEQ ID NO: 39;
c. the heavy chain variable region comprises the amino acid sequence of SEQ ID NO: 71, and the light chain variable region comprises the amino acid sequence of SEQ ID NO: 72, 97, or 102.

4. The anti-TF antibody according to any one of claims 1 to 3, wherein the anti-TF antibody is an antibody fragment, and preferably, the antibody fragment is selected from the group consisting of Fab, Fab', F(ab')2, Fd, Fv, scFv, dsFv, and dAb.

5. The anti-TF antibody according to any one of claims 1 to 4, comprising a heavy chain constant region and a light chain constant region, wherein preferably, the heavy chain constant region is a human IgG1, IgG2, IgG3, or IgG4 heavy chain constant region, and the light chain constant region is a human κ or λ chain constant region; more preferably, the heavy chain constant region comprises the amino acid sequence of SEQ ID NO: 54, and the light chain constant region comprises the amino acid sequence of SEQ ID NO: 55.

6. The anti-TF antibody according to any one of claims 1 to 5, wherein the anti-TF antibody is selected from the group consisting of antibodies comprising a heavy chain and a light chain according to any one of the following:
a. a heavy chain having at least 80% sequence identity to SEQ ID NO: 69, 60, 136, 137, or 138, and/or a light chain having at least 80% sequence identity to SEQ ID NO: 70, 82, 61, 139, 140, or 141; or
a heavy chain having at least 80% sequence identity to SEQ ID NO: 107, and/or a light chain having at least 80% sequence identity to SEQ ID NO: 108;
b. a heavy chain having at least 80% sequence identity to SEQ ID NO: 56 or 122, and/or a light chain having at least 80% sequence identity to SEQ ID NO: 57, 123, 124, 125, 126, 127, 128, or 129; or
a heavy chain having at least 80% sequence identity to SEQ ID NO: 109, and/or a light chain having at least 80% sequence identity to SEQ ID NO: 110;
c. a heavy chain having at least 80% sequence identity to SEQ ID NO: 79, 152, 153, 154, 155, 156, 157, 158, 159, 160, 161, 162, 163, or 164, and/or a light chain having at least 80% sequence identity to SEQ ID NO: 80, 165, 170, 166, 167, 168, or 169;
preferably,
a. a heavy chain having at least 80% sequence identity to SEQ ID NO: 69, and/or a light chain having at least 80% sequence identity to SEQ ID NO: 70 or 82; or
a heavy chain having at least 80% sequence identity to SEQ ID NO: 60, and/or a light chain having at least 80% sequence identity to SEQ ID NO: 61; or
a heavy chain having at least 80% sequence identity to SEQ ID NO: 107, and/or a light chain having at least 80% sequence identity to SEQ ID NO: 108; or
a heavy chain having at least 80% sequence identity to SEQ ID NO: 136, and/or a light chain having at least 80% sequence identity to SEQ ID NO: 139; or
a heavy chain having at least 80% sequence identity to SEQ ID NO: 137, and/or a light chain having at least 80% sequence identity to SEQ ID NO: 139; or
a heavy chain having at least 80% sequence identity to SEQ ID NO: 60, and/or a light chain having at least 80% sequence identity to SEQ ID NO: 139; or
a heavy chain having at least 80% sequence identity to SEQ ID NO: 138, and/or a light chain having at least 80% sequence identity to SEQ ID NO: 139; or
a heavy chain having at least 80% sequence identity to SEQ ID NO: 136, and/or a light chain having at least 80% sequence identity to SEQ ID NO: 140; or
a heavy chain having at least 80% sequence identity to SEQ ID NO: 137, and/or a light chain having at least 80% sequence identity to SEQ ID NO: 140; or
a heavy chain having at least 80% sequence identity to SEQ ID NO: 60, and/or a light chain having at least 80% sequence identity to SEQ ID NO: 140; or
a heavy chain having at least 80% sequence identity to SEQ ID NO: 138, and/or a light chain having at least 80% sequence identity to SEQ ID NO: 140; or
a heavy chain having at least 80% sequence identity to SEQ ID NO: 136, and/or a light chain having at least 80% sequence identity to SEQ ID NO: 61; or
a heavy chain having at least 80% sequence identity to SEQ ID NO: 137, and/or a light chain having at least 80% sequence identity to SEQ ID NO: 61; or
a heavy chain having at least 80% sequence identity to SEQ ID NO: 138, and/or a light chain having at least 80% sequence identity to SEQ ID NO: 61; or
a heavy chain having at least 80% sequence identity to SEQ ID NO: 136, and/or a light chain having at least 80% sequence identity to SEQ ID NO: 141; or
a heavy chain having at least 80% sequence identity to SEQ ID NO: 137, and/or a light chain having at least 80% sequence identity to SEQ ID NO: 141; or
a heavy chain having at least 80% sequence identity to SEQ ID NO: 60, and/or a light chain having at least 80% sequence identity to SEQ ID NO: 141; or
a heavy chain having at least 80% sequence identity to SEQ ID NO: 138, and/or a light chain having at least 80% sequence identity to SEQ ID NO: 141;
b. a heavy chain having at least 80% sequence identity to SEQ ID NO: 56, and/or a light chain having at least 80% sequence identity to SEQ ID NO: 57; or
a heavy chain having at least 80% sequence identity to SEQ ID NO: 109, and/or a light chain having at least 80% sequence identity to SEQ ID NO: 110;
c. a heavy chain having at least 80% sequence identity to SEQ ID NO: 79, and/or a light chain having at least 80% sequence identity to SEQ ID NO: 80, 165, or 170;
more preferably,
a. the heavy chain comprises the amino acid sequence of SEQ ID NO: 69, and the light chain comprises the amino acid sequence of SEQ ID NO: 70; or
the heavy chain comprises the amino acid sequence of SEQ ID NO: 69, and the light chain comprises the amino acid sequence of SEQ ID NO: 82; or
the heavy chain comprises the amino acid sequence of SEQ ID NO: 60, and the light chain comprises the amino acid sequence of SEQ ID NO: 61; or
the heavy chain comprises the amino acid sequence of SEQ ID NO: 107, and the light chain comprises the amino acid sequence of SEQ ID NO: 108; or
the heavy chain comprises the amino acid sequence of SEQ ID NO: 136, and the light chain comprises the amino acid sequence of SEQ ID NO: 139; or
the heavy chain comprises the amino acid sequence of SEQ ID NO: 137, and the light chain comprises the amino acid sequence of SEQ ID NO: 139; or
the heavy chain comprises the amino acid sequence of SEQ ID NO: 60, and the light chain comprises the amino acid sequence of SEQ ID NO: 139; or
the heavy chain comprises the amino acid sequence of SEQ ID NO: 138, and the light chain comprises the amino acid sequence of SEQ ID NO: 139; or
the heavy chain comprises the amino acid sequence of SEQ ID NO: 136, and the light chain comprises the amino acid sequence of SEQ ID NO: 140; or
the heavy chain comprises the amino acid sequence of SEQ ID NO: 137, and the light chain comprises the amino acid sequence of SEQ ID NO: 140; or
the heavy chain comprises the amino acid sequence of SEQ ID NO: 60, and the light chain comprises the amino acid sequence of SEQ ID NO: 140; or
the heavy chain comprises the amino acid sequence of SEQ ID NO: 138, and the light chain comprises the amino acid sequence of SEQ ID NO: 140; or
the heavy chain comprises the amino acid sequence of SEQ ID NO: 136, and the light chain comprises the amino acid sequence of SEQ ID NO: 61; or
the heavy chain comprises the amino acid sequence of SEQ ID NO: 137, and the light chain comprises the amino acid sequence of SEQ ID NO: 61; or
the heavy chain comprises the amino acid sequence of SEQ ID NO: 138, and the light chain comprises the amino acid sequence of SEQ ID NO: 61; or
the heavy chain comprises the amino acid sequence of SEQ ID NO: 136, and the light chain comprises the amino acid sequence of SEQ ID NO: 141; or
the heavy chain comprises the amino acid sequence of SEQ ID NO: 137, and the light chain comprises the amino acid sequence of SEQ ID NO: 141; or
the heavy chain comprises the amino acid sequence of SEQ ID NO: 60, and the light chain comprises the amino acid sequence of SEQ ID NO: 141; or
the heavy chain comprises the amino acid sequence of SEQ ID NO: 138, and the light chain comprises the amino acid sequence of SEQ ID NO: 141;
b. the heavy chain comprises the amino acid sequence of SEQ ID NO: 56, and the light chain comprises the amino acid sequence of SEQ ID NO: 57; or
the heavy chain comprises the amino acid sequence of SEQ ID NO: 109, and the light chain comprises the amino acid sequence of SEQ ID NO: 110;
c. the heavy chain comprises the amino acid sequence of SEQ ID NO: 79, and the light chain comprises the amino acid sequence of SEQ ID NO: 80; or
the heavy chain comprises the amino acid sequence of SEQ ID NO: 79, and the light chain comprises the amino acid sequence of SEQ ID NO: 165; or
the heavy chain comprises the amino acid sequence of SEQ ID NO: 79, and the light chain comprises the amino acid sequence of SEQ ID NO: 170.

7. A nucleic acid molecule encoding the anti-TF antibody according to any one of claims 1 to 6.

8. A host cell, comprising the nucleic acid molecule according to claim 7.

9. An immunoconjugate, comprising the anti-TF antibody according to any one of claims 1 to 6 and an effector molecule, wherein the effector molecule is conjugated to the anti-TF antibody; preferably, the effector molecule is selected from the group consisting of an anti-tumor agent, an immunomodulator, a biological response modifier, a lectin, a cytotoxic drug, a chromophore, a fluorophore, a chemiluminescent compound, an enzyme, a metal ion, and any combination thereof.

10. An antibody-drug conjugate of general formula (Pc-L-Y-D) or a pharmaceutically acceptable salt thereof: 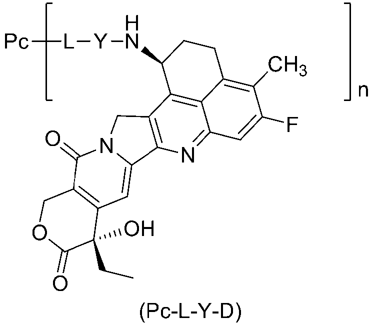 wherein:
Y is -O-(CR^{a}R^{b})ₘ-CR¹R²-C(O)-;
wherein R^{a} and R^{b} are identical or different and are each independently selected from the group consisting of a hydrogen atom, a deuterium atom, halogen, and C₁₋₆ alkyl;
R¹ is C₃₋₆ cycloalkyl-C₁₋₆ alkyl or C₃₋₆ cycloalkyl;
R² is selected from the group consisting of a hydrogen atom, C₁₋₆ haloalkyl, and C₃₋₆ cycloalkyl; or
R¹ and R², together with the carbon atom to which they are attached, form C₃₋₆ cycloalkyl;
m is an integer from 0 to 4;
L is a linker unit;
n is 1 to 10; preferably, n is 1 to 8; more preferably, n is 2 to 8;
Pc is the anti-TF antibody according to any one of claims 1 to 6.

11. The antibody-drug conjugate of general formula (Pc-L-Y-D) or the pharmaceutically acceptable salt thereof according to claim 10, wherein the linker unit -L- is -L¹-L²-L³-L⁴-, wherein
L¹ is selected from the group consisting of -(succinimid-3-yl-*N*)-W-C(O)-, -CH₂-C(O)-NR³-W-C(O)-, and -C(O)-W-C(O)-, wherein W is selected from the group consisting of C₁₋₆ alkylene and C₁₋₆ alkylene-C₃₋₆ cycloalkyl, wherein the C₁₋₆ alkylene or C₁₋₆ alkylene-C₃₋₆ cycloalkyl is each independently optionally substituted with one or more substituents selected from the group consisting of halogen, hydroxy, cyano, amino, C₁₋₆ alkyl, C₁₋₆ haloalkyl, deuterated C₁₋₆ alkyl, C₁₋₆ alkoxy, and C₃₋₆ cycloalkyl;
L² is selected from the group consisting of -NR⁴(CH₂CH₂O)p¹CH₂CH₂C(O)-, - NR⁴(CH₂CH₂O)p¹CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, chloroalkyl, deuterated alkyl, alkoxy, and cycloalkyl;
L⁴ is selected from the group consisting of -NR⁵(CR⁶R⁷)ₜ-, -C(O)NR⁵-, -C(O)NR⁵(CH₂)ₜ-, and a chemical bond, wherein t is an integer from 1 to 6;
R³, R⁴, and R⁵ are identical or different and are each independently selected from the group consisting of a hydrogen atom, C₁₋₆ alkyl, C₁₋₆ haloalkyl, deuterated C₁₋₆ alkyl, and C₁₋₆ hydroxyalkyl;
R⁶ and R⁷ are identical or different and are each independently selected from the group consisting of a hydrogen atom, halogen, C₁₋₆ alkyl, C₁₋₆ haloalkyl, deuterated C₁₋₆ alkyl, and C₁₋₆ hydroxyalkyl;
preferably, the linker unit -L¹-L²-L³-L⁴- is as follows:
L¹ is 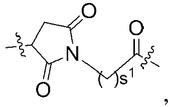
wherein s¹ is an integer from 2 to 8;
L² is a chemical bond;
L³ is a tetrapeptide residue; preferably, L³ is a tetrapeptide residue of GGFG (SEQ ID NO: 105);
L⁴ is -NR⁵(CR⁶R⁷)t-, wherein R⁵, R⁶, and R⁷ are identical or different and are each independently a hydrogen atom or C₁₋₆ alkyl, and t is 1 or 2;
the L¹ terminus of -L- is connected to Pc, and the L⁴ terminus is connected to Y.

12. The antibody-drug conjugate of general formula (Pc-L-Y-D) or the pharmaceutically acceptable salt thereof according to claim 10 or 11, being an antibody-drug conjugate of general formula (Pc-Lₐ-Y-D) or a pharmaceutically acceptable salt thereof: 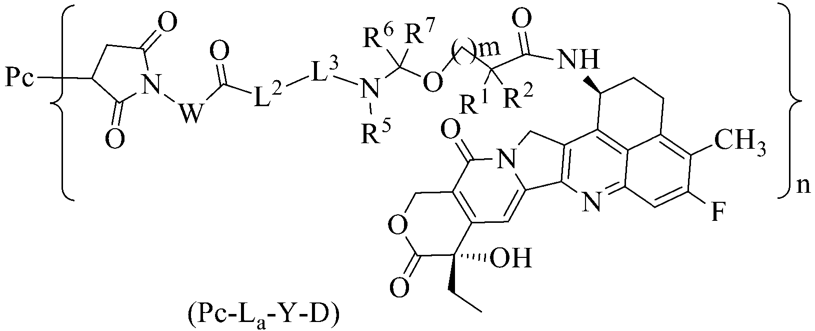 wherein:
Pc is the anti-TF antibody according to any one of claims 1 to 6;
m is an integer from 0 to 4;
n is 1 to 10;
R¹ is C₃₋₆ cycloalkyl-C₁₋₆ alkyl or C₃₋₆ cycloalkyl;
R² is selected from the group consisting of a hydrogen atom, C₁₋₆ haloalkyl, and C₃₋₆ cycloalkyl; or
R¹ and R², together with the carbon atom to which they are attached, form C₃₋₆ cycloalkyl;
W is selected from the group consisting of C₁₋₆ alkylene and C₁₋₆ alkylene-C₃₋₆ cycloalkyl,
wherein the C₁₋₆ alkylene and C₁₋₆ alkylene-C₃₋₆ cycloalkyl are each independently optionally substituted with one or more substituents selected from the group consisting of halogen, hydroxy, cyano, amino, alkyl, chloroalkyl, deuterated alkyl, alkoxy, and cycloalkyl;
L² is selected from the group consisting of -NR⁴(CH₂CH₂O)p¹CH₂CH₂C(O)-, - NR⁴(CH₂CH₂O)p¹CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, chloroalkyl, deuterated alkyl, alkoxy, and cycloalkyl;
R⁴ and R⁵ are selected from the group consisting of a hydrogen atom, alkyl, haloalkyl, deuterated alkyl, and hydroxyalkyl;
R⁶ and R⁷ are identical or different and are each independently selected from the group consisting of a hydrogen atom, halogen, alkyl, haloalkyl, deuterated alkyl, and hydroxyalkyl.

13. The antibody-drug conjugate of general formula (Pc-L-Y-D) or the pharmaceutically acceptable salt thereof according to any one of claims 10 to 12, being an antibody-drug conjugate of general formula (Pc-9-A) or a pharmaceutically acceptable salt thereof: 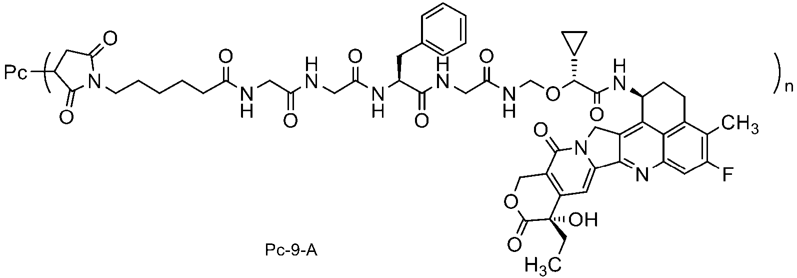 wherein:
n is 1 to 8;
Pc is the anti-TF antibody according to any one of claims 1 to 6.

14. A method for preparing an antibody-drug conjugate of general formula (Pc-Lₐ-Y-D) or a pharmaceutically acceptable salt thereof, comprising the following steps: 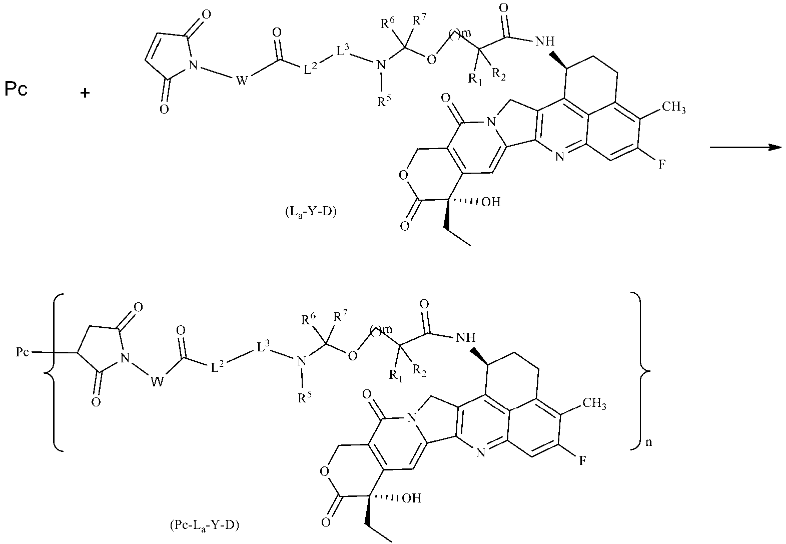
subjecting reduced Pc to a coupling reaction with a compound of general formula (Lₐ-Y-D) to give the antibody-drug conjugate of general formula (Pc-Lₐ-Y-D) or the pharmaceutically acceptable salt thereof;
wherein:
Pc is the anti-TF antibody according to any one of claims 1 to 6;
W, L², L³, R¹, R², R⁵-R⁷, m, and n are as defined in claim 12.

15. A pharmaceutical composition, comprising the anti-TF antibody according to any one of claims 1 to 6, or the nucleic acid molecule according to claim 7, or the antibody-drug conjugate or the pharmaceutically acceptable salt thereof according to any one of claims 10 to 13, and one or more pharmaceutically acceptable excipients, diluents, or carriers.

16. Use of the anti-TF antibody according to any one of claims 1 to 6, or the nucleic acid molecule according to claim 7, or the antibody-drug conjugate or the pharmaceutically acceptable salt thereof according to any one of claims 10 to 13, or the pharmaceutical composition according to claim 15 in the manufacture of a drug for treating a tumor or cancer, wherein
preferably, the tumor or cancer is selected from the group consisting of breast cancer, pancreatic cancer, lung cancer, esophageal cancer, ovarian cancer, cervical cancer, endometrial cancer, prostate cancer, bladder cancer, fallopian tube cancer, peritoneal cancer, colorectal cancer, head and neck cancer, and squamous cell carcinoma.
